# EUROPEAN PATENT SPECIFICATION

(11) **EP 4 188 501 B1**
(45) Date of publication and mention of the grant of the patent: **28.10.2026**
(21) Application number: 21729130.1
(22) Date of filing: 07.05.2021
(51) Int. Cl.: A61M 25/00, A61M 25/06

(54) **EXPANDABLE SHEATH FOR INTRODUCING AN ENDOVASCULAR DELIVERY DEVICE INTO A BODY**
EXPANDIERBARE HÜLLE ZUM EINFÜHREN EINER ENDOVASKULÄREN ABGABEVORRICHTUNG IN EINEN KÖRPER
GAINE EXPANSIBLE DESTINÉE À INTRODUIRE UN DISPOSITIF D'ACHEMINEMENT ENDOVASCULAIRE DANS UN CORPS

(30) Priority: 31.07.2020 US 202063059772 P
(43) Date of publication of application: 07.06.2023
(73) Proprietor: Edwards Lifesciences Corporation, Irvine, CA 92614-5688 (US)
(72) Inventor: LEE, Jeong Soo, Irvine, CA 92614 (US); GAFFNEY, Leah Paige, Irvine, CA 92614 (US); KIM, Judy, Irvine, CA 92614 (US); MORA, David, Lake Forest, CA 92630 (US); ANDERSON, Elizabeth Anne, Irvine, CA 92614 (US); TRAN, Sonny, Irvine, CA 92614 (US); SALEH, Nasser William, Irvine, CA 92614 (US); TAMIR, Ilan, Irvine, CA 92614 (US); FINE, Maxwell Harrison, Irvine, CA 92614 (US); NGUYEN, Duy, Corona, CA 92883 (US); NGUYEN, Kim D., Irvine, CA 92614 (US); NGUYEN, Thanh V., Irvine, CA 92604 (US); BULMAN, Erik, Irvine, CA 92614 (US)
(74) Representative: Eisenführ Speiser
(86) International application number: PCT/US2021/031275
(87) International publication number: WO 2022/026026

(56) References cited:
- US-B2- 8 690 936

## Description

### CROSS-REFERENCE TO RELATED APPLICATIONS

This application claims the benefit of U.S. Provisional Application No.63/059,772, filed July 31, 2020.

### FIELD

The present application concerns aspects of a sheath for use with catheter-based technologies for repairing and/or replacing heart valves, as well as for delivering a prosthetic device, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

However, delivery and/or removal of prosthetic devices and other material to or from a patient still poses a significant risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

Accordingly, there remains a need in the art for an improved introducer sheath for endovascular systems used for implanting valves and other prosthetic devices.

US 8,690,936 B2 describes embodiments of an expandable sheath that is used in con junction with a catheter assembly to introduce a prosthetic device, such as a tissue heart valve, into a patient. Some embodiments comprise a sheath with inner and outer tubular layers, and an intermediate tubular layer comprising a shape memory alloy. Other embodiments comprise one or more layers having one or more longitudinal notches or cuts to facilitate expansion of the sheath.

### SUMMARY

The present invention relates to a sheath as defined by appended claim 1, and to a method of making a sheath as defined by appended claim 14. The disclosure may provide further aspects. In the case that an aspect does not require all features of one of claims 1 and 14, this does not render the missing feature an optional element of the claimed invention. The description of such aspect may still serve understanding of specific features included in the invention and its functionality.

The disclosed herein an expandable sheath can minimize trauma to the vessel by allowing for temporary expansion of a portion of the introducer sheath to accommodate a delivery system, followed by a return to the original diameter once the delivery system passes through. Some aspects can comprise a sheath with a smaller profile than that of prior art introducer sheaths. Furthermore, as described in certain aspects, the disclosed sheath can reduce the length of time a procedure takes, as well as reduce the risk of a longitudinal or radial vessel tear or plaque dislodgement because only one sheath is required, rather than several different sizes of sheaths. In still further aspects, the present expandable sheath can require only a single vessel insertion, as opposed to requiring multiple insertions for the dilation of the vessel.

In one aspect disclosed herein is a sheath for delivering a medical device, wherein the sheath has a proximal and a distal end and comprises an elongated tube forming an outer layer of the sheath that is positioned at at least the proximal end of the sheath and extending along at least a portion of a length of the sheath, having an inner surface and an outer surface, and wherein the elongated tube comprises a first polymer layer, wherein the first polymer layer comprises a first compound composition comprising: from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition; wherein the elongated tube is configured to reversibly expand from an initial diameter d₀ in an unexpended position to an expanded diameter dₑ in an expanded position upon passage of a medical device; and wherein the sheath exhibits at least a 10 % reduction in an insertion force when compared with a substantially identical reference sheath that does not comprise the first polymer layer.

In one aspect, the first polymer can have a substantially same durometer along a total length of the elongated tube. Yet, in other aspects, a durometer of the first polymer at a proximal end of the elongated tube can be different from a durometer of the first polymer at a distal end of the elongated tube.

In one aspect, wherein the elongated tube comprises two or more polymer layers. In such an exemplary aspect, the elongated tube comprises at least a second polymer layer comprising a second compound composition comprising from greater than 0 wt% to 100 wt% of a second polymer comprising polyether block amide, a polyurethane, or a composition thereof. In certain exemplary aspects, the second compound composition can further comprise up to 20 % of tackiness reducing additive based on a total weight of the second compound composition.

In still further aspects, the second polymer layer can comprise PEBAX^{®}. While in other aspects, the second polymer layer can comprise polyurethane.

In some aspects, the sheath disclosed herein can exhibit at least a 20% reduction in an insertion force when compared with a substantially identical reference sheath that does not comprise the first polymer layer.

In still further aspects, the disclosed sheath for introducing a prosthetic device comprises an inner layer and an outer layer. In one aspect, the sheath as disclosed herein further comprises an expandable tubular inner liner extending along the length of the sheath and comprising at least one folded portion, wherein the expandable inner liner has an inner surface and an outer surface, wherein the inner surface of the expandable inner liner defines a lumen and forms an inner surface of the at least one folded portion, and wherein the outer surface extends circumferentially to form an outer surface of the at least one folded portion; and a first outer tubular layer extending at least partially along the length of the sheath and having an inner surface and an outer surface, wherein the inner surface of the first outer tubular layer further extends at least partially around the outer surface of the inner liner such that at least a portion of the inner surface of the first outer tubular layer is positioned adjacent to the outer surface of the at least one folded portion of the inner liner; wherein the elongated tube is positioned such that at least a portion of the inner surface of the elongated tube overlies at least a portion of the outer surface of the first outer tubular layer.

In still further aspects, the disclosed herein sheath has a proximal and a distal end and comprises: an expandable tubular inner liner comprising at least one folded portion, wherein the expandable inner liner has an inner surface and an outer surface, wherein the inner surface of the expandable inner liner defines a lumen and forms an inner surface of the at least one folded portion, and wherein the outer surface extends circumferentially to form an outer surface of the at least one folded portion, a first outer tubular layer having an inner surface and an outer surface, wherein the inner surface of the first outer tubular layer extends at least partially around the outer surface of the inner liner such that at least a portion of the inner surface of the first outer tubular layer is positioned adjacent to the outer surface of the at least one folded portion of the inner liner; and an elongated tube forming a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer, wherein the elongated tube comprises a first polymer layer, wherein the first polymer layer comprises a first compound composition comprising from greater than 0 wt% to less than 100 wt % of a polymer comprising a polyether block amide, a polyurethane, or a combination thereof; less than about 65 % of an inorganic filler based on a total weight of the first compound composition; and up to about 20 % of a solid lubricant filler based on a total weight of the first compound composition.

Also disclosed herein is an aspect describing a sheath for delivering a medical device, wherein the sheath has a proximal and a distal end and comprises an expandable tubular inner liner comprising at least one folded portion, wherein the expandable inner liner has an inner surface and an outer surface, wherein the inner surface of the expandable inner liner defines a lumen and forms an inner surface of the at least one folded portion, and wherein the outer surface extends circumferentially to form an outer surface of the at least one folded portion and wherein the outer surface of the inner liner is selectively etched; a first outer tubular layer having an inner surface and an outer surface, wherein the inner surface of the outer layer extends at least partially around the outer surface of the inner liner such that at least a portion of the inner surface of the outer layer is positioned adjacent to at least a portion of the outer surface of the at least one folded portion of the inner liner; and an elongated tube forming a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer, wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65% of an inorganic filler based on a total weight of the first compound composition; and up to about 20 % of a solid lubricant filler based on a total weight of the first compound composition.

Also disclosed herein are aspects, where in an addition to the elongated tube as described in any of the preceding aspects, the sheath further comprises a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of a cylinder having a longitudinal axis; wherein the variable diameter inner liner is configured to reversible expand from a predetermined rest diameter *dᵣ* to an expanded diameter *d₁* by sliding the first edge of the sheet along at least a portion of the inner surface and sliding the second edge of the sheet along the at least a portion of outer surface, during application of a radial outward force by passage of a medical device through the lumen of the inner liner; and wherein the elongated tube is positioned such that the inner surface of the elongated tube overlies at least a portion of the outer surface of the inner liner.

Also disclosed herein is a sheath for delivering a medical device, wherein the sheath has a proximal and a distal end and comprises: a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of the cylinder having a longitudinal axis; wherein the variable diameter inner liner is configured to reversible expand from a predetermined rest diameter *dᵣ* to an expanded diameter *d₁* by sliding the first edge of the sheet along at least a portion of the inner surface and sliding the second edge of the sheet along the at least a portion of outer surface, during application of a radial outward force by passage of a medical device through the lumen of the inner liner; and an elongated tube forming an outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the inner liner, wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition.

Also disclosed herein is an expandable sheath comprising: an inner tubular layer comprising a longitudinal slit and partially defining an inner lumen; a first outer tubular layer enveloping the inner layer, the outer tubular layer comprising a longitudinally extending, folded flap that overlies a portion of an outer surface of the outer layer when the sheath is in an unexpanded state; and an elongated tube forming a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer, wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition; wherein an outwardly directed radial force from a prosthetic device moving through the inner lumen widens the longitudinal slit and unfolds the folded flap to allow expansion of the sheath.

Still further disclosed herein, is a sheath for delivering a medical device, the sheath comprising: a continuous inner layer defining a lumen therethrough, the inner layer including a first fold and a second fold and an overlapping folded portion extending circumferentially between the first and second folds, the folded portion comprising overlap in a radial direction of at least two thicknesses of the inner layer; a discontinuous first outer tubular layer extending at least partially around the inner layer, the first outer tubular layer having an overlapping portion and an underlaying portion, at least a portion of the folded portion of the inner layer is positioned between the overlapping portion and the underlaying portion; and an elongated tube forming a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer, wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition; wherein at least a portion of the sheath is configured to locally expand from an unexpanded configuration in which the lumen has a first diameter to an expanded configuration in which the lumen has a second diameter larger than the first diameter due to an outwardly directed radial force exerted by a medical device against the inner layer, and then locally contract at least partially back to the unexpanded configuration as the prosthetic device passes through the lumen.

Also disclosed herein is a sheath delivering a medical device comprising: a continuous inner layer defining a lumen therethrough, the inner layer including a first fold and a second fold and an overlapping folded portion extending circumferentially between the first and second folds, the folded portion comprising overlap in a radial direction of at least two thicknesses of the inner layer; a discontinuous first outer tubular layer extending at least partially around the inner layer, the first outer tubular layer having an overlapping portion and an underlaying portion, at least a portion of the folded portion of the inner layer is positioned between the overlapping portion and the underlaying portion; a coiled wire along a length of the sheath, the coil wire providing uniform bending of the sheath to prevent kinking; and an elongated tube forming a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer, wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition, wherein at least a portion of the sheath is configured to locally expand from an unexpanded configuration in which the lumen has a first diameter to an expanded configuration in which the lumen has a second diameter larger than the first diameter due to an outwardly directed radial force exerted by a medical device against the inner layer, and then locally contract at least partially back to the unexpanded configuration as the prosthetic device passes through the lumen.

Also disclosed herein is a method of making a sheath having a proximal end and a distal end and comprising: a) extruding a tubular body to form an elongated tube comprising a first polymer layer, wherein the first polymer layer comprises a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; b) disposing the elongated tube on the sheath such that the elongated tube forms an outer layer of the sheath, and wherein the elongated tube is positioned at at least the proximal end of the sheath and extending along at least a portion of a length of the sheath, wherein the elongated tube is configured to reversibly expand from an initial diameter d₀ in an unexpended position to an expanded diameter dₑ in an expanded position upon passage of a medical device; and wherein the formed sheath exhibits at least a 10% reduction in an insertion force when compared with a substantially identical reference sheath that does not comprise the first polymer layer.

Also disclosed herein is the method further comprising providing: a) a continuous inner layer defining a lumen therethrough, the inner layer including a first fold and a second fold and an overlapping folded portion extending circumferentially between the first and second folds, the folded portion comprising overlap in a radial direction of at least two thicknesses of the inner layer; b) a discontinuous first outer tubular layer extending at least partially around the inner layer, the first outer tubular layer having an overlapping portion and an underlaying portion, at least a portion of the folded portion of the inner layer is positioned between the overlapping portion and the underlaying portion; and c) a coiled wire along a length of the sheath, the coil wire providing uniform bending of the sheath to prevent kinking; and disposing the elongated tube such that the inner surface of the elongated tube overlies at least a portion of the outer surface of the first outer tubular layer, and wherein at least a portion of the sheath is configured to locally expand from an unexpanded configuration in which the lumen has a first diameter to an expanded configuration in which the lumen has a second diameter larger than the first diameter due to an outwardly directed radial force exerted by a medical device against the inner layer, and then locally contract at least partially back to the unexpanded configuration as the prosthetic device passes through the lumen.

Also further disclosed herein is the method further comprising: providing: a) a circumferentially continuous first elastic outer tubular layer defining an initial elastic lumen extending axially therethrough, the initial elastic lumen having an initial diameter; and b) an inner tubular layer extending through the initial elastic lumen of the first elastic outer tubular layer and comprising at least three circumferentially spaced, longitudinally extending thick wall segments and at least three circumferentially spaced, longitudinally extending thin wall segments, each thin wall segment extending between two adjacent thick wall segments to define an expanded lumen extending axially through the inner tubular layer, the expanded lumen having an expanded diameter larger than the initial diameter of the initial elastic lumen; c) wherein the inner tubular layer, in a compressed condition, forms at least three circumferentially spaced folds, each of the circumferentially spaced folds including a three-layer thickness in a radial direction comprised of portions of two adjacent thick wall segments and a thin wall segment sandwiched therebetween; wherein the inner tubular layer, in a locally expanded condition, has the thick wall segments and the thin wall segments unfolded and expanded apart; and wherein the inner tubular layer is configured to be urged by the first elastic outer tubular layer at least partially back to the compressed condition after passage of an implant through the expanded lumen; and disposing the elongated tube such that the inner surface of the elongated tube overlies at least a portion of the outer surface of the first elastic outer layer.

Also disclosed herein is a method comprising: providing: a) an expandable tubular inner liner extending along the length of the sheath and comprising at least one folded portion, wherein the expandable inner liner has an inner surface, and an outer surface, wherein the inner surface of the expandable inner liner defines a lumen and forms an inner surface of the at least one folded portion, and wherein the outer surface extends circumferentially to form an outer surface of the at least one folded portion; b) a first outer tubular layer extending at least partially along the length of the sheath and having an inner surface and an outer surface, wherein the inner surface of the first outer tubular layer further extends at least partially around the outer surface of the inner liner such that at least a portion of the inner surface of the first outer tubular layer is positioned adjacent to the outer surface of the at least one folded portion of the inner liner; and disposing the elongated tube such that the inner surface of the elongated tube overlies at least a portion of the outer surface of the first outer tubular layer, and wherein at least a portion of the sheath is configured to locally expand from an unexpanded configuration in which the lumen has a first diameter to an expanded configuration in which the lumen has a second diameter larger than the first diameter due to an outwardly directed radial force exerted by a medical device against the inner layer, and then locally contract at least partially back to the unexpanded configuration as the prosthetic device passes through the lumen.

The foregoing and other features and advantages of the disclosure will become more apparent from the following detailed description, which proceeds with reference to the accompanying figures. In the following, 1 inch can be converted into 25.4 mm, and 1 psi can be converted into 0.0689 bar.

### BRIEF DESCRIPTION OF THE DRAWINGS

**FIG. 1** is an elevation view of a sheath according to the present disclosure along with an endovascular delivery apparatus for implanting a prosthetic valve.
**FIGs. 2A****, B,** and **D** are section views of an exemplary sheath for introducing a prosthetic device into a patient, and **FIG. 2C** is a perspective view of one component of such a sheath.
**FIG.** 3 is an elevation view of the sheath shown in **FIG. 2****.**
**FIGs. 4A-4B** show elevation views of two aspects of a sheath according to the present disclosure, having varying outer diameters.
**FIG. 5** illustrates an elevation view of one aspect of a sheath, expanded at a first location to accommodate a delivery system.
**FIG. 6** shows an elevation view of the sheath, expanded at a second location farther down the sheath.
**FIG. 7** shows a section view of another aspect of a sheath that further comprises an outer covering or shell.
**FIG. 8** illustrates an elevation view of one aspect of a sheath with an outer covering or shell.
**FIG. 9** illustrates a partial elevation view of one aspect of an intermediate tubular layer that can be used to construct a sheath according to the present disclosure.
**FIG. 10** illustrates a partial elevation view of another aspect of an intermediate tubular layer having a variable diamond design.
**FIG. 11** illustrates a partial elevation view of another aspect of an intermediate tubular layer having a diamond design with spring struts.
**FIG. 12** illustrates a partial elevation view of another aspect of an intermediate tubular layer having a diamond design with straight struts.
**FIG. 13** illustrates a partial elevation view of another aspect of an intermediate tubular layer having a saw tooth design with spring struts.
**FIG. 14** illustrates a partial elevation view of another aspect of an intermediate tubular layer having a saw tooth design with straight struts.
**FIG. 15** illustrates a partial elevation view of another aspect of an intermediate tubular layer having a diamond design with straight struts.
**FIG. 16** illustrates a partial elevation view of another aspect of an intermediate tubular layer having a helical or spiral design.
**FIG. 17** illustrates a partial elevation view of another aspect of an intermediate tubular layer having a diamond design with non-straight struts.
**FIG. 18** illustrates a partial elevation view of another aspect of an intermediate tubular layer having an alternative diamond design with non-straight struts.
**FIG. 19** illustrates a partial elevation view of another aspect of an intermediate tubular layer having yet another diamond design with non-straight struts.
**FIG. 20** illustrates a partial elevation view of another aspect of an intermediate tubular layer having a diamond design with struts.
**FIG. 21** illustrates a partial elevation view of another aspect of an intermediate tubular layer having a design similar to that shown in **FIG. 20****,** but with additional struts.
**FIG. 22** illustrates a partial elevation view of another aspect of an intermediate tubular layer having a diamond design with spiral struts.
**FIG. 23** illustrates a partial elevation view of another aspect of an intermediate tubular layer having a diamond design with adjacent struts.
**FIG. 24** illustrates a section view of one aspect of a sheath having a longitudinal notch.
**FIG. 25** shows a section view of one aspect of a sheath having a longitudinal cut in the inner layer.
**FIG. 26** shows a perspective view of an exemplary sheath having a plurality of notches or cuts in the outer tubular layer in one aspect.
**FIG. 27A** illustrates a section view of one aspect of a sheath, wherein the outer tubular layer contains a longitudinal cut, and the inner layer extends into the gap created by the cut in the outer tubular layer, in an unexpanded configuration; and **FIGs. 27B-27E** show section views of various aspects of a sheath in the unexpanded configuration.
**FIG. 28** shows a section view of the sheath of **FIG. 27A** in an expanded configuration.
**FIGs. 29A-29D** show section views of various aspects of a sheath having overlapping sections.
**FIG. 30** illustrates a block diagram of one aspect of a method of making a sheath according to the present disclosure.
**FIG. 31** illustrates a block diagram of another aspect of a method of making a sheath according to the present disclosure.
**FIGs. 32A-32H** illustrates section or elevation views of various method steps of the methods shown in **FIGs. 30-31****.**
**FIG. 33** illustrates a plan view of one aspect of a sheath having a partial slit or score line.
**FIG. 34** illustrates a plan view of another aspect of a sheath having a partial slit or score line.
**FIG. 35** is an elevation view of an expandable sheath according to the present disclosure and a representative housing.
**FIG. 36** is an enlarged cutaway view of the distal end of the sheath of **FIG. 35****.**
**FIG. 37** is a section view of the distal end of the sheath of **FIG. 35****,** taken along line **37-37** in **FIG. 36****.**
**FIG. 38** is a section view of a proximal section of the sheath of **FIG. 35****,** taken along line **38-38** in **FIG. 35****.**
**FIG. 39** is a section view of the sheath of **FIG. 35** in a rest (unexpanded) configuration, taken along line **39-39** in **FIG. 35****.**
**FIG. 40** is the section view of the sheath of **FIG. 39****,** in an expanded configuration.
**FIG. 41** shows an elevation view of an expandable sheath having an elastic outer cover, according to another aspect.
**FIG. 42** illustrates a section view of the sheath of **FIG. 41****,** taken along line **42-42 in** **FIG. 41****.**
**FIG. 43** illustrates the sectional view of the sheath shown in **FIG. 42****,** in an expanded configuration.
**FIG. 44** illustrates a section view of another aspect of an expandable sheath.
**FIG. 45** shows an expanded configuration of the sheath of **FIG. 44****.**
**FIG. 46** illustrates a section view of another aspect of an expandable sheath.
**FIG. 47** shows an expanded configuration of the sheath of **FIG. 46****.**
**FIG. 48** illustrates a section view of another aspect of an expandable sheath according to the present disclosure.
**FIG. 49** illustrates a section view of another aspect of an expandable sheath.
**FIG. 50** is a section view of an example sheath in an unexpanded configuration.
**FIG. 51** is a section view of the sheath of **FIG. 50** in an expanded configuration.
**FIG. 52** is a section view of the sheath of **FIG. 50****,** including an outer jacket.
**FIG. 53** is a section view of the sheath of **FIG. 35** in a rest (unexpanded) configuration, including an outer jacket, taken along line **39-39** in **FIG. 35****.**
**FIG. 54** is a section view of the sheath of **FIG. 53** in a rest (unexpanded) configuration, taken along line **39-39 in** **FIG. 35****.**
**FIG. 55** is a section view of the sheath of **FIG. 54****,** in an expanded configuration.
**FIG. 56** is a section view of the sheath of **FIG. 54** in a rest (unexpanded) configuration, including a lubricant between the outer layer and the outer jacket.
**FIG. 57** is a section view of the sheath of **FIG. 54** in a rest (unexpanded) configuration, including a lubricant and a bonding strip.
**FIG. 58** is a bottom perspective view of the sheath of **FIG. 57****.**
**FIG. 59** is a bottom perspective view of the sheath of **FIG. 57****.**
**FIG. 60A** is a top perspective view of the sheath of **FIG. 54****.**
**FIG. 60B** is a section view of an exemplary sheath in one aspect.
**FIGs. 61A-61****B** are section views of aspects of a sheath for introducing a medical device into a patient, and **FIG. 61C** is a perspective view of one of the components of the exemplary sheath.
**FIGs. 62A-B** illustrate a section view of one aspect of an exemplary inner liner: **FIG. 62A** depicts an unexpanded configuration, while **FIG. 62B** depicts an expanded configuration.
**FIGs. 63A-63E** **and 36H** show section views of various aspects of exemplary sheaths. **FIGS. 63F, 63G****,** and **63I** show perspective views of various aspects of exemplary sheaths.

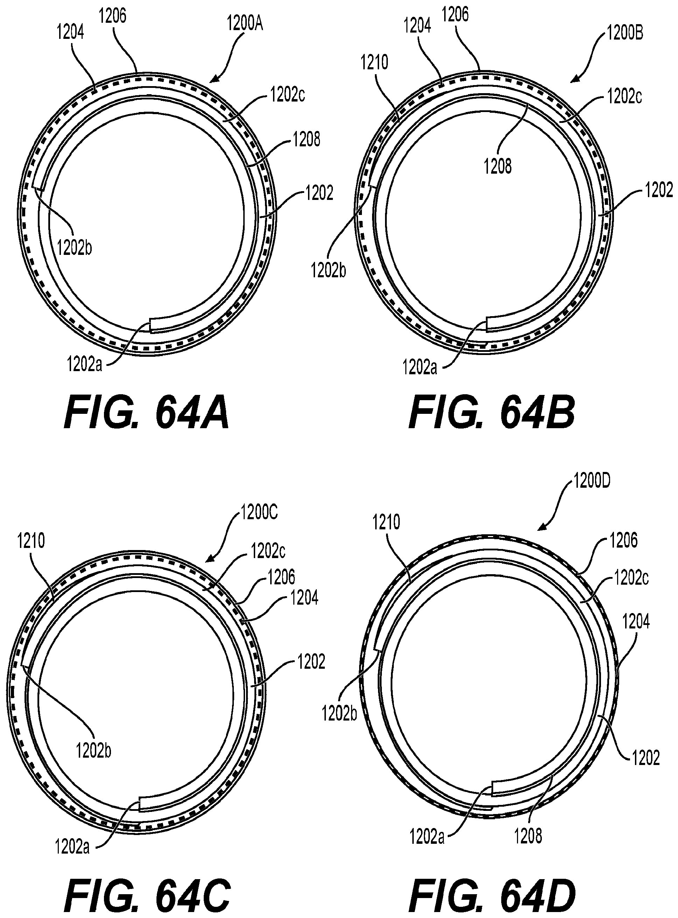

**FIGs. 64A-64D** are section views of the distal end of the exemplary sheath; **FIG. 64A** shows a section view of the exemplary sheath with a lubricant disposed between the sliding and overlaying portions of the sheet and the braid that is not embedded into the elastomeric polymer layer; **FIG. 64B** shows a section view of the exemplary sheath with a lubricant disposed between the sliding and overlaying portions of the sheet and a lubricant disposed between the inner liner and outer layer, where the braid that is not embedded into the elastomeric polymer layer; **FIG. 64C** shows a section view of the exemplary sheath with a lubricant disposed between the inner liner and outer layer, where the braid that is not embedded into the elastomeric polymer layer, with and without the lubricant; **FIG. 64D** shows a section view of the exemplary sheath with a lubricant disposed between the sliding and overlaying portions of the sheet and a lubricant disposed between the inner liner and outer layer, where the braid is at least partially embedded into the elastomeric polymer layer.

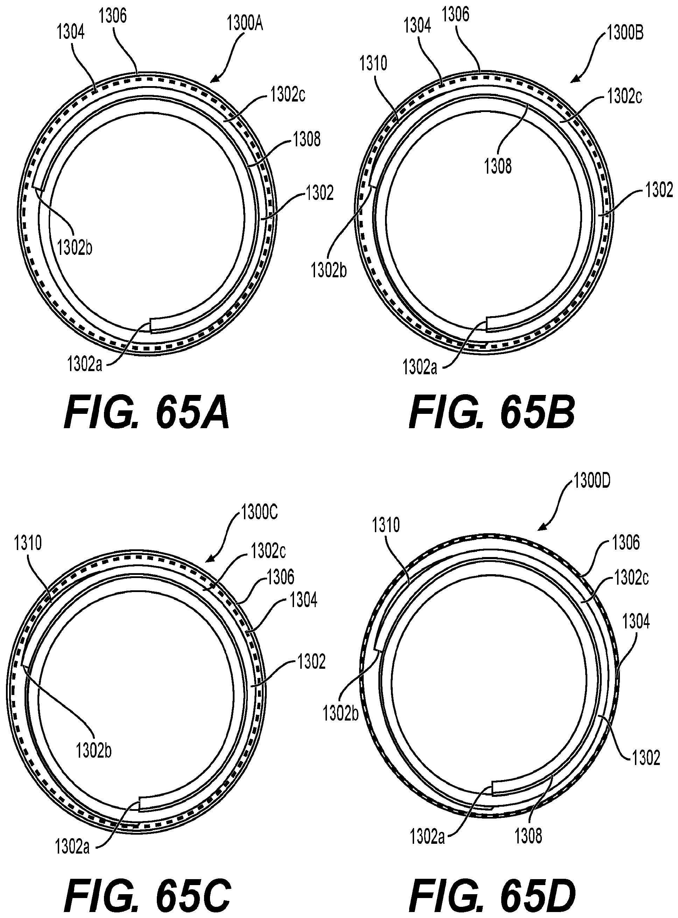

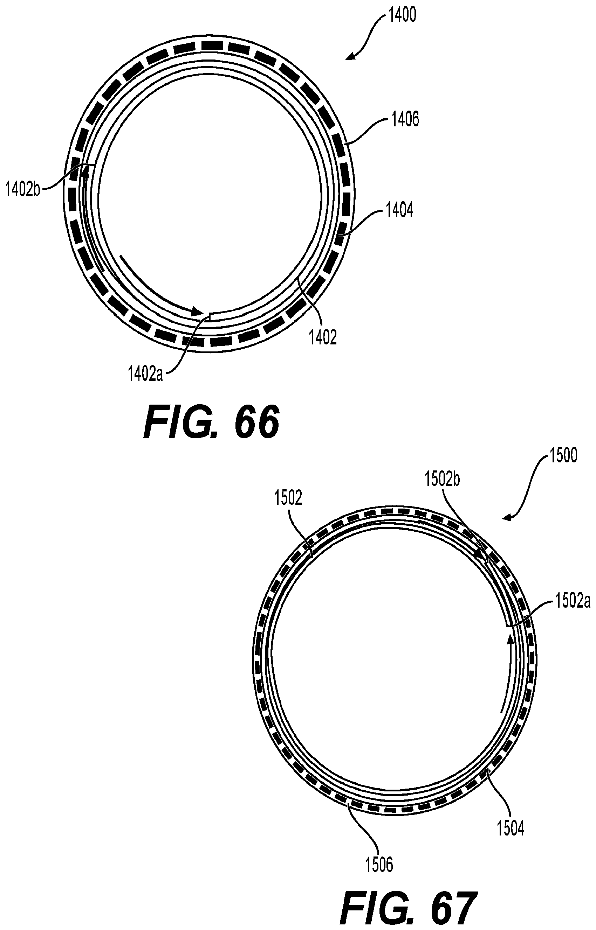

**FIGs. 65A-D** illustrate a section view of a proximal section of the sheath; **FIG. 65A** shows a section view of the exemplary sheath with a lubricant disposed between the sliding and overlaying portions of the sheet and the braid that is not embedded into the elastomeric polymer layer; **FIG. 65B** shows a section view of the exemplary sheath with a lubricant disposed between the sliding and overlaying portions of the sheet and a lubricant disposed between the inner liner and outer layer, where the braid that is not embedded into the elastomeric polymer layer; **FIG. 66C** shows a section view of the exemplary sheath with a lubricant disposed between the inner liner and outer layer, where the braid that is not embedded into the elastomeric polymer layer, with and without the lubricant; **FIG. 66D** shows a section view of the exemplary sheath with a lubricant disposed between the sliding and overlaying portions of the sheet and a lubricant disposed between the inner liner and outer layer, where the braid is at least partially embedded into the elastomeric polymer layer.
**FIG. 66** is a section view of the sheath in a rest (unexpanded) configuration, taken along the distal end.
**FIG. 67** shows a section view of the sheath of **FIG. 66** in an expanded configuration.
**FIG. 68** illustrates a block diagram of one aspect of a method of making a sheath according to the present disclosure.
**FIG. 69** illustrates a block diagram of another aspect of a method of making a sheath according to the present disclosure.
**FIGs. 70A-70J** illustrates section or side views of various method steps of the methods shown in **FIGs. 68-69****.**
**FIG. 71** is a section view of an exemplary sheath in one aspect.
**FIG. 72** is a section view of an exemplary sheath in one aspect.
**FIG. 73** is an elevation view of an exemplary elongated tube according to another aspect.
**FIG. 74** is a cross-section view of an exemplary elongated tube taken along section line A-A of **FIG. 73****.**
**FIG. 75** is a section view of an exemplary elongated tube in a rest (unexpanded) configuration, take along section lines B-B of **FIG 73****.**
**FIG. 76** is a partial section view of an exemplary elongated tube in one aspect.
**FIG. 77** is a section view of another exemplary elongated tube in a rest (unexpanded) configuration, including a single reinforcing member, taken along section lines B-B of **FIG. 73****.**

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "polymer" includes aspects having two or more such polymers unless the context clearly indicates otherwise.

It is appreciated that certain features of the disclosure, which are, for clarity, described in the context of separate aspects, can also be provided in combination in a single aspect. Conversely, various features of the disclosure, which are, for brevity, described in the context of a single aspect, can also be provided separately or in any suitable combination.

As used herein, the terms "optional" or "optionally" mean that the subsequently described event or circumstance may or may not occur and that the description includes instances where said event or circumstance occurs and instances where it does not.

It is also to be understood that the terminology used herein is for the purpose of describing particular aspects only and is not intended to be limiting. As used in the specification and the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

References in the specification and concluding claims to parts by weight of a particular element or component in a composition or article denotes the weight relationship between the element or component and any other elements or components in the composition or article for which a part by weight is expressed. Thus, in a composition or a selected portion of a composition containing 2 parts by weight of component X and 5 parts by weight component Y, X and Y are present at a weight ratio of 2:5 and are present in such ratio regardless of whether additional components are contained in the composition.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Furthermore, when numerical ranges of varying scope are set forth herein, it is contemplated that any combination of these values inclusive of the recited values may be used. Further, ranges can be expressed herein as from "about" one particular value and/or to "about" another particular value. When such a range is expressed, another aspect includes from the one particular value and/or to the other particular value.

Similarly, when values are expressed as approximations, by use of the antecedent "about," it will be understood that the particular value forms another aspect. It will be further understood that the endpoints of each of the ranges are significant both in relation to the other endpoint and independently of the other endpoint. Unless stated otherwise, the term "about" means within 5% (e.g., within 2% or 1%) of the particular value modified by the term "about."

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

As used herein, the term "composition" is intended to encompass a product comprising the specified ingredients in the specified amounts, as well as any product which results, directly or indirectly, from a combination of the specified ingredients in the specified amounts.

A weight percent of a component, unless specifically stated to the contrary, is based on the total weight of the formulation or composition in which the component is included.

As used herein, the term "substantially" means that the subsequently described event or circumstance completely occurs or that the subsequently described event or circumstance generally, typically, or approximately occurs.

As used herein, the term "substantially," when used in reference to a composition, refers to at least about 80%, at least about 85%, at least about 90%, at least about 91%, at least about 92%, at least about 93%, at least about 94%, at least about 95%, at least about 96%, at least about 97%, at least about 98%, at least about 99%, or about 100% by weight, based on the total weight of the composition, of a specified feature or component.

As used herein, the term "substantially," in, for example, the context "substantially free" refers to a composition having less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the terms "substantially identical reference composition" or "substantially identical reference article" refer to a reference composition or article comprising substantially identical components in the absence of an inventive component. In another exemplary aspect, the term "substantially," in, for example, the context "substantially identical reference composition," refers to a reference composition comprising substantially identical components and wherein an inventive component is substituted with a common in the art component.

Further, the terms "coupled" and "associated" generally mean electrically, electromagnetically, and/or physically (e.g., mechanically or chemically) coupled or linked and does not exclude the presence of intermediate elements between the coupled or associated items.

It will be understood that, although the terms "first," "second," etc., may be used herein to describe various elements, components, regions, layers and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of example aspects.

It is understood that the terms "layer" and "liner" can be used interchangeably. It is further understood that for the purposes of the current disclosure, the term "outer jacket" refers to the elongated tube having the disclosed herein composition and characteristics.

It is further understood that the phrases "insertion force" and "push force" can be used interchangeably.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein are interpreted accordingly.

As used herein, the term "atraumatic" is commonly known in the art and refers to a device or a procedure that minimized tissue injury.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate, effective amount will be readily determined by one of ordinary skill in the art using only routine experimentation.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

While aspects of the present disclosure can be described and claimed in a particular statutory class, such as the system statutory class, this is for convenience only, and one of ordinary skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred in any respect. This holds for any possible non-express basis for interpretation, including matters of logic with respect to arrangement of steps or operational flow, plain meaning derived from grammatical organization or punctuation, or the number or type of aspects described in the specification.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

The present disclosure may be understood more readily by reference to the following detailed description of various aspects of the disclosure and the examples included therein and to the Figures and their previous and following description.

The present disclosure may be understood more readily by reference to the following detailed description of various aspects of the disclosure and the examples included therein and to the Figures and their previous and following description.

### SHEATH

Disclosed aspects of an expandable sheath can minimize trauma to the vessel by allowing for temporary expansion of a portion of the introducer sheath to accommodate the delivery system, followed by a return to the original diameter once the device passes through. In some aspects, the sheath can comprise a sheath with a smaller profile (*e.g*., a smaller diameter in the rest configuration) than that of prior art introducer sheaths. Furthermore, as disclosed in the present aspects, the sheath can reduce the length of time a procedure takes, as well as reduce the risk of a longitudinal or radial vessel tear or plaque dislodgement because only one sheath is required, rather than several different sizes of sheaths. In certain aspects, the present expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices (*e.g*., stents, prosthetic heart valves, stented grafts, etc.) into many types of vascular and non-vascular body lumens (e.g., veins, arteries, esophagus, ducts of the biliary tree, intestine, urethra, fallopian tube, other endocrine or exocrine ducts, etc.).

Also, disclosed herein aspects refer to the sheaths having a reduced insertion force as compared to any other available commercial sheaths. As one of ordinary skill in the art would readily appreciate, the reduction in the insertion force of the sheath and any medical device passing through results in reduced or substantially eliminated danger to the patients during the medical procedure. It is understood that the insertion force of the inventive sheaths, reference sheaths, and any other commercially available sheaths is measured using the same standardized technique for a proper comparison. In such aspects, a tensile tester, Instron 3366, has been used to measure a simulated insertion force that is required for a prosthetic valve to enter the described sheath. An additional constriction tube has been used to simulate vascular elasticity that contributes to push force. In certain exemplary aspects, the test is performed in a water bath in a temperature range from room temperature to a normal body temperature (for example and without limitation from about 20 °C to less than about 40 °C). The specimen can be kept in a straight configuration. The test can be performed with a tapered mandrel to simulate a valve entering the sheath.

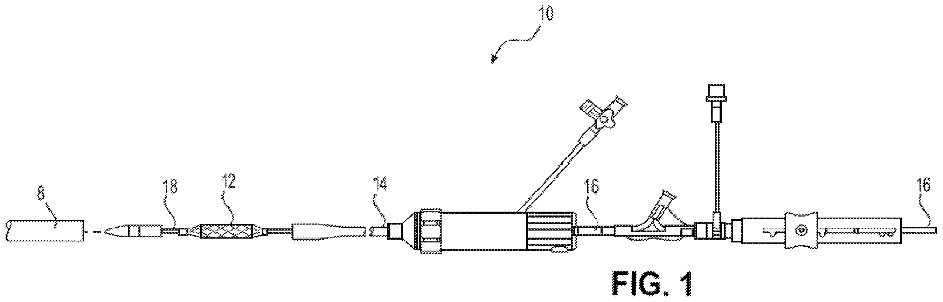

**FIG. 1** illustrates a sheath **8** according to the present disclosure, in use with a representative delivery apparatus **10,** for delivering a prosthetic device **12,** such as a tissue heart valve to a patient. The apparatus **10** can include a steerable guide catheter **14** (also referred to as a flex catheter), a balloon catheter **16** extending through the guide catheter **14,** and a nose catheter **18** extending through the balloon catheter **16.** The guide catheter **14,** the balloon catheter **16,** and the nose catheter **18** in the illustrated aspect are adapted to slide longitudinally relative to each other to facilitate delivery and positioning of the valve (prosthetic device) **12** at an implantation site in a patient's body, as described in detail below. Generally, sheath **8** is inserted into a vessel, such as the transfemoral vessel, passing through the skin of the patient, such that the distal end of the sheath **8** is inserted into the vessel. Sheath **8** can include a hemostasis valve at the opposite, proximal end of the sheath. The delivery apparatus **10** can be inserted into the sheath **8,** and the prosthetic device **12** can then be delivered and implanted within the patient.

The present disclosure relates to various configurations of the sheath. These exemplary aspects are directed to a sheath for delivering a medical device, wherein the sheath has a proximal and a distal end and comprises an elongated tube forming an outer layer of the sheath that is positioned at at least the proximal end of the sheath and extending along at least a portion of a length of the sheath, having an inner surface and an outer surface, and wherein the elongated tube comprises a first polymer layer, wherein the first polymer layer comprises a first compound composition comprising from greater than 0 wt% to less than 100 wt% of a first polymer comprising a polyether block amide, a polyurethane, or a combination thereof; less than about 65 % of an inorganic filler based on a total weight of the first compound composition; and up to about 20 % of a solid lubricant filler based on a total weight of the first compound composition; wherein the elongated tube is configured to reversibly expand from an initial diameter d₀ in an unexpended position to an expanded diameter dₑ in an expanded position upon passage of a medical device; and wherein the sheath exhibits at least a 10 % reduction in an insertion force when compared with a substantially identical reference sheath that does not comprise the first polymer layer.

It is understood, however, that also disclosed are aspects where the disclosed sheaths can comprise additional components. These exemplary aspects are disclosed herein, as shown below in detail.

In certain aspects, the elongated tube comprises a first polymer layer. In such exemplary aspects, the first polymer layer can comprise a first compound composition comprising from greater than 0 wt% to less than 100 wt%, including exemplary values of about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof.

In still further aspects, the first compound composition can comprise from greater than about 35 wt% to less than about 80 wt%, including exemplary values of about 40 wt%, about 45 wt%, about 50 wt%, about 55 wt%, about 60 wt%, about 65 wt%, about 70 wt%, and about 75 wt% of a polymer comprising a polyether block amide, a polyurethane, or any combination thereof.

In certain aspects, the polymer in the first compound composition comprises a polyether block amide. In such exemplary aspects, the polyether block amide can comprise PEBAX^{®} from Arkema. In yet further aspects, the polymer can comprise polyurethane, for example, NEUSoft^{®}. While in still further aspects, the polymer can compromise a combination of the polyether block amide, such as, for example, PEBAX^{®} and polyurethane. It is further understood that if the mixture of the polymers is present, such a mixture can comprise each component in any amount relative to another component to provide the desired polymer falling within the disclosed above range.

In still further aspects, the first compound composition can comprise less than about 65 wt% of an inorganic filler based on a total weight of the first compound composition, including exemplary values of less than about 60 wt%, less than about 55 wt%, less than about 50 wt%, less than about 45 wt%, less than about 40 wt%, less than about 35 wt%, less than about 30 wt%, less than about 25 wt%, less than about 20 wt%, less than about 15 wt%, less than about 10 wt%, less than about 5 wt%, and less than about 1 wt% of the inorganic filler.

In yet further aspects, the inorganic filler can be present in an amount of at least about 1 wt%, at least about 2 wt%, at least about 5 wt%, at least about 10 wt%, at least about 15 wt%, at least about 20 wt%, at least about 25 wt%, at least about 30 wt%, at least about 35 wt%, at least about 40 wt%, at least about 45 wt%, at least about 50 wt%, or at least about 55 wt%.

In still further aspects, the inorganic filler can comprise any inorganic materials that can be used as a filler and are acceptable for the desired application. In certain exemplary and unlimiting aspects, the inorganic filler can comprise bismuth oxychloride, barium sulfate, bismuth subcarbonate, calcium carbonate, aluminum trihydrate, barite, kaolin clay, limestone, or any combination thereof. Again it is understood that the inorganic filler can comprise a combination of the various fillers. In such exemplary aspects, an amount of each filler in the combination can be in any range to provide a final combination that falls within the disclosed above range.

In still further aspects, the first compound composition can comprise up to about 20 wt% of a solid lubricant filler based on a total weight of the first compound composition, including exemplary values of about 0.01 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, and about 19.9 wt%. In yet further aspects, the solid lubricant filler can be present up to about 20 wt%, up to about 15 wt%, or up to about 10 wt% based on a total weight of the first compound composition.

In still further aspects, the solid lubricant filler can comprise any additive that is known to reduce friction and behave as a lubricant. In such exemplary and unlimiting aspects, the solid lubricant filler can comprise one or more of graphene, reduced graphene oxide, carbon black, boron nitride, silicones, talc, polytetrafluorethylene (PTFE), fluorinated ethylene propylene, and the like. In still further aspects, the solid lubricant comprises a PTFE filler. In yet further aspects, the PTFE filler is a powder.

In still further aspects, the first compound composition can further comprise at least one tackiness reducing compound. Any compounds known in the art as capable of reducing the tackiness of the polymer composition can be considered and used for the purpose of this disclosure. In yet further exemplary and unlimiting aspects, the at least one tackiness reducing compound comprises ProPell^{™} from Foster Corporation

In certain aspects, the at least one tackiness reducing compound is present in an amount from 0 wt% to about 20 wt%, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the first compound composition. In still further aspects, the at least one tackiness reducing compound is present in any amount having a value between any two foregoing values. For example and without limitation, the at least one tackiness reducing compound can be present in an amount from about 1 wt% to about 5 wt%, or from about 5 wt% to about 10 wt% based on a total weight of the first compound composition.

In still further aspects and as disclosed herein, the first polymer has a substantially same durometer along a total length of the elongated tube. It is understood, however, the durometer of the first polymer of the elongated tube can also be varied along the length of the tube. For example, and without limitation, disclosed herein are aspects where a durometer of the first polymer at a proximal end of the elongated tube is different from a durometer of the first polymer at a distal end of the elongated tube.

In still further aspects, the first polymer in the first polymer layer has a Shore D from about 20D to about 72D, including exemplary values of about 25D, about 30D, about 35D, about 40D, about 45D, about 50D, about 55D, about 60D, about 65D, and about 70D. In still further aspects, the first polymer in the first polymer layer has a Shore D from about 20D to about 35D. In still further aspects, the first polymer in the first polymer layer has a Shore D of about 30D. Yet, in still further aspects, the first polymer in the first polymer layer has a Shore D of about 25D.

It is understood that the elongated tube, as disclosed herein, can comprise aspects where only one polymer layer is present. Yet, in other aspects, two or more polymer layers can be present in the elongated tube. In such exemplary aspects, the elongated tube comprises at least a second polymer layer comprising a second compound composition comprising from greater than 0 wt% to 100 wt% of a second polymer comprising polyether block amide, a polyurethane, or a composition thereof. Similar to the first compound composition, the second polymer can be present in any amount that falls within the disclosed range. For example, the second polymer can be present in the second compound composition from greater than 0 wt%, about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof. In yet further aspects, the second polymer can be present in the second compound composition from greater than about 95 wt% to less than about 99wt%, including exemplary values of about 95.5 wt %, about 96 wt%, 96.5 wt %, about 97 wt%, about 97.5 wt %, about 98 wt%, and about 98.5 wt%.

In yet further aspects, the second compound composition can further comprise up to 20 wt% of a tackiness reducing additive, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the second compound composition. In still further aspects, the at least one tackiness reducing compound is present in any amount having a value between any two foregoing values. For example and without limitation, the at least one tackiness reducing compound can be present in an amount from about 1 wt% to about 5 wt%, or from about 5 wt% to about 10 wt% based on a total weight of the second compound composition. In still further aspects and as disclosed herein, the second compound composition can be substantially free of a solid lubricant filler.

It is further understood that in certain aspects, the first polymer in the first compound composition can be the same as the second polymer in the second compound composition. Yet, in other aspects, the first polymer in the first compound composition is different from the second polymer in the second compound composition. In yet further aspects, the second polymer layer comprises PEBAX^{®}. While in further aspects, the second polymer layer can comprise polyurethane, for example, NEUSoft^{®} from PolyOne.

In still further aspects, the second polymer has a Shore D from about 20D to about 35D. Yet, in further aspects, the second polymer has a Shore D of about 25D or about 35D.

In still further aspects, the second compound composition can be substantially free of an inorganic filler. While in certain aspects, the inorganic filler can be present in the second compound composition in any amount from greater than 0 wt% to less than 100 wt%, including exemplary values of about 0.01 wt%, about 0.05 wt%, about 0.1 wt%, about 0.5 wt%, about 1 wt%, about 5 wt%, about 10 wt%, about 20 wt%, about 30 wt%, about 40 wt%, about 50 wt%, about 60 wt%, about 70 wt%, about 80 wt%, about 90 wt%, and about 95 wt%. In the aspects where the inorganic filler is present in the second compound composition, such inorganic filler can comprise any filler disclosed above.

In still further aspects, and as disclosed herein, the elongated tube has a predetermined thickness, and wherein at least about 10%, at least about 20%, at least about 30%, at least about 40%, at least about 50%, at least about 60%, at least about 70%, at least about 80%, at least about 90%, or 100% of the predetermined thickness comprises the first and/or the second compound composition comprising the first and/or the second polymer having a Shore D equal to or lower than about 30D.

In still further aspects, the predetermined thickness of the elongated tube can vary along a length of the sheath. While in other aspects, the predetermined thickness of the elongated tube is the same along a length of the sheath. Yet, in further aspects, the predetermined thickness of the elongated tube is greater at the proximal end. In still further aspects, the predetermined thickness of the elongated tube is up to 6 mils, for example, and without limitation from about 1 mil to about 6 mils, including exemplary values of about 1.5 mils, about 2 mils, about 2.5 mils, about 3 mils, about 3.5 mils, about 4 mils, about 4.5 mils, about 5 mils, about 5.5 mils, and about 5.9 mils.

In still further aspects, the first polymer layer and the second polymer layer can have the same thickness. While in other aspects, the first polymer layer and the second polymer layer have different thicknesses. For example, in some aspects, the first polymer layer has a thickness of about 1 mil to about 5 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, and about 4.9 mils. Yet still, in further aspects, the second polymer layer can have a thickness of about 2 mils to about 6 mils, including exemplary values of 2.1 mils, about 2.2 mils, about 2.3 mils, about 2.4 mils, about 2.5 mils, about 2.6 mils, about 2.7 mils, about 2.8 mils, about 2.9 mils, about 3.0 mils, 3.1 mils, about 3.2 mils, about 3.3 mils, about 3.4 mils, about 3.5 mils, about 3.6 mils, about 3.7 mils, about 3.8 mils, about 3.9 mils, about 4.0 mils, about 4.1 mils, about 4.2 mils, about 4.3 mils, about 4.4 mils, about 4.5 mils, about 4.6 mils, about 4.7 mils, about 4.8 mils, about 4.9 mils, about 5.1 mils, about 5.2 mils, about 5.3 mils, about 5.4 mils, about 5.5 mils, about 5.6 mils, about 5.7 mils, about 5.8 mils, and about 5.9 mils.

In still further aspects, the predetermined thickness of the elongated tube is greater at the proximal end. While in other aspects, the predetermined thickness of the elongated tube is smaller at the distal end as compared to the predetermined thickness of the elongated tube at the proximal end.

In still further aspects where two or more layers are present in the elongated tube, the first polymer layer can define the inner surface of the elongated tube, while the second polymer layer can define the outer surface of the elongated tube. However, there are also aspects, where the first polymer layer defines the outer surface of the elongated tube, while the second polymer layer defines the inner surface of the elongated tube. It is also understood that other aspects are also enclosed, where one or more additional polymer layers are disposed between the first polymer layer and the second polymer layer.

In still further aspects, the elongated tube can be extruded. In the aspects where the first and the second polymer layers are present, such polymer layers can be coextruded. In still further aspects, the first polymer layer can be substantially bonded to the second polymer layer. In such exemplary aspects, the first polymer layer substantially does not delaminate from the second polymer layer. It is understood that in some aspects, the bonding can be physical or chemical or any other type known in the art.

In still further aspects, any sheath that comprises the disclosed herein elongated tube can exhibit at least about 10% reduction, at least about 15% reduction, at least 20% reduction, at least about 25% reduction, at least about 30% reduction, at least about 35% reduction, at least about 40% reduction, at least about 45% reduction, or at least about 50% reduction in an insertion force when compared with a substantially identical reference sheath that does not comprise the first polymer layer.

In still further aspects, any sheath that comprises the disclosed herein elongated tube can exhibit an insertion force of less than about 55 N, less than about 50 N, less than about 45 N, less than about 40 N, less than about 35 N, or less than about 35 N when a medical device is pushed through the sheath.

In still further aspects, the elongated tube can also exhibit a friction force of less than about 10 N, or less than about 9 N, or less than about 8 N, or less than about 7 N, or less than about 6 N, or even less than about 5 N, in the dry state against a substrate surface comprising one or more of polytetrafluoroethylene, fluorinated ethylene propylene, or high-density polyethylene having a diameter of about 0.300".

In still further aspects, the elongated tube can exhibit a hoop force at 10 mm extension (about 85% strain) of less than about 10 N, or less than about 9 N., or less than about 8 N, or less than about 7 N, or less than about 6 N, or even less than about 5 N. In such exemplary aspects, the elongated tube can have a diameter of about 0.290" (7.4 mm) and wall thickness as disclosed herein. In aspects where the elongated tube has a diameter of about 0.290" (7.4 mm) and a total wall thickness of about 0.0045", with a sample length of about 0.25" (6.4 mm), a hoop direction forces at 10 mm extension can be less than about 8 N. It is understood that in some exemplary and unlimiting aspects, a low force at 10 mm extension is desired for low sheath expansion force.

In still further aspects, the elongated tube can exhibit an elongation at break of ranging between about 650 % and about 800 %, including exemplary values of about 680 %, about 700 %, about 710 %, about 750 %, and about 780 %. It is understood that in some exemplary and unlimiting aspects, a high elongation is preferable for expansion to a larger diameter before the elongated tube breaks.

In still further aspects, the elongated tube is substantially kink resistant.

In certain aspects, the elongated tube extends along a portion of the length of the sheath. In such exemplary aspects, the elongated tube can be positioned at the proximal end of the sheath, or in the middle of the sheath, or at the distal portion of the sheath. While in other aspects, the elongated tube extends along the whole length of the sheath. In such exemplary aspects, the elongated tube can be positioned at the proximal end of the sheath and extend to the distal end of the sheath.

In still further aspects, the sheath can further comprise an expandable tubular inner liner extending along the length of the sheath and comprising at least one folded portion, wherein the expandable inner liner has an inner surface and an outer surface, wherein the inner surface of the expandable inner liner defines a lumen and forms an inner surface of the at least one folded portion, and wherein the outer surface extends circumferentially to form an outer surface of the at least one folded portion; and a first outer tubular layer extending at least partially along the length of the sheath and having an inner surface and an outer surface, wherein the inner surface of the first outer tubular layer further extends at least partially around the outer surface of the inner liner such that at least a portion of the inner surface of the first outer tubular layer is positioned adjacent to the outer surface of the at least one folded portion of the inner liner; wherein the elongated tube is positioned such that at least a portion of the inner surface of the elongated tube overlies at least a portion of the outer surface of the first outer tubular layer.

In still further aspects, the elongated tube disclosed herein can comprise at least two polymer layers. In still further aspects, the elongated tube disclosed herein can comprise at least one intermediate reinforcement layer disposed between the first polymer layer and the second polymer layer. In still further aspects, the at least one intermediate reinforcement layer is a polymer layer.

In some aspects, the at least one intermediate layer can extend along the whole circumference of the elongated tube. In yet further aspects where the first polymer layer forms the inner surface of the elongated tube and the second polymer layer forms the outer surface of the elongated tube, the intermediate layer is disposed between the outer surface of the first polymer and the inner surface of the second polymer layer. Yet in other aspects, and as disclosed above, if the second polymer layer forms the inner surface of the elongated tube and the first polymer layer forms the outer surface of the elongated tube, the intermediate layer is disposed between the outer surface of the second polymer layer and the inner surface of the first polymer layer. In still further aspects, the intermediate reinforcement layer can bond the first and second polymer layers and can also assist in bonding the elongated tubing as a whole to an inner member of the sheath.

In still further aspects, the at least one intermediate layer has a finite width that is smaller than the circumference of the elongated tube. In such aspects, the at least one intermediate layer can be inserted as a strip between the first and the second polymer layers. In some exemplary and unlimiting aspects, if the elongated tube has a distal outer diameter of about 0.200", the strip can have a width between about 0.010" to about 0.150", including exemplary values of about 0.03", about 0.035", about 0.04", about 0.045", about 0.05", about 0.055", about 0.06", about 0.065", about 0.07", about 0.075", about 0.08", about 0.085", about 0.09", about 0.095", about 0.10", about 0.105", about 0.110", about 0.115", about 0.120", about 0.125", about 0.130", about 0.135", about 0.140", and about 0.145". It is understood that the widths shown above are exemplary, and if the distal outer diameter of the elongated sheath has a size different from 0.200", the strip width can be adjusted in the same or a different ratio.

In still further aspects, the at least one intermediate layer has a finite width that is smaller than the circumference of the elongated tube. In such aspects, the at least one intermediate layer can be inserted as a strip between the first and the second polymer layers. In some exemplary and unlimiting aspects, if the elongated tube has a distal outer diameter of about 0.200", the strip can have a width between about 5% to about 50% of the circumference of the elongated tube. In still further aspects, the total combined width of the strips is about 5%, about 15%, about 20%, about 25%, about 30%, about 35%, about 40%, about 45%, or about 50% of the circumference of the elongated tube. It is understood that the widths shown above are exemplary, and if the distal outer diameter of the elongated sheath has a size different from 0.200", the strip width can be adjusted in the same or a different ratio.

In still further aspects, the elongated tube can comprise two or more intermediate layers. In such aspects, the two or more intermediate layers can be disposed, as individual strips, circumferentially between the first and the second polymer layers at a predetermined distance from each other. In aspects where the two or more intermediate layers are disposed between the first and the second polymer layers of the elongated tube, a total combined width of all the strips is about 5% to about 50% of the circumference of the elongated tube. In still further aspects, the total combined width of the strips is about 5%, about 15%, about 20%, about 25%, about 30%, about 35%, about 40%, about 45%, or about 50% of the circumference of the elongated tube.

In still further aspects, the at least one intermediate layer is configured to provide an axial reinforcement to the elongated tube and, as a result, to the sheath where the elongated tube can be used. In such exemplary aspects, the at least one intermediate layer can be disposed along the length of the elongated tube or along a portion of the length of the elongated tube.

In some aspects, the portion of the length of the elongated tube where the at least one intermediate layer is disposed is positioned at the distal end and/or proximal end of the elongated tube. In yet other aspects, the at least one intermediate layer can also be positioned anywhere along the length of the elongated tube.

It is further understood that in the aspects where the intermediate layer is present as one or more strips disposed circumferentially along the length of the sheath, the width of the strip can be the same along the length, or it can vary along the length. In aspects where the strips' width varies along the length of the elongated tube, such a strip can have any of the disclosed above width values.

In still further aspects, the first polymer layer used in this exemplary elongated tube can be any of the first polymer layers described above. In still further exemplary and unlimiting aspects, the first polymer layer forms the inner surface of the elongated tube and comprises a first compound composition comprising from greater than 0 wt% to less than 100 wt% of a first polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition.

Any of the disclosed above inorganic fillers, and solid lubricant fillers can be present in any amount as disclosed. For example, the inorganic filler can comprise bismuth oxychloride, barium sulfate, bismuth subcarbonate, calcium carbonate, aluminum trihydrate, barite, kaolin clay, limestone, or any combination thereof. In yet other aspects, the inorganic filler can be present in at least 10 wt %. In still further aspects, the inorganic filler can be present in an amount of less than about 50 wt % based on a total weight of the first compound composition.

In yet further aspects, the solid lubricant filler can comprise a PTFE filler.

The first compound can also comprise any of the disclosed above additives. For example, the compound can comprise at least one tackiness reducing compound in an amount from about 1 wt% to about 20 wt%.

In still further exemplary aspects, the polymer present in the first compound can have Shore D from about 20D to about 35D, including exemplary values of about 22D, about 25D, about 27D, about 30D, and about 32D.

In yet further aspects, a durometer of the first polymer in the first polymer layer at a proximal end of the elongated tube can be different from a durometer of the first polymer in the first polymer layer at a distal end of the elongated tube.

In still further aspects, the polymer in the first compound can comprise poly ether block amide, for example, PEBAX^{®}. While in other aspects, the polymer in the first compound can comprise polyurethane. In still further aspects, the first compound can also comprise polyamide.

In still further aspects, the thickness of the first polymer layer can be from about 1 mil to about 5 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, and about 4.9 mils.

In still further aspects, the second polymer layer can comprise any of the disclosed above polymers. In some aspects, the second polymer layer can comprise a second compound composition comprising from greater than 0 wt% to 100 wt% of a second polymer comprising polyether block amide, a polyurethane, or a composition thereof. In still further aspects, the second polymer layer can comprise a polyamide. In yet some other aspects, the second compound can also comprise any of the fillers or additives disclosed above. While in some aspects, the second compound does not comprise the solid lubricant fillers disclosed herein. While in still further aspects, the second compound can comprise a tackiness reducing additive described in this disclosure. In some aspects, the second polymer can be a polyurethane. In still further aspects, the polyurethane is a thermoplastic polyurethane. While in still further aspects, the second polymer can be a blend comprising a polyurethane with a styrene block copolymer. In still further aspects, the blend can further comprise additional polymers and copolymers. For example, ether-based polymers can be present in the blend. In some exemplary and unlimiting aspects, the second polymer can be chosen from commercially available polymers sold under the trade name of Neusoft^{™}. In still further aspects, the second polymer can have a Shore A durometer from about 20A to about 75A, including exemplary values of about 25A, about 30A, about 35A, about 40A, about 45A, about 50A, about 55A, about 60A, about 65 A, and about 70A. In yet further aspects, the second polymer can have a Shore A durometer of less than 60A. In some exemplary aspects, the second polymer can be Neusoft^{™} 597-50A.

In still further aspects, the thickness of the second polymer layer can be from about 1 mil to about 6 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, about 4.9 mils, about 5.1 mils, about 5.2 mils, about 5.3 mils, about 5.4 mils, about 5.5 mils, about 5.6 mils, about 5.7 mils, about 5.8 mils, and about 5.9 mils. In still further aspects, the thickness of the at least one intermediate reinforcement layer can be anywhere between about 1 mil to about 6 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, about 4.9 mils, about 5.1 mils, about 5.2 mils, about 5.3 mils, about 5.4 mils, about 5.5 mils, about 5.6 mils, about 5.7 mils, about 5.8 mils, and about 5.9 mils.

In still further aspects, the at least one intermediate layer can comprise any of the polymers disclosed herein. In some aspects, the at least one intermediate layer can comprise the first compound disclosed above. Yet, in other aspects, the at least one intermediate layer can comprise the second compound disclosed above. While in still further aspects, the at least one intermediate layer can comprise the first compound. Yet, in still further aspects, the at least one intermediate layer can comprise any polymers that are known in the art and suitable for the desired application. In some aspects, the at least one intermediate layer can comprise polyether block amide, polyurethane, or a combination thereof. While in still further aspects, the at least one intermediate layer is a polyether block amide, for example, PEBAX^{®}. While in still further aspects, the intermediate layer is a polyurethane. In such exemplary aspects, the at least one intermediate layer does not comprise a solid lubricant filler, such as a PTFE. In yet other aspects, the at least one intermediate layer does not comprise an inorganic filler. In still further aspects, the at least one intermediate layer can comprise a polymer comprising PEBAX^{®} or polyurethane having a Shore D (or Shore A) durometer between about 45D (85A) to about 90D, including exemplary values of about 50D, about 55D, about 60D, about 65D, about 70D, about 72D, about 75D, about 80D, and about 85D.

In yet further aspects, the at least one intermediate reinforcement layer can comprise a polyolefin. In still further aspects, the at least one intermediate reinforcement layer can comprise a polyethylene, a polypropylene, a graft modified polyethylene or polypropylene. In yet further aspects, the at least one intermediate reinforcement layer can comprise the grafted low-density polyethylene (LDPE), grafted medium density polyethylene, grafted ultra-low-density polyethylene (ULDPE) grafted high density polyethylene (HDPE), grafted heterogeneously branched linear low-density polyethylene (LLDPE), grafted homogeneously branched linear ethylene polymers and substantially linear ethylene polymers, grafted polypropylene, or ethylene vinyl acetate (EVA), or any combination thereof. In such exemplary aspects, a maleic anhydride or an acrylic acid can be used to graft the disclosed above polymers. In still further aspects, the at least one intermediate reinforcement layer can comprise a maleic anhydride or an acrylic acid grafted low-density polyethylene. In yet further aspects, the at least one intermediate reinforcement layer can comprise a maleic anhydride or an acrylic acid grafted polypropylene. In still further aspects, the at least one intermediate reinforcement layer can comprise a maleic anhydride or an acrylic acid grafted ethylene vinyl acetate. In still further aspects, the at least one intermediate reinforcement layer can comprise a maleic anhydride grafted polyolefin sold under a trademark of OREVAC^{®}.

In still further aspects, any of the disclosed above at least one intermediate reinforcement layer can thermally bond the elongated tube to the inner member of the sheath. In still further aspects, the intermediate reinforcement layer can be extruded to be positioned between the first polymer layer and the second polymer layer. In still further aspects, the at least one intermediate reinforcement layer can be fused with the first and second polymer layers by at least one of heat or compression.

In still further aspects, the elongated tube as disclosed herein comprising the at least one intermediate reinforcement layer can exhibit an expansion force of less than about 50 N, less than about 49N, less than about 48N, less than about 47N, less than about 46N, less than about 45N, less than about 44N, less than about 43N, less than about 42N, less than about 41N, or even less than about 40N.

In still further aspects, the elongated tube as disclosed herein that comprises the at least one intermediate reinforcement layer can exhibit a burst pressure greater than about 8 psi, greater than about 8.5 psi, greater than about 9 psi, greater than about 9.5 psi, greater than about 10 psi, greater than about 10.5 psi, greater than about 11 psi, greater than about 11.5 psi, about 12 psi, greater than about 12.5 psi, greater than about 13 psi, greater than about 13.5 psi, greater than about 14 psi, greater than about 14.5 psi, or greater than about 15 psi.

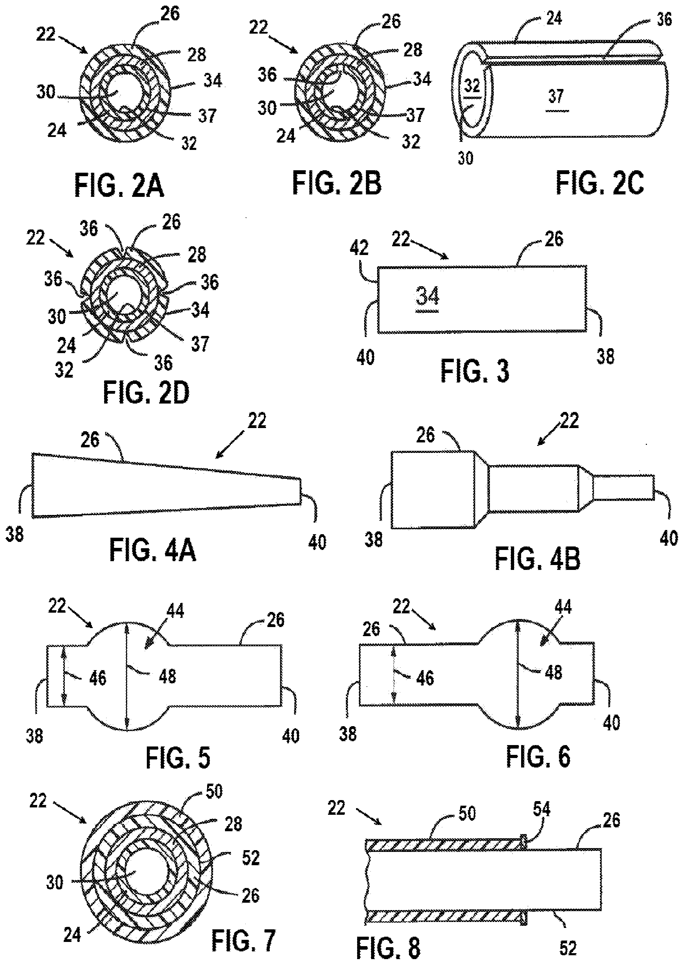

**FIGS. 2A, 2B,** and **2D** show section views of aspects of a sheath **22** for use with a delivery apparatus such as that shown in **FIG. 1****.** Example expandable sheaths are also disclosed in U.S. Patent Application No. 12/249,867, filed October 10, 2008 (now U.S. Patent No. 8,690,936), U.S. Patent Application No. 13/312,739, filed December 6, 2011 (now U.S. Patent No. 8,790,387), U.S. Patent Application No. 14/248,120 filed on April 8, 2014 (now U.S. Patent No. 9,301,840), U.S. Patent Application No. 14/324,894, filed July 7, 2014 (now U.S. Patent No. 9,301,841), U.S. Patent Application No. 15/057,953, filed March 1, 2016 (now U.S. Patent No. 9,987,134), U.S. Patent Application No. 15/997,587, filed June 4, 2018, U.S. Patent Application No. 16/149,953, filed on October 2, 2018 (now U.S. Patent No. 10,524,905), U.S. Patent Application No. 16/149,956, filed on October 2, 2018 (now U.S. Patent No. 10,517,720), U.S. Patent Application No. 16/149,960, filed on October 2, 2018 (now U.S. Patent No. 10,524,906, and U.S. Patent Application No. 16/149,969, filed on October 2, 2018 (now U.S. Patent No. 10,524,907).

**FIG. 2C** shows a perspective view of one aspect of an inner layer (or tubular inner liner) **24** for use with the sheath **22.** Sheath **22** includes an inner layer, such as inner polymeric tubular liner **24,** an outer layer, such as outer polymeric tubular layer **26.** Sheath can also include an intermediate tubular layer **28** disposed between the inner and outer polymeric tubular layers (liner) **24, 26.** The sheath **22** defines a lumen **30** through which a delivery apparatus can travel into a patient's vessel in order to deliver, remove, repair, and/or replace a prosthetic device. Such introducer sheaths **22** can also be useful for other types of minimally invasive surgery, such as any surgery requiring introduction of an apparatus into a subject's vessel. For example, the sheath **22** also can be used to introduce other types of delivery apparatus for placing various types of intraluminal devices (*e.g*., stents, stented grafts, etc.) into many types of vascular and non-vascular body lumens (*e.g*., veins, arteries, esophagus, ducts of the biliary tree, intestine, urethra, fallopian tube, other endocrine or exocrine ducts, etc.).

The outer polymeric tubular layer **26** and the inner polymeric tubular liner **24** can comprise, for example, polytetrafluoroethylene (PTFE) (e.g., Teflon^{®}), polyimide, PEEK, polyurethane, nylon, polyethylene, polyamide, polyether block amides (e.g., PEBAX^{®}), polyether block ester copolymer, polyesters, fluoropolymers, polyvinyl chloride, thermoset silicone, latex, poly-isoprene rubbers, polyolefin, other medical grade polymers, or combinations thereof. In yet other aspects, the outer tubular layer can also comprise a high-density polyethylene (HDPE).

The intermediate tubular layer **28** can comprise a shape memory alloy such as Nitinol and/or stainless steel, cobalt chromium, spectra fiber, polyethylene fiber, aramid fiber, or combinations thereof.

The inner polymeric tubular liner **24** can advantageously be provided with a low coefficient of friction on its inner surface. For example, the inner polymeric tubular liner **24** can have a coefficient of friction of less than about 0.5, less than about 0.1, less than about 0.05, or even less than about 0.01. Some aspects of a sheath **22** can include an additional lubricious liner on the inner surface **32** of the inner polymeric tubular liner **24.** Such a liner can facilitate the passage of a delivery apparatus through the lumen **30** of the sheath **22.** Examples of suitable lubricious liners include materials that can reduce the coefficient of friction of the inner polymeric tubular liner **24,** such as PTFE, polyethylene, polyvinylidene fluoride, and combinations thereof. Suitable materials for a lubricious liner also include other materials desirably having a coefficient of friction of less than about 0.5, less than about 0.1, less than about 0.05, or even less than about 0.01.

The inner diameter of the intermediate tubular layer **28** varies depending on the application and size of the delivery apparatus and prosthetic device. In some aspects, the inner diameter ranges from about 0.005 inches to about 0.400 inches, including exemplary values of about 0.01 inches, about 0.05 inches, about 0.100 inches, about 0.120 inches, about 0.150 inches, about 0.170 inches, about 0.200 inches, bout 0.220 inches, about 0.250 inches, about 0.270 inches, about 0.300 inches, bout 0.320 inches, about 0.350 inches, and about 0.370 inches.

The thickness of the intermediate tubular layer **28** can be varied depending on the desired amount of radial expansion, as well as the strength required. For example, the thickness of the intermediate tubular layer **28** can be from about 0.002 inches to about 0.0025 inches, including exemplary values of about 0.003 inches, about 0.004 inches, about 0.005 inches, about 0.006 inches, about 0.007 inches, about 0.008 inches, 0.009 inches, about 0.010 inches, about 0.011 inches, about 0.012 inches, about 0.013 inches, about 0.014 inches, about 0.015 inches, about 0.016 inches, about 0.017 inches, about 0.018 inches, 0.019 inches, about 0.020 inches, about 0.021 inches, about 0.022 inches, about 0.023 inches, and about 0.024 inches.

The thicknesses of the inner polymeric tubular liner **24** and the outer polymeric tubular layer **26** can also be varied depending on the particular application of the sheath **22.** In some aspects, the thickness of the inner polymeric tubular liner **24** ranges from about 0.0005 inches to about 0.010 inches, including exemplary values of about 0.0006 inches, about 0.0007 inches, about 0.0008 inches, about 0.0009 inches, about 0.001 inches, about 0.002 inches, 0.003 inches, about 0.004 inches, about 0.005 inches, about 0.006 inches, about 0.007 inches, about 0.008 inches, and about 0.009 inches. In one aspect, the thickness can be about 0.002 inches. Outer polymeric tubular layers **26** can have a thickness of from about 0.002 inches to about 0.015 inches, including exemplary values of about 0.003 inches, about 0.004 inches, about 0.005 inches, about 0.006 inches, about 0.007 inches, about 0.008 inches, 0.009 inches, about 0.010 inches, about 0.011 inches, about 0.012 inches, about 0.013 inches, and about 0.014 inches. In certain aspects, the outer polymeric tubular layer **26** can have a thickness of about 0.010 inches.

The hardness of each layer of the sheath **22** can also be varied depending on the particular application and desired properties of the sheath **22.** In some aspects, the outer polymeric tubular layer **26** has a Shore D durometer of about 25D to about 75D.

Additionally, some aspects of a sheath **22** can include an exterior hydrophilic coating on the outer surface **34** of the outer polymeric tubular layer **26.** Such a hydrophilic coating can facilitate the insertion of the sheath **22** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings, are also suitable for use with the sheath **22.**

In some aspects, the outer surface **34** of the outer polymeric tubular layer **26** can be modified. For example, surface modifications such as plasma etching can be performed on the outer surface **34.** Similarly, other surfaces, both outer and inner, can be surface modified according to certain aspects and desired application. In some aspects, surface modification can improve adhesion between the layers in the areas of the modification.

In certain aspects, the outer surface of the first outer tubular layer can be at least partially etched. In yet still, further aspects, the outer surface of the inner liner can be selectively etched around the circumference, linearly along at least a portion of the length of the sheath, or a combination thereof.

The sheath **22** also can have at least one radiopaque filler or marker. The radiopaque filler or marker can be associated with the outer surface 34 of the outer polymeric tubular layer **26.** Alternatively, the radiopaque filler or marker can be embedded or blended within the outer polymeric tubular layers **26.** Similarly, the radiopaque filler or marker can be associated with a surface of the inner polymeric tubular liner **24** or the intermediate tubular layer **28** or embedded within either or both of those layers.

Suitable materials for use as a radiopaque filler or marker include, for example, barium sulfite, bismuth trioxide, titanium dioxide, bismuth subcarbonate, or combinations thereof. The radiopaque filler can be mixed with or embedded in the material used to form the outer polymeric tubular layer **26** and can comprise from about 5% to about 45% by weight, including exemplary values of about 10% by weight, about 15% by weight, about 20% by weight, about 30% by weight, about 35% by weight, and about 40% by weight of the outer polymeric tubular layer. More or less radiopaque material can be used in some aspects, depending on the particular application.

In some aspects, the inner polymeric tubular liner **24** can comprise a substantially uniform cylindrical tube. In alternative aspects, the inner polymeric tubular liner **24** can have at least one section of discontinuity along its longitudinal axis to facilitate radial expansion of the inner polymeric tubular liner **24.** For example, the inner polymeric tubular liner **24** can be provided with one or more longitudinal notches and/or cuts **36** extending along at least a portion of the length of the sheath **22.** Such notches or cuts **36** can facilitate radial expansion of the inner polymeric tubular liner **24,** thus accommodating passage of a delivery apparatus or other device. Such notches and/or cuts 36 can be provided near the inner surface **32,** near the outer surface **37,** and/or substantially through the entire thickness of the inner polymeric tubular liner **24.** In aspects with a plurality of notches and/or cuts **36,** such notches and/or cuts **36** can be positioned such that they are substantially equally spaced from one another circumferentially around the inner polymeric tubular liner **24.** Alternatively, notches and cuts **36** can be spaced randomly in relation to one another or any other desired pattern. Some or all of any provided notches and/or cuts **36** can extend longitudinally along substantially the entire length of the sheath **22.** Alternatively, some or all of any provided notches and/or cuts **36** can extend longitudinally only along a portion of the length of the sheath **22.**

As shown in **FIGS. 2B** and **2C** (which illustrates only the inner polymeric tubular liner **24**), in some aspects, the inner polymeric tubular liner **24** contains at least one notch or cut **36** that extends longitudinally and parallel to an axis defined by the lumen **30,** extending substantially the entire length of the sheath **22**. Thus, upon introduction of a delivery apparatus, the inner polymeric tubular liner **24** can split open along the notch and/or cut **36** and expand, thus accommodating the delivery apparatus.

Additionally or alternatively, as shown in **FIG. 2D****,** the outer polymeric tubular layer **26** can comprise one or more notches and/or cuts **36**. Notches and/or cuts **36**, in some aspects, do not extend through the entire thickness of the outer polymeric tubular layer **26**. The notches and/or cuts **36** can be separable upon radial expansion of the sheath **22**. The outer polymeric tubular layer **26** can be retractable longitudinally or able to be pulled back away from the intermediate tubular layer **28** and the inner polymeric tubular liner **24**. In aspects with a retractable outer polymeric tubular layer **26**, the outer polymeric tubular layer **26** can be retracted to accommodate or facilitate passage of a delivery apparatus through the lumen and then can be replaced to its original position on the sheath **22**.

**FIG. 3** illustrates an elevation view of the sheath **22** shown in **FIG. 2A**. In this view, only the outer polymeric tubular layer **26** is visible. The sheath **22** comprises a proximal end **38** and a distal end **40** opposite the proximal end **38**. The sheath **22** can include a hemostasis valve inside the lumen of the sheath **22**, at or near the proximal end **38** of the sheath **22**.

Additionally, the sheath **22** can comprise a soft tip **42** at the distal end **40** of the sheath **22**. Such a soft tip **42** can be provided with a lower hardness than the other portions of the sheath **22**. In some aspects, the soft tip **42** can have a Shore hardness from about 25D to about 40D, including polymers having a Shore hardness of about 30D or about 35D.

As shown in **FIG. 3**, the unexpanded original outer diameter of the sheath **22** can be substantially constant across the length of the sheath **22**, substantially from the proximal end **38** to the distal end **40**. In alternative aspects, such as the ones illustrated in **FIGS. 4A-4B****,** the original unexpanded outer diameter of the sheath **22** can decrease from the proximal end **38** to the distal end **40**. As shown in the aspect in **FIG. 4A****,** the original unexpanded outer diameter can decrease along a gradient, from the proximal end **38** to the distal end **40**. In alternative aspects, such as the one shown in **FIG. 4B****,** the original unexpanded outer diameter of sheath **22**, can incrementally step down along the length of the sheath **22,** wherein the largest original unexpanded outer diameter is near the proximal end **38,** and the smallest original unexpanded outer diameter is near the distal end **40** of the sheath **22**.

As shown in **FIGS. 5-6****,** the sheath **22** can be designed to locally expand as the prosthetic device is passed through the lumen of the sheath **22** and then substantially return to its original shape once the prosthetic device has passed through that portion of the sheath **22.** For example, **FIG. 5** illustrates a sheath **22** having a localized bulge **44,** representative of a device being passed through the internal lumen of the sheath **22.** **FIG. 5** shows the device close to the proximal end **38** of the sheath **22**, close to the area where the device is introduced into the sheath **22.** **FIG. 6** shows the sheath **22** of **FIG. 5****,** with the device having progressed further along the sheath **22**. The localized bulge **44** is now closer to the distal end **40** of the sheath **22**, and thus, is about to be introduced to a patient's vessel. As evident from **FIGS. 5** and **6****,** once the localized bulge associated with the device has passed through a portion of the lumen of the sheath **22**, that portion of the sheath **22** can automatically return to its original shape and size, at least in part due to the materials and structure of the sheath b.

The sheath **22** has an unexpanded inner diameter equal to the inner diameter of the inner polymeric tubular liner (not visible in **FIG**S. **5-6**) and an unexpanded outer diameter **46** equal to the outer diameter of the outer polymeric tubular layer **26**. The sheath **22** is designed to be expanded to an expanded inner diameter and an expanded outer diameter **48,** which are larger than the unexpanded inner diameter and the unexpanded outer diameter **46,** respectively. In one representative aspect, the unexpanded inner diameter is about 16 Fr, and the unexpanded outer diameter **46** is about 19 Fr, while the expanded inner diameter is about 26 Fr, and the expanded outer diameter **48** is about 29 Fr. Different sheaths **22** can be provided with different expanded and unexpanded inner and outer diameters, depending on the size requirements of the delivery apparatus for various applications. Additionally, some aspects can provide more or less expansion depending on the particular design parameters, the materials, and/or configurations used.

In some aspects of a sheath according to the present disclosure, and as shown in section in **FIG. 7** and in elevation in **FIG. 8****,** the sheath **22** comprises an outer covering (or a second outer layer) **50,** formed by the elongated tube as disclosed herein. In such aspects, the elongated tube can be positioned such that at least a portion of the inner surface of the elongated tube overlies at least a portion of the outer surface **52** of the first outer tubular layer **26**. In such aspects, where the elongated tube **50** is present, and it forms the outer covering (or the most outer layer), the outer tubular layer **26** can also be referred to as a first outer tubular layer **26,** while the elongated tube can be referred to as a second. The outer polymeric covering **50** can provide a protective covering for the underlaying sheath **22**. In some aspects, the outer polymeric covering **50** can contain a self-expandable sheath in a crimped or constrained state and then release the self-expandable sheath upon removal of the outer polymeric covering **50.**

For example, in some aspects of a self-expandable sheath, the intermediate tubular layer **28** can comprise Nitinol and/or other shape memory alloys, and the intermediate tubular layer **28** can be crimped or radially compressed to a reduced diameter within the outer polymeric tubular layer **26** and the outer polymeric covering **50.** Once the self-expandable sheath is at least partially inserted into a patient's vessel, the outer polymeric covering **50** can be slid back, peeled away, or otherwise at least partially removed from the sheath. To facilitate removal of the outer polymeric covering 50, a portion of the outer polymeric covering **50** can remain outside the patient's vessel, and that portion can be pulled back or removed from the sheath to allow the sheath to expand. In some aspects, substantially, the entire outer polymeric covering (the elongated tube as disclosed herein) **50** can be inserted, along with the sheath, into a patient's vessel. In these aspects, an external mechanism attached to the outer polymeric covering **50** can be provided, such that the outer polymeric covering can be at least partially removed from the sheath once the sheath is inserted into a patient's vessel.

In some aspects, once no longer constrained by the outer polymeric covering **50,** the radially compressed intermediate layer **28** can self-expand, causing expansion of the sheath along the length of the intermediate tubular layer **28**. In some aspects, portions of the sheath can radially collapse, at least partially returning to the original crimped state, as the sheath is being withdrawn from the vessel after completion of the surgical procedure. In some aspects, such collapse can be facilitated and/or encouraged by an additional device or layer that, in some aspects, can be mounted onto a portion of the sheath prior to the sheath's insertion into the vessel.

The outer polymeric covering **50,** in some aspects, is not adhered to the other layers of the sheath **22**. For example, the outer polymeric covering **50** may be slidable with respect to the underlaying sheath, such that it can be easily removed or retracted from its initial position on the sheath **22**.

Suitable materials for the outer polymeric covering **50** are disclosed in detail above.

Turning now to the intermediate tubular layer **28,** several different configurations are possible. The intermediate tubular layer **28** is generally a thin, hollow, substantially cylindrical tube comprising an arrangement, pattern, structure, or configuration of wires or struts, however, other geometries can also be used. The intermediate tubular layer 28 can extend along substantially the entire length of the sheath **22,** or alternatively, can extend only along a portion of the length of sheath **22**. Suitable wires can be round, ranging from about 0.0005 inches thick to about 0.10 inches thick, or flat, ranging from about 0.0005 inches x 0.003 inches to about 0.003 inches x 0.007 inches. However, other geometries and sizes are also suitable for certain aspects. If a braided wire is used, the braid density can be varied. Some aspects have a braid density of from about thirty picks per inch to about eighty picks per inch and can include up to thirty-two wires in various braid patterns.

One representative aspect of an intermediate tubular layer comprises a braided Nitinol composite, which is at least partially encapsulated by an inner polymeric tubular member and an outer polymeric tubular member disposed on inner and outer surfaces of the intermediate tubular layer, respectively. Such encapsulation by polymeric layers can be accomplished by, for example, fusing the polymeric layers to the intermediate tubular layer or dip coating the intermediate tubular layer. In some aspects, an inner polymeric tubular member, an intermediate tubular layer, and an outer polymeric tubular layer can be arranged on a mandrel, and the layers can then be thermally fused or melted into one another by placing the assembly in an oven or otherwise heating it. The mandrel can then be removed from the resulting sheath. In other aspects, dip coating can be used to apply an inner polymeric tubular member to the surface of a mandrel. The intermediate tubular layer can then be applied, and the inner polymeric tubular member allowed to cure. The assembly can then be dip coated again, such as to apply a thin coating of, for example, polyurethane, which will become the outer polymeric tubular member of the sheath. The sheath can then be removed from the mandrel.

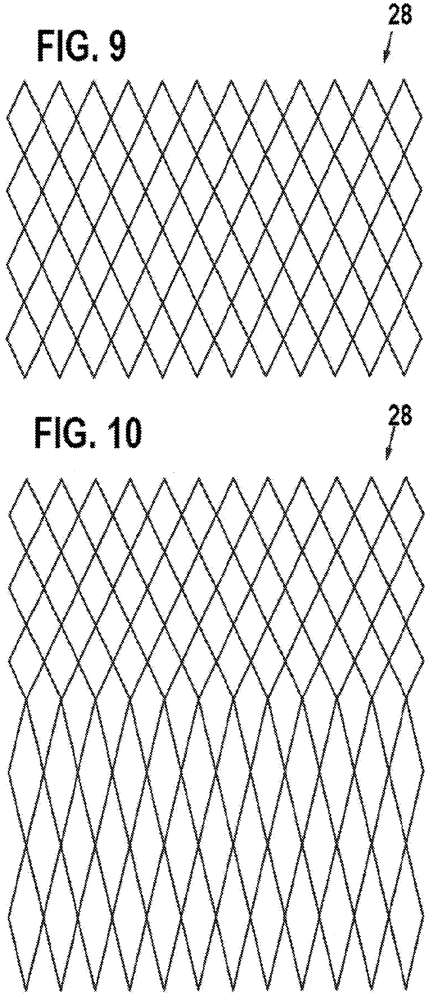

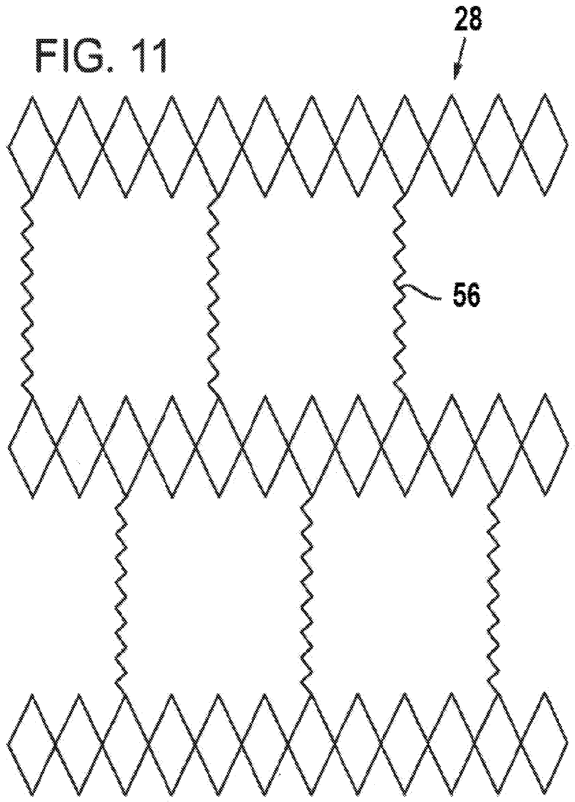

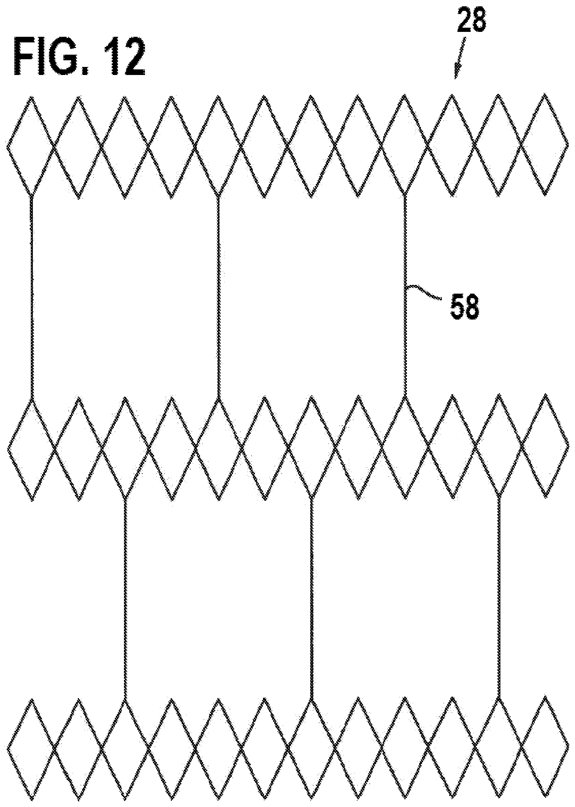

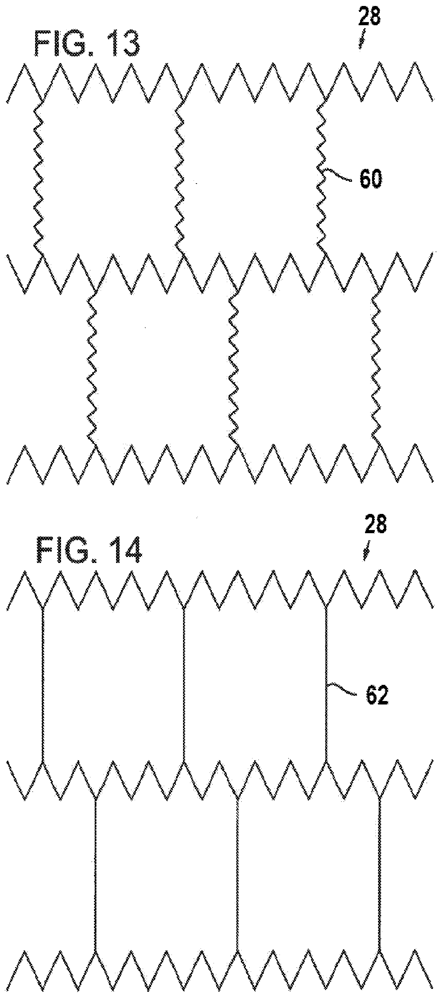

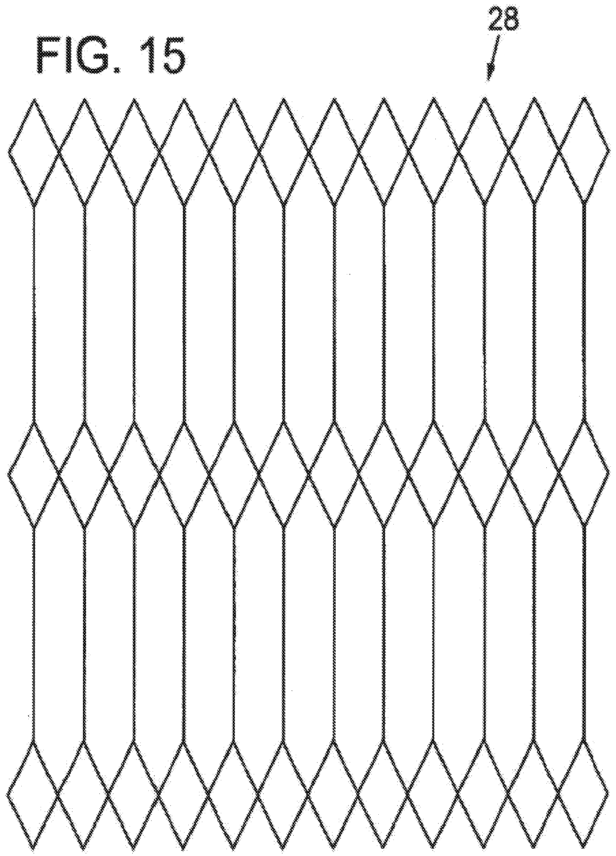

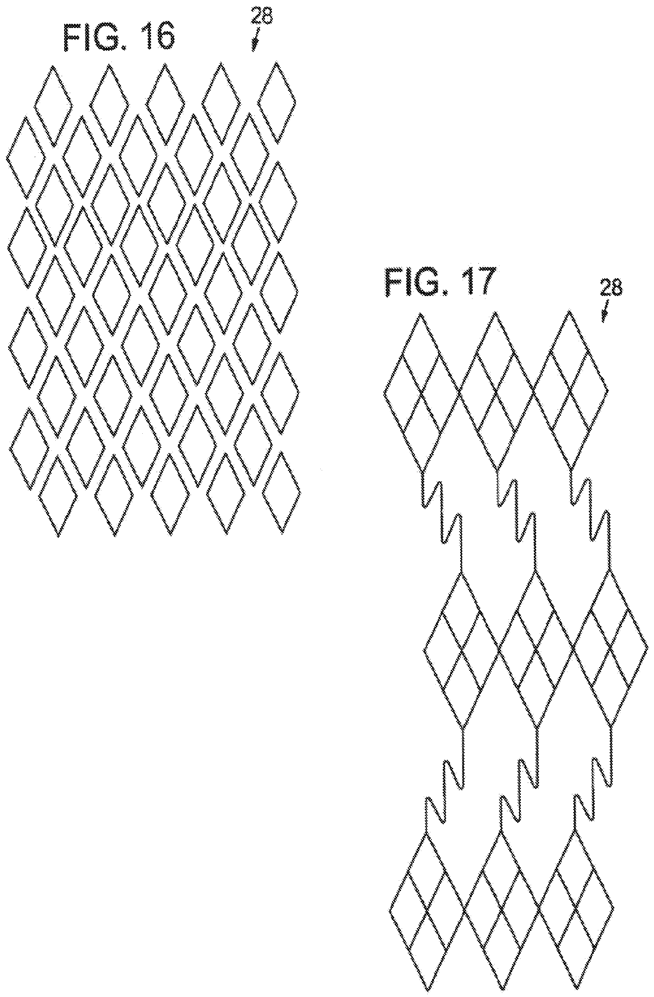

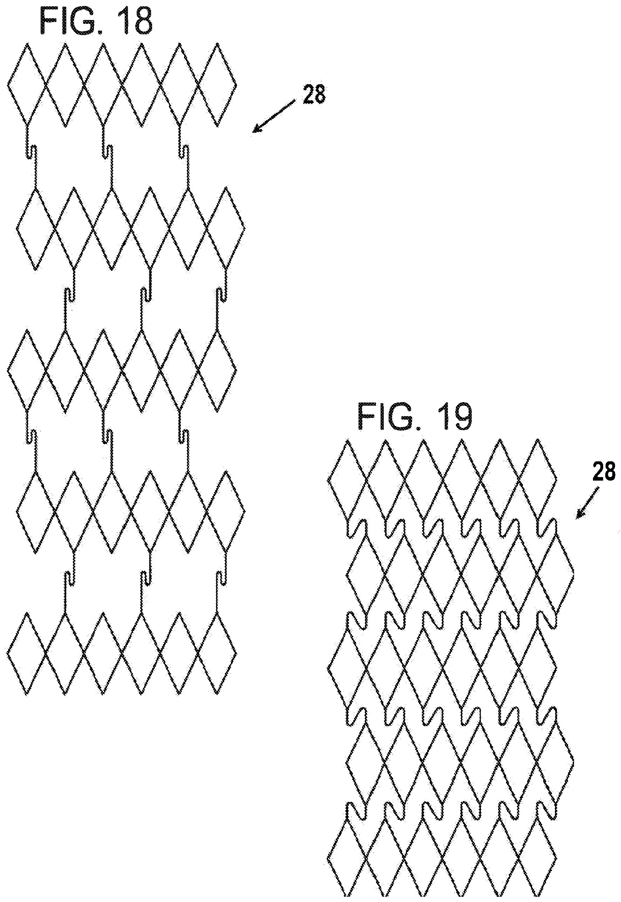

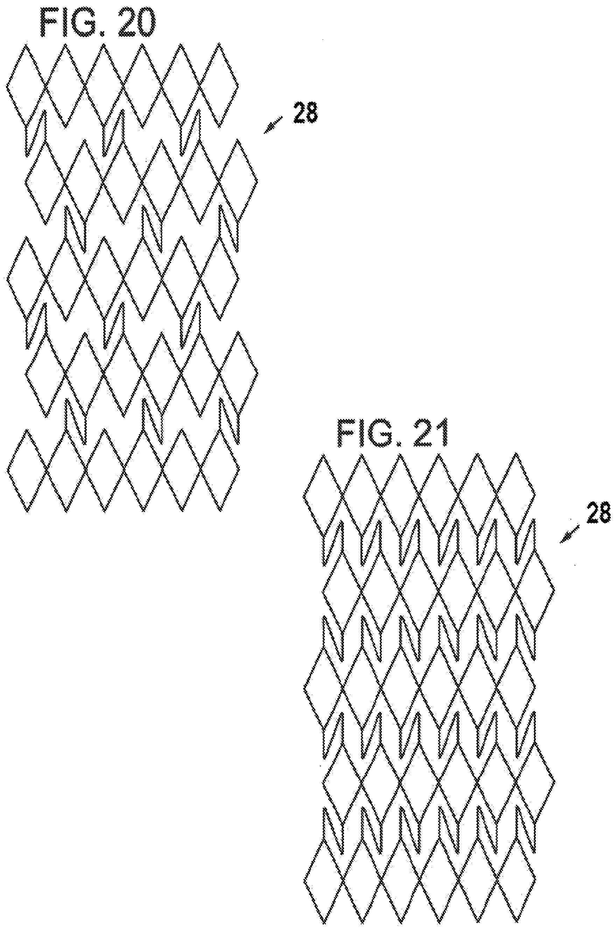

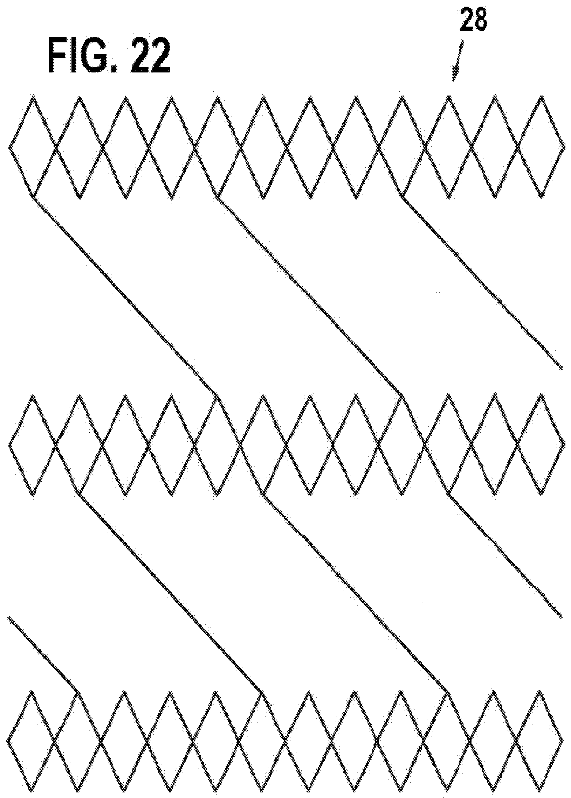

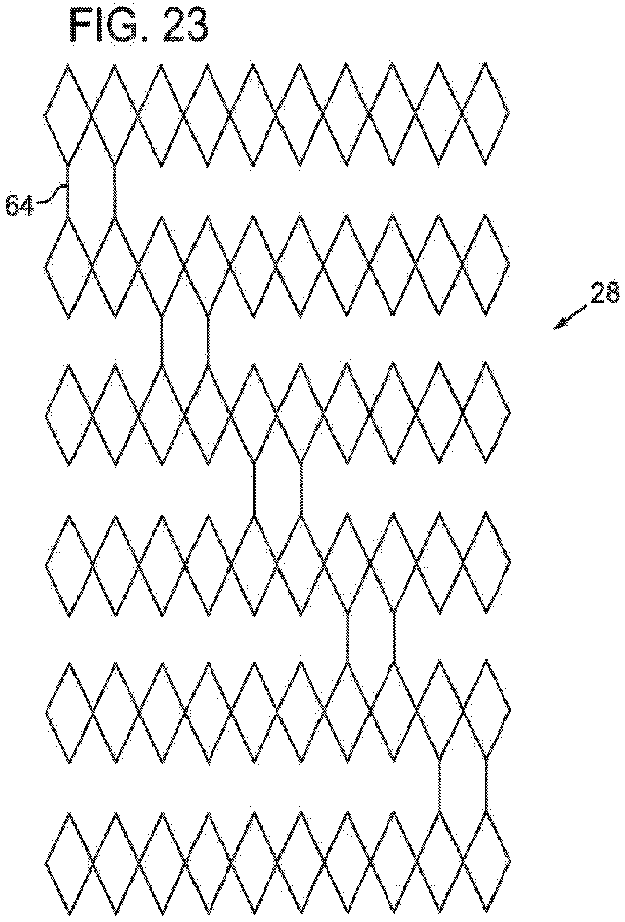

Additionally, the intermediate tubular layer **28** can be, for example, braided or laser cut to form a pattern or structure, such that the intermediate tubular layer **28** is amenable to radial expansion. **FIGS. 9-23** illustrate partial elevation views of various structures for the intermediate tubular layer. Some illustrated structures, such as those shown in **FIGS. 11-14** and **23,** including at least one discontinuity. For example, the struts **56, 58, 60, 62, 64,** shown in **FIGS. 11****,** **12****,** **13, 14****,** and **23****,** respectively, resulting in a discontinuous intermediate tubular layer **28** in that the struts **56, 58, 60, 62, 64** separate adjacent sections of the intermediate tubular layer **28** from each other, where the sections are spaced apart from each other along a longitudinal axis parallel to the lumen of the sheath. Thus, the structure of the intermediate tubular layer **28** can vary from section to section, changing along the length of the sheath.

The structures shown in **FIGS. 9-23** are not necessarily drawn to scale. Components and elements of the structures can be used alone or in combination within a single intermediate tubular layer **28.** The scope of the intermediate tubular layer **28** is not meant to be limited to these particular structures; they are merely exemplary aspects.

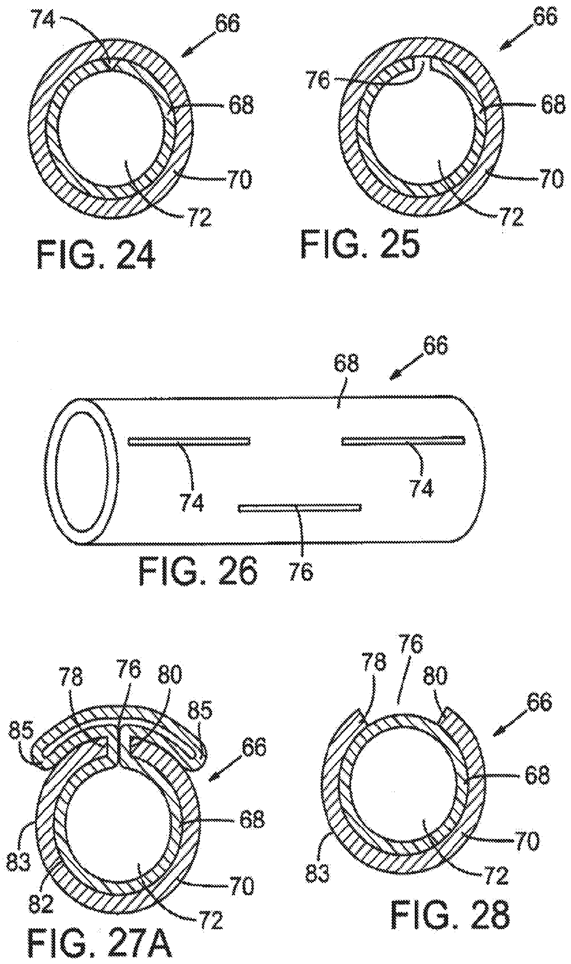

Alternative aspects of a sheath for introducing a prosthetic device are also described. For example, **FIGS. 24-26** illustrate a section view and a perspective view, respectively, of a sheath **66** for introducing a prosthetic device into a body. The sheath **66** comprises an inner layer, such as inner polymeric liner **68,** an outer layer, such as outer polymeric tubular layer **70,** and a hemostasis valve (not shown). The inner polymeric liner **68** and the outer polymeric tubular layer **70** at least partially enclose a lumen **72,** through which a delivery apparatus and prosthetic device can pass from outside the patient's body into the patient's vessel. Either or both of the inner polymeric liner **68** and the outer polymeric layer **70** can be provided with at least one longitudinal notch and/or cut to facilitate radial expansion of the sheath.

For example, **FIG. 24** illustrates a longitudinal notch **74** in the inner polymeric liner **68** that can facilitate radial expansion of the sheath **66.** The longitudinal notch **74** can separate or split open completely upon application of a radial force due to insertion of a delivery apparatus or prosthetic device. Similarly, **FIG. 25** illustrates a longitudinal cut **76** in the inner polymeric liner **68** that can also facilitate radial expansion of the sheath **66**. The outer polymeric layer **70** can, additionally or alternatively, comprise one or more longitudinal cuts **76** or notches **74**. Such cuts and/or notches, whether in the inner polymeric liner **68** or the outer polymeric layer **70,** can extend substantially through the entire thickness of the layer or can extend only partially through the thickness of the layer. The cuts and/or notches can be positioned at or near the inner or outer surface, or both surfaces, of the inner and/or outer polymeric layers **68, 70**.

**FIG. 26** illustrates a perspective view of one aspect of an inner polymeric liner **68** with longitudinal notches **74** and a longitudinal cut **76**. More or fewer notches **74** and/or cuts **76** can be provided. For clarity, the outer polymeric layer **70** is not shown in **FIG. 26****.** As shown in **FIG. 26****,** longitudinal notches **74** and/or cuts **76** can extend only along a portion of the length of sheath **66**. In alternative aspects, one or more notches **74** and/or cuts **76** can extend substantially along the entire length of the sheath **66.** Additionally, notches **74** and/or cuts **76** can be positioned randomly or patterned.

One particular aspect of a sheath **66** comprises a sheath having a notch or cut in the outer polymeric layer **70** or the inner polymeric layer (liner) **68** that extends longitudinally along approximately 75% of the length of the sheath **66**. If such a notch or cut extends only partially through the associated layer, it can have a relatively low tear force, such as a tear force of about 0.5 Ibs., so that the notch splits open relatively easily during use.

The inner polymeric liner **68** and the outer polymeric layer **70** can optionally be adhered together or otherwise physically associated with one another. The amount of adhesion between the inner polymeric liner **68** and the outer polymeric layer **70** can be variable over the surfaces of the layers. For example, little to no adhesion can be present at areas around or near any notches and/or cuts present in the layers so as not to hinder radial expansion of the sheath **66**. Adhesion between the layers can be created by, for example, thermal bonding and/or coatings. Aspects of a sheath **66** can be formed from an extruded tube, which can serve as the inner polymeric layer (liner) **68.** The inner polymeric layer (liner) **68** can be surface-treated, such as by plasma etching, chemical etching, or other suitable methods of surface treatment. By treating the surface of the inner polymeric liner **68,** the outer surface of the inner polymeric liner **68** can have areas with altered surface angles that can provide better adhesion between the inner polymeric liner **68** and the outer polymeric layer **70**. The treated inner polymeric liner can be dip coated in, for example, a polyurethane solution to form the outer polymeric layer **70**. In some configurations, the polyurethane may not adhere well to untreated surface areas of the inner polymeric liner **68**. Thus, by surface treating only surface areas of the inner polymeric liner **68** that are spaced away from the areas of expansion (*e.g*., the portion of the inner polymeric liner **68** near notches **74** and/or cuts **76**), the outer polymeric layer **70** can be adhered to some areas of the inner polymeric liner **68,** while other areas of the inner polymeric liner **68** remain free to slide relative to the outer polymeric layer **70,** thus allowing for expansion of the diameter of the sheath **66.** Thus, areas around or near any notches **74** and/or cuts **76** can experience little to no adhesion between the layers, while other areas of the inner and outer polymeric layers **68, 70** can be adhesively secured or otherwise physically associated with each other.

As with previously disclosed aspects, the aspects illustrated in **FIGS. 24-26** can be applied to sheaths having a wide variety of inner and outer diameters. Applications can utilize a sheath of the present disclosure with an inner diameter of the inner polymeric liner **68** that is expandable to an expanded diameter of from about 3 Fr to about 26 Fr, including exemplary values of about 5 Fr, about 10 Fr, about 15 Fr, about 20 Fr, and about 25 Fr. The expanded diameter can vary slightly along the length of the sheath **66**. For example, the expanded outer diameter at the proximal end of the sheath **66** can range from about 3 Fr to about 28 Fr, including exemplary values of about 5 Fr, about 10 Fr, about 15 Fr, about 20 Fr, and about 25 Fr, while the expanded outer diameter at the distal end of the sheath **66** can range from about 3 Fr to about 25 Fr, including exemplary values of about 5 Fr, about 10 Fr, about 15 Fr, about 20 Fr, and about 25 Fr. Aspects of a sheath **66** can expand to an expanded outer diameter that is from about 10% greater than the original unexpanded outer diameter to about 100% greater than the original unexpanded outer diameter.

In some aspects, the outer diameter of the sheath **66** gradually decreases from the proximal end of the sheath **66** to the distal end of the sheath **66**. For example, in one aspect, the outer diameter can gradually decrease from about 26 Fr at the proximal end to about 18 Fr at the distal end. The diameter of the sheath **66** can transition gradually across substantially the entire length of the sheath **66.** In other aspects, the transition or reduction of the diameter of the sheath **66** can occur only along a portion of the length of the sheath **66**. For example, the transition can occur along a length from the proximal end to the distal end, where the length can range from about 0.5 inches to about the entire length of sheath **66**.

Suitable materials for the inner polymeric liner **68** can have high elastic strength and include materials discussed in connection with other aspects, especially Teflon (PTFE), polyethylene (*e.g*., high density polyethylene), fluoropolymers, or combinations thereof. In some aspects, the inner polymeric liner **68** preferably has a low coefficient of friction, such as a coefficient of friction of from about 0.01 to about 0.5, including exemplary values of about 0.05, about 0.1, about 0.15, about 0.2, about 0.25, about 0.3, about 0.35, about 0.4, and about 0.45. In yet some other aspects of a sheath, **66** can comprise an inner polymeric liner **68** having a coefficient of friction of about 0.1 or less, or 0.05 or less.

Likewise, suitable materials for the outer polymeric layer **70** include materials discussed in connection with other aspects and other thermoplastic elastomers and/or highly elastic materials.

The Shore hardness of the outer polymeric layer **70** can be varied for different applications and aspects. Some aspects include an outer polymeric layer with a Shore hardness of from about 25A to about 80A, including exemplary values of about 30A, about 35A, about 40A, about 45A, about 50A, about 55A, about 60A, about 65A, about 70A, and about 80A. Yet, in other aspects, an outer polymeric layer can have a Shore hardness of from about 20D to about 40D, including exemplary values of about 25D, about 30D, and about 35D. One aspect comprises a readily available polyurethane with a Shore hardness of 72A. Another particular aspect comprises a polyethylene inner polymeric layer dipped in polyurethane or silicone to create the outer polymeric layer.

The sheath **66** can also include a radiopaque filler or marker, as described above. In some aspects, a distinct radiopaque marker or band can be applied to some portion of the sheath **66**. For example, a radiopaque marker can be coupled to the inner polymeric liner **68,** the outer polymeric layer **70,** and/or can be positioned in between the inner and outer polymeric layers **68, 70**.

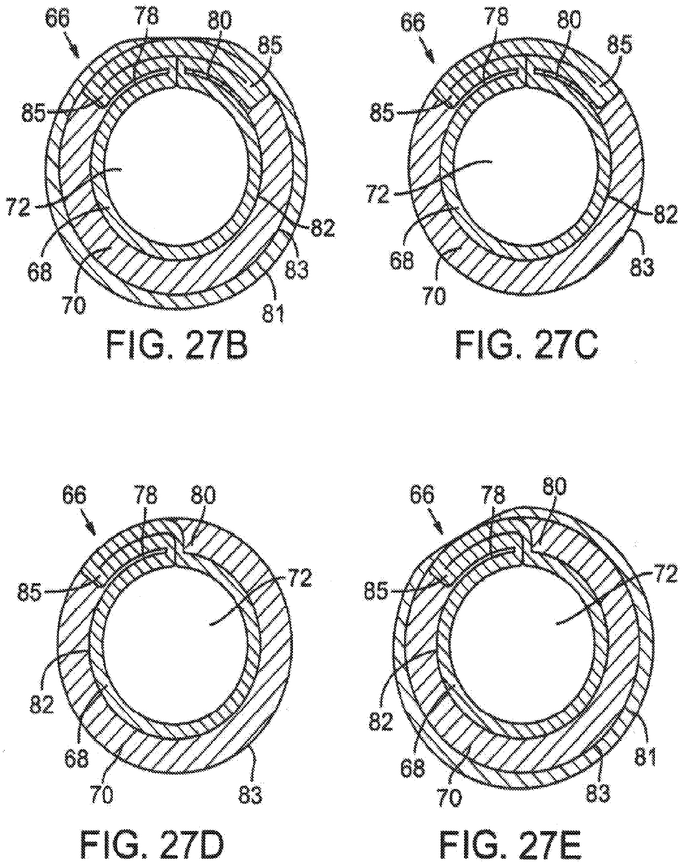

**FIGS. 27A-27E** and **28** illustrate section views of various aspects of unexpanded **(****FIGS. 27A-27E****)** and expanded (**FIG. 28**) sheaths **66** according to the present disclosure. The sheath **66** includes a split outer polymeric tubular layer **70** having a longitudinal cut **76** through the thickness of the outer polymeric tubular layer **70** such that the outer polymeric tubular layer **70** comprises a first portion **78** and a second portion **80** separable from one another along the cut **76**. An expandable inner polymeric liner **68** is associated with an inner surface **82** of the outer polymeric tubular layer **70,** and, in the unexpanded configuration shown in **FIG. 27A****,** a portion of the inner polymeric liner **68** extends through a gap created by the cut **76** and can be compressed between the first and second portions **78, 80** of the outer polymeric tubular layer **70**. Upon expansion of the sheath **66,** as shown in **FIG. 28****,** first and second portions **78,** 80 of the outer polymeric tubular layer **70** have separated from one another, and the inner polymeric liner **68** is expanded to a substantially cylindrical tube. In some aspects, two or more longitudinal cuts **76** may be provided through the thickness of the outer polymeric tubular layer **70**. In such aspects, a portion of the inner polymeric liner **68** may extend through each of the longitudinal cuts **76** provided in the outer polymeric tubular layer **70**.

In certain aspects, the inner polymeric liner **68** comprises one or more materials that are elastic and amenable to folding and/or pleating. For example, **FIG. 27A** illustrates an inner polymeric liner **68** with folded regions **85**. As seen in **FIGS. 27A-****27E,** the sheath **66** can be provided with one or more folded regions **85**. Such folded regions **85** can be provided along a radial direction and substantially conform to the circumference of the outer polymeric tubular layer **70**. At least a portion of the folded regions **85** can be positioned adjacent the outer surface **83** of the outer polymeric tubular layer **70**.

Additionally, as shown in **FIGS. 27B** and **27E****,** at least a portion of the folded region or regions **85** can be overlapped by an outer covering, such as outer polymeric covering **81**. In such aspects, the outer polymeric covering **81** can be the elongated tube as disclosed herein. The outer polymeric covering **81** can be adjacent at least a portion of the outer surface **83** of the outer polymeric tubular layer **70**. The outer polymeric covering **81** serves to at least partially contain the folded regions **85** of the inner polymeric liner **68** and can also prevent the folded regions **85** from separating from the outer polymeric tubular layer **70** when, for example, the sheath **66** undergoes bending. In some aspects, the outer polymeric covering (or elongated tube as described herein) **81** can be at least partially adhered to the outer surface **83** of the outer polymeric tubular layer **70**. The outer polymeric covering (elongated tube) **81** in certain aspects can increase the stiffness and/or durability of the sheath **66,** while in other aspects and as also disclosed herein can reduce the push force required to push a medical device through the sheath. In certain aspects, however, and as shown in **FIGS. 27B** and **27E****,** the outer polymeric covering **81** may not entirely overlap the circumference of the sheath **66**. For example, the outer polymeric covering **81** may be provided with first and second ends, where the ends do not contact one another. In these aspects, only a portion of the folded region **85** of the inner polymeric liner **68** is overlapped by the outer polymeric covering **81**.

In aspects having a plurality of folded regions **85,** the regions can be equally displaced from each other around the circumference of the outer polymeric tubular layer **70.** Alternatively, the folded regions can be off-center, different sizes, and/or randomly spaced apart from each other. While portions of the inner polymeric liner **68** and the outer polymeric tubular layer **70** can be adhered or otherwise coupled to one another, the folded regions **85** preferably are not adhered or coupled to the outer polymeric tubular layer **70**. For example, adhesion between the inner polymeric liner **68** and the outer polymeric tubular layer **70** can be highest in areas of minimal expansion.

One particular aspect of the sheath, as illustrated in **FIGS. 27A-28****,** comprises a polyethylene (e.g., high density polyethylene) outer polymeric tubular layer **70** and a PTFE inner polymeric liner **68**. However, other materials are suitable for each layer, as described above. Generally, suitable materials for use with the outer polymeric tubular layer **70** include materials having a high stiffness or modulus of strength that can support expansion and contraction of the inner polymeric liner **68**.

In certain aspects, where the inner liner **68** comprises one or more folded portions, and as disclosed above, the inner liner can be selectively etched. In such aspects, at least a portion of the outer surface of the at least one folded portion of the inner liner is not etched along at least a portion of the sheath length. In still further aspects, the outer surface of the inner liner comprises one or more nonetched portions along the sheath length. Again, in the aspects where are a plurality of nonetched portions, such portions can be at any location on the sheath. In certain aspects, for example, and without limitation, each of the one or more nonetched portions is followed by an etched portion. While in other aspects, the one or more nonetched portions comprise the outer surface of the at least one folded portion.

In certain aspects, the sheaths disclosed herein can exhibit an insertion force of less than about 60 N, less than about 50 N, less than about 40 N, or less than about 30 N. In still further aspects, the sheath can exhibit a reduction in an insertion force of at least about 10%, at least about 20%, at least about 30%, at least about 40%, at least about 50%, at least about 60%, at least about 70%, at least about 80%, at least about 90%, or at least about 100%, when compared to a substantially identical reference sheath that does not comprise the first compound composition and the selectively etched inner liner

In still further aspects, where the sheath comprises an inner liner that is selectively etched and has nonetched portions as disclosed above, such a sheath can exhibit even a substantial reduction in the insertion force when compared to a substantially identical sheath that does not comprise similar nonetched portions.

In some aspects, the outer polymeric tubular layer **70** comprises the same material or combination of materials along the entire length of the outer polymeric tubular layer **70**. In alternative aspects, the material composition can change along the length of the outer polymeric tubular layer **70**. For example, the outer polymeric tubular layer can be provided with one or more segments, where the composition changes from segment to segment. In one particular aspect, the Durometer rating of the composition changes along the length of the outer polymeric tubular layer **70** such that segments near the proximal end comprise a stiffer material or combination of materials, while segments near the distal end comprise a softer material or combination of materials. This can allow for a sheath 66 having a relatively stiff proximal end at the point of introducing a delivery apparatus while still having a relatively soft distal tip at the point of entry into the patient's vessel.

As with other disclosed aspects, the aspects of sheath **66** shown in **FIGS. 27A-****28** can be provided in a wide range of sizes and dimensions. For example, the sheath **66** can be provided with an unexpanded inner diameter of from about 3 Fr to about 26 Fr, including exemplary values of about 5 Fr, about 10 Fr, about 15 Fr, about 20 Fr, and about 25 Fr. In some aspects, the sheath **66** has an unexpanded inner diameter of from about 15 Fr to about 16 Fr. In some aspects, the unexpanded inner diameter of the sheath **66** can range from about 3 Fr to about 26 Fr, including exemplary values of about 5 Fr, about 10 Fr, about 15 Fr, about 20 Fr, and about 25 Fr at or near the distal end of sheath **66,** while the unexpanded inner diameter of the sheath **66** can range from about 3 Fr to about 28 Fr, including exemplary values of about 5 Fr, about 10 Fr, about 15 Fr, about 20 Fr, and about 25 Fr at or near the proximal end of sheath **66.** For example, in one unexpanded aspect, the sheath **66** can transition from an unexpanded inner diameter of about 16 Fr at or near the distal end of the sheath **66** to an unexpanded inner diameter of about 26 Fr at or near the proximal end of the sheath **66.**

The sheath **66** can be provided with an unexpanded outer diameter of from about 3 Fr to about 30 Fr, including exemplary values of about 5 Fr, about 10 Fr, about 15 Fr, about 20 Fr, and about 25 Fr and, in some aspects has an unexpanded outer diameter of from about 18 Fr to about 19 Fr. In some aspects, the unexpanded outer diameter of the sheath **66** can range from about 3 Fr to about 28 Fr, including exemplary values of about 5 Fr, about 10 Fr, about 15 Fr, about 20 Fr, and about 25 Fr at or near the distal end of sheath **66,** while the unexpanded outer diameter of the sheath **66** can range from about 3 Fr to about 30 Fr, including exemplary values of about 5 Fr, about 10 Fr, about 15 Fr, about 20 Fr, and about 25 Fr at or near the proximal end of sheath **66**. For example, in one unexpanded aspect, the sheath **66** can transition from an unexpanded outer diameter of about 18 Fr at or near the distal end of the sheath **66** to an unexpanded outer diameter of about 28 Fr at or near the proximal end of the sheath **66**.

The thickness of the inner polymeric liner **68** can vary, but in some preferred aspects is from about 0.002 inches to about 0.015 inches, including exemplary values of about 0.003 inches, about 0.004 inches, about 0.005 inches, about 0.006 inches, about 0.007 inches, about 0.008 inches, about 0.009 inches, about 0.010 inches, about 0.011 inches, about 0.012 inches, about 0.013 inches, and about 0.014 inches. In some aspects, expansion of the sheath **66** can result in expansion of the unexpanded outer diameter of from about 10% or less to about 430% or more, for example, and without limitation about 1%, about 5%, about 10%, about 20%, about 50%, about 70%, about 100%, about 120%, about 150%, about 200%, about 250%, about 300%, about 350%, about 400%, about 450%, about 500%, or even more.

As with other illustrated and described aspects, the aspects shown in **FIGS. 27A-****28** can be provided with a radiopaque filler and/or a radiopaque tip marker, as described above. The sheath **66** can be provided with a radiopaque tip marker provided at or near the distal tip of the sheath **66**. Such a radiopaque tip marker can comprise materials such as those suitable for the radiopaque filler, platinum, iridium, platinum/iridium alloys, stainless steel, other biocompatible metals, or combinations thereof.

In still further aspects, where the folded portions can be present, the at least one folded portion can comprise a first folded edge and a second folded edge and an overlapping portion extending circumferentially between the first and second folded edges, the overlapping portion comprising an overlap in a radial direction of at least two thicknesses of the inner liner, and wherein the first folded edge is configured to move closer to the second folded edge to shorten the overlapping portion at a local axial location during application of a radial outward force by passage of the medical device and wherein shortening of the overlapping portion corresponds with a local expansion of the lumen. Yet in other aspects, the at least one folded portion comprises a first folded edge and a second folded edge and an overlapping portion extending circumferentially between the first and second folded edges, the overlapping portion comprising an overlap in a radial direction of at least two thicknesses of the inner liner, wherein the first folded edge is configured to move closer to the second folded edge to shorten the overlapping portion at a local axial location during application of a radial outward force by passage of the medical device and wherein shortening of the overlapping portion corresponds with a local expansion of the lumen, and wherein the outer layer includes a first longitudinally extending edge and a second longitudinally extending edge configured to separate as the sheath expands, the fist longitudinal extending edge and an overlapping portion of the outer layer extending over the second longitudinally extending edge when the sheath is not expanded.

In still further aspects, it is understood that the inner liner can be configured to expand to a substantially cylindrical tube.

In still further aspects, the sheath disclosed herein and comprising the elongated tube, as described herein, can further comprise an inner tubular layer comprising a longitudinal slit and partially defining an inner lumen, a first outer tubular layer enveloping the inner layer, the outer tubular layer comprising a longitudinally extending, folded flap that overlies a portion of an outer surface of the outer layer when the sheath is in an unexpanded state, and the elongated tube positioned such that the inner surface of the elongated tube overlies at least a portion of the outer surface of the first outer tubular layer.

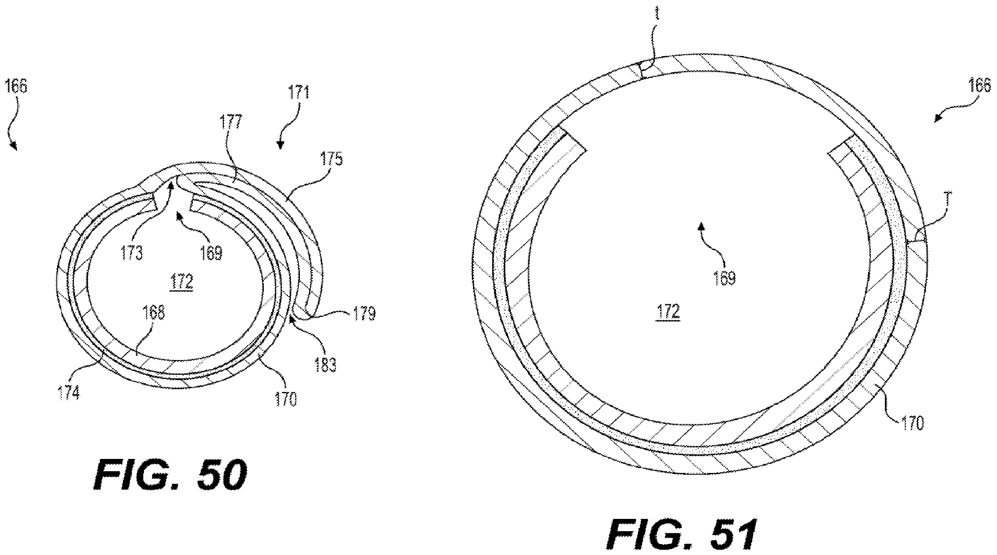

Such exemplary aspects are described in detail below. **FIGS. 50** and **51** show cross-sectional views of an expandable sheath **166** having an inner tubular layer **168** with a longitudinal slit **169.** In some aspects, the longitudinal slit **169** extends the entire length of the inner tubular layer **168.** A first outer tubular layer **170** envelops the inner tubular layer **168** and includes a longitudinally extending, folded flap **171.** In some aspects, the folded flap **171** extends the entire length of the first outer tubular layer **170.** The folded flap **171** overlies a portion of the outer surface **183** of the first outer tubular layer **170** when the sheath is in an unexpanded state (**FIG. 50**). When a prosthetic device is moved through an inner lumen **172** of the sheath **166,** it applies an outwardly directed radial force on the inner tubular layer **168** that widens the longitudinal slit **169** and unfolds the folded flap **171.** **FIG. 51** shows the sheath **166** in the unexpanded state, with the longitudinal slit **169** widened, and the first outer tubular layer **170** unfolded.

The folded flap **171** of the first outer tubular layer **170** has a base **173.** The base **173** can be positioned radially outwardly from the longitudinal slit **169.** In some aspects, the base **173** is centered over the longitudinal slit **169.** The folded flap **171** further includes a longitudinally extending overlying portion **175** extending from the base **173** to a longitudinally extending crease **179** at the edge of the flap **171**. The longitudinally extending overlying portion **175** overlies a longitudinally extending underlaying portion **177** and is separated from the underlaying portion **177** by the crease **179.** Underlaying portion **177** contacts the outer surface **183** of the first outer tubular layer **170** when the sheath is in an unexpanded state, as shown in **FIG. 50**.

Some aspects of sheath **166** can include multiple longitudinally extending folded flaps that overlie portions of the outer surface **183** of the first outer tubular layer **170,** positioned at various locations around the circumference of sheath **166**. For example, 2, 3, 4, 5, 6, 7, 8, 9, or 10 longitudinally extending folded flaps can be positioned around the circumference. In some aspects, these multiple folded flaps are equally spaced around the circumference of the sheath **166.**

The folded flap **171** extends circumferentially around a portion of the sheath **166**. In some aspects, the longitudinally extending flap **171** extends around about 20% to about 40% of the outer circumference of the first outer tubular layer **170** when the sheath 166 is unexpanded (including about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, and about 40% of the outer circumference of the first outer tubular layer 170). In one example, for a sheath having a 14F (4.7 millimeters) unexpanded outer diameter, the folded flap extends around about 85 to about 120 degrees (including exemplary values of about 90, about 95, about 100, about 105, about 110, and 115 degrees), or about 23%-35%, (including exemplary values of about 24%, about 25%, about 26%, about 27%, about 28%, about 29%, about 30%, about 31%, about 32%, about 33%, and about 34%) of the outer circumference (resulting in an inner lumen having an expanded diameter of about 7.6 millimeters to about 8.4 millimeters, including exemplary values of about 7.7 mm, about 7.8 nm, about 7.9 mm, about 8.0 mm, about 8.1 mm, about 8.2 mm, and about 8.3 mm, which can be used with a valve having a 6.4 millimeter crimped outer diameter, and a 26 millimeter expanded outer diameter).

**In** **FIG. 51****,** the wall thickness of flap **171** is labeled ***t***, and the wall thickness of other parts of the first outer tubular layer **170** are labeled ***T***. In some aspects, a portion of the flap **171** (such as, for example, the underlaying portion **177** or the overlying portion **175**) can have a wall thickness ***t*** that is thinner than a wall thickness (***T***) of other portions of the outer tubular layer. This variation in wall thickness promotes even column strength around the circumference of the sheath **166**, which reduces kinking and minimizing the total outer diameter of the sheath. The wall thickness variation can also facilitate the folding process. In some aspects, the entire flap **171** has a wall thickness ***t*** that is thinner than a wall thickness ***T*** of the remainder of the outer tubular layer. In one example, the wall thickness of ***t*** can be from about 0.003 inches to about 0.007 inches, including exemplary values of about 0.004 inches, about 0.005 inches, and about 0.006 inches, while the wall thickness of ***T*** can be from about 0.008 inches to about 0.012 inches, including exemplary values of about 0.009 inches and about 0.01 inches. In other aspects, such as the one shown in **FIG. 51**, the wall thickness ***t*** of flap **171** is about equal to the wall thickness ***T*** of the remainder of the first outer tubular layer **170.**

The first outer tubular layer **170** is formed of a material having a low coefficient of friction, a high tensile modulus, and a high ultimate tensile strength in order to improve the push force transmission through the length of sheath **166** while reducing kinking. Good push force transmission means that the force applied by the practitioner to advance the sheath is predictable, responsive, and consistent along the length of the sheath. However, an excessively high tensile modulus may limit the ability of the longitudinally extending flap **171** to open, which could hamper push force transmission. A desirable range for the tensile modulus of the first outer tubular layer **170** is from about 300 MPa to about 2,000 MPa (including about 300 MPa, about 400 MPa, about 500 MPa, about 600 MPa, about 700 MPa, about 800 MPa, about 900 MPa, about 1000 MPa, about 1100 MPa, about 1200 MPa, about 1300 MPa, about 1400 MPa, about 1500 MPa, about 1600 MPa, about 1700 MPa, about 1800 MPa, about 1900 MPa, and about 2000 MPa). In some aspects, the tensile modulus may preferably be at least 700 MPa. High axial and radial stiffnesses enable the sheath to be easily inserted and to resist collapse within the body.

The ultimate tensile strength of the first outer tubular layer **170** can be at least 50 MPa. A high ultimate tensile strength helps to prevent the material from tearing while the prosthetic device is advancing through the sheath.

In some exemplary and unlimiting aspects, the first outer tubular layer **170** of the aspects shown in **FIGS. 50** and **51** can comprise high density polyethylene (HDPE), polyamide, co-polyamide, polyether block amide (PEBAX^{®}), or a blend of polyamide. Materials having shape memory properties are advantageous because the first outer tubular layer **170** can be given a bias toward the folded state (for example, by heat setting). This facilitates refolding of the first outer tubular layer **170** after passage of a prosthetic device. PEBAX^{®} is an exemplary shape memory material that may be heat set toward the folded state.

In some aspects, the outer surface **183** of the first outer tubular layer **170** can include a hydrophilic coating. In some aspects, a bond can be created between the underlaying portion **177** of the folded flap **171** and the outer surface **183** of the first outer tubular layer **170.** The bond can be a thermal bond (with portions of the contacting layers melted together), or it can be a separate layer of adhesive.

As discussed above, the inner tubular layer **168** of the aspects shown in **FIGS. 50** and **51** include a longitudinal slit **169.** The inner tubular layer **168** can comprise a first longitudinally extending end **178** and a second longitudinally extending end **180,** the first and second longitudinally extending ends **178, 180** defining the longitudinal slit **171.**

The inner tubular layer **168** forms a low friction barrier between a passing prosthetic device and the first higher friction outer tubular layer **170.** The inner tubular layer **168** extends around at least 80% (or at least 85%, or at least 90%, or at least 95%) of the circumference of the inner lumen **172** when the sheath **166** is in an unexpanded state. This high degree of coverage limits contact between the passing prosthetic device and the first higher friction outer tubular layer **170.** In some aspects, the coefficient of friction per ASTM D1894 of the inner tubular layer **168** (static or dynamic) is 0.30 or less, or 0.25 or less, or 0.20 or less, or 0.1 or less, or 0.05 or less. In other aspects, the coefficient of friction is 0.25 or less.

In some aspects, the low-friction inner tubular layer **168** comprises, or is formed of, a material having a tensile modulus of at least about 300 MPa (and up to about 1,400 MPa, including exemplary values of about 350 MPa, about 400 MPa, about 450 MPa, about 500 MPa, about 550 MPa, about 600 MPa, about 650 MPa, about 700 MPa, about 750 MPa, about 800 MPa, about 850 MPa, about 900 MPa, about 1,000 MPa, about 1,050 MPa, about 1,100 MPa, about 1,150 MPa, about 1,200 MPa, about 1,250 MPa, about 1,300 MPa, and about 1,350 MPa) to provide good push force transmission while providing kink resistance. This material could be, for example, high density polyethylene or a fluoropolymer. Exemplary fluoropolymers include polytetrafluoroethylene, ethylene fluorinated ethylene propylene, or perfluoro alkoxy.

A tie layer **174** can be positioned between the two layers, thereby adhering the inner tubular layer **168** to the first outer tubular layer **170.** The tie layer **174** can be formed of polyurethane or functionalized polyolefin in some aspects. In some aspects, the contacting surface of the inner tubular layer **168** can be etched to improve bonding to the tie layer. For example, an inner tubular layer **168** that includes, or is formed of, a fluoropolymer might be etched on its outer surface to improve thermal bonding to the tie layer **174.** In yet further aspects, the inner tubular layer **168** can be selectively etched as described above.

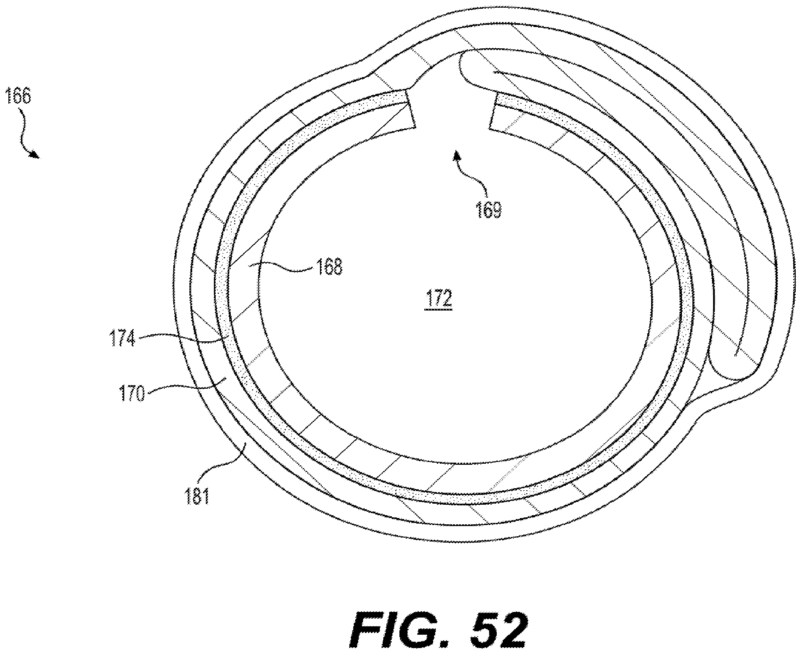

As shown in **FIG. 52****,** the sheath comprises the disclosed in detail herein the elongated tube forming a second outer layer **181.** In such aspects, the elongated tube forms a second outer layer having an inner surface and an outer surface and wherein the elongated tube is positioned at at least the proximal end of the sheath and extending along at least a portion of a length of the sheath, such that the inner surface of the elongated tube overlies at least a portion of the outer surface of the first outer tubular layer. It is understood that this second outer layer, in some aspects, is also referred to as an outer jacket. The outer jacket **181** is formed as disclosed herein and can comprise a first polymer layer, wherein the first polymer layer comprises a first compound composition comprising from greater than 0 wt% to less than 100 wt % of a polymer comprising a polyether block amide, a polyurethane, or a combination thereof; less than about 65 wt% of an inorganic filler based on a total weight of the first compound composition; and up to about 20 wt% of a solid lubricant filler based on a total weight of the first compound composition. The additional exemplary aspects of the composition and characteristics of such a second outer layer (outer jacket) are disclosed in detail above.

As mentioned above, in some aspects, the first outer tubular layer **170** can be included or can be formed of a shape memory material (for example, a heat-set polymer such as PEBAX^{®}) that facilitates refolding of the first outer tubular layer **170** after expansion. In aspects disclosed herein, the second outer layer (outer jacket) **181** can extend over and envelop the first outer tubular layer **170.** The refolding of the first outer tubular layer **170** can preferably return the sheath **166** back to its original outer diameter or to a value close to the original outer diameter (for example, to within about 10%, about 20%, about 30%, about 40%, or about 50% of the original outer diameter).

Methods of using the sheath of **FIGS. 50-52** include first inserting an expandable sheath **166** into the vasculature of a subject and advancing the prosthetic device through an inner lumen **172** of the expandable sheath **166.** The prosthetic device applies an outwardly directed radial force to the inner tubular layer **168** of the expandable sheath **166.** In some aspects, the outwardly directed radial force is transmitted through the inner tubular layer **168,** the tie layer **174,** and the first outer tubular layer **170**. The outwardly directed radial force widens a longitudinal slit **169** in the inner tubular layer 168. The widening of the longitudinal slit **169** travels the full length of the expandable sheath in some aspects.

The outwardly directed radial force further unfolds the longitudinally extending flap **171** of the first outer tubular layer **170** to expand the expandable sheath. The unfolding of the flap **171** can include sliding a longitudinally extending overlying portion **175** circumferentially against a longitudinally extending underlaying portion **177** of flap 171. The underlaying portion **177** can slide circumferentially against an outer surface **183** of the first outer tubular layer **170**. In some aspects, the unfolding of the longitudinally extending flap **171** occurs at a position radially outward from the longitudinal slit **169** of the inner tubular layer **168**. The unfolding of the flap **171** can extend the full length of the expandable sheath **166** in some aspects.

The longitudinal slit **169** of the inner tubular layer **168** narrows once the outwardly directed radial force has ceased (i.e., once the prosthetic device has passed by). The slit **169** may narrow back to its original width or to a value close to the original width (for example, to a value within 10% of the original width). The narrowing can occur along the entire length of the sheath **166**. The prosthetic device is then delivered to the procedure site.

The longitudinally extending flap **171** at least partially refolds once the prosthetic device ceases to apply the outwardly directed radial force (i.e., once it has passed by). In some aspects, the longitudinally extending flap **171** refolds itself due to a shape memory bias toward the folded state. In some aspects, an inwardly directed radial force is applied to the outer surface **183** of the first outer tubular layer **170** to refold the longitudinally extending flap **171** (for example, by the second outer layer (outer jacket) **181**).

An example method of making the sheath is as follows. These steps are not meant to be limiting. The steps given can be reordered as needed. Other steps can be added, or in other examples, some steps may not be necessary. Sizes are approximate. Disclosed herein is an exemplary aspect of making a sheath where the elongated tube or recoverable outer jacket covers the whole length of the expandable sheath shaft assembly. In certain aspects, the elongate tube and the expandable sheath shaft assembly can be about 380 mm long (15 inches). 1) Start with a PTFE inner layer of about 0.200 inch inner diameter (ID), wall thickness about 0.004 inches, 2) load PTFE inner layer on to a tapering mandrel from about 0.200 inches to about 0.187 inches, 3) stretch on to an 0.187 inch outer diameter (OD) mandrel section under heat, 4) flare proximal end of 0.200 inch ID PTFE to 0.340 inches ID under heat, 5) by expanding with air pressure, load tie layer such as Tecoflex 80A having about 0.200 inch ID and 0.004 inch wall thickness over the PTFE inner layer along the body section, 6) adhere tie layer to inner layer, for example, by covering it with FEP (fluorinated ethylene propylene) heat shrink tubing and applying heat, 7) remove FEP thermal shrink tubing if it was used, 8) create a longitudinal slit along the body section of the assembly, 9) load the subassembly having a slit on to a 0.187 inch OD mandrel, 10) load outer layer over the body, 11) fold outer layer, 12) heat set the fold, for example, by inserting the subassembly with a fold inside a heat shrink tubing and placing the assembly into the oven, 13) remove the shrink tubing if it was used, 14) add the second outer layer; and 14) remove sheath from the mandrel.

The outer jacket can be expanded to a larger diameter by applying air pressure to the inner diameter of the outer jacket. The assembled sheath can then be inserted into the expanded outer jacket for the desired length. Air pressure can then be released to allow the outer jacket to decrease in diameter to the original diameter.

Also disclosed an exemplary aspect of making a sheath where the elongated tube or recoverable outer jacket covers the proximal side of the expandable sheath shaft assembly. In such exemplary aspects, the sheath shaft having about 380 mm (15 inches) length can be assembled. For example, a tapered mandrel can be inserted into the sheath shaft assembly's inner diameter. The outer jacket or strain relief tubing, as disclosed here, can be cut to about 105 mm +/- 10 mm (4.15" +/- 0.40") and positioned on the sheath shaft assembly. The outer jacket can have an inner diameter big enough to slide the outer jacket over the sheath shaft assembly, especially with the outer jacket comprised of the low friction PTFE incorporating layer. An FEP (fluoroethylene propylene) heat shrink tubing can then be flared on the proximal end. Flared FEP heat shrink tubing can be slid over the outer jacket. Heat can be used to fuse the outer jacket to the sheath shaft assembly. The distal end of the outer jacket can be heated to adhere to the distal end of the outer jacket to the sheath shaft assembly. FEP heat shrink tubing can then be removed.

Further, the mandrel can be removed. Still, further, the proximal end of the sheath and the outer jacket assembly can beflared over a heated flaring tool. It is understood that in some exemplary and unlimiting aspects, other manufacturing steps can be present such as the flushing tube and the housing bonding, hydrophilic coating, inspection, the valves, and the sleeve assembly, and leak test etc.

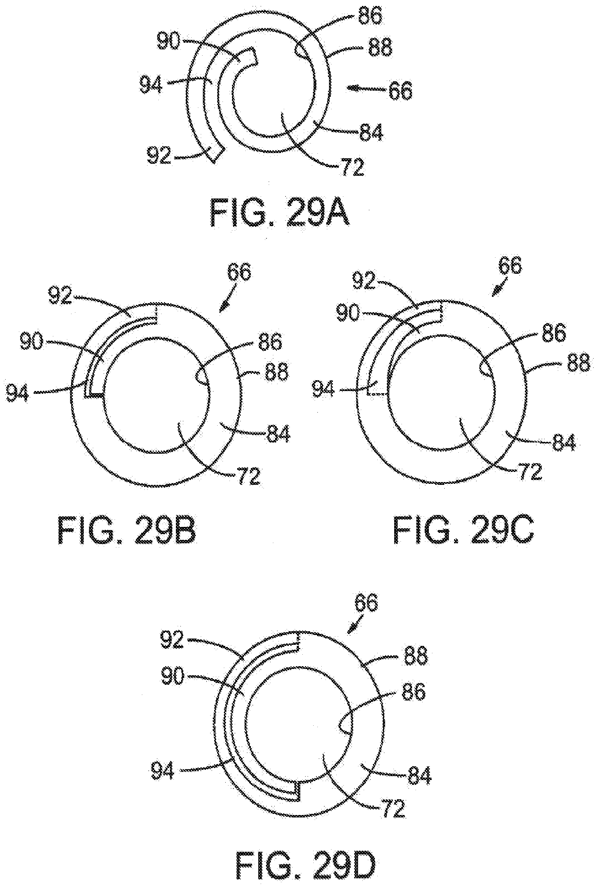

**FIGS. 29A-29D** show section views of other possible configurations of a sheath **66** for introducing a prosthetic device into a patient's vasculature. The sheath **66** comprises a polymeric tubular layer **84** having an inner surface **86** and an outer surface **88.** The thickness of the polymeric tubular layer **84** extends from the inner surface **86** to the outer surface **88.** As shown in **FIGS. 29B-29D****,** the polymeric tubular layer **84** can be formed with at least a first angular portion **90** of reduced thickness adjacent the inner surface **86** and a second angular portion **92** of reduced thickness adjacent the outer surface **88,** with the second portion **92** at least partially overlapping the first portion **90.** **FIG. 29A** illustrates a similar configuration, where a second portion **92** at least partially overlaps a first portion **90** in a partial coil configuration. In the aspect of **FIG. 29A****,** the second portion **92** and the first portion **90** can have the same thickness.

In preferred aspects, the first and second portions **90, 92** are not adhered to one another. In some aspects, and as best seen in **FIG. 29A****,** there can be a small gap **94** between the first and second portions **90, 92** that can give the sheath **66** the appearance of having two interior lumens **72, 94.** **FIGS. 29A-29D** illustrate the sheath **66** in unexpanded configurations. Preferably, upon expansion of the sheath **66,** the ends of the first and second portions **90, 92** abut or are in close proximity to each other to reduce or eliminate any gap between them.

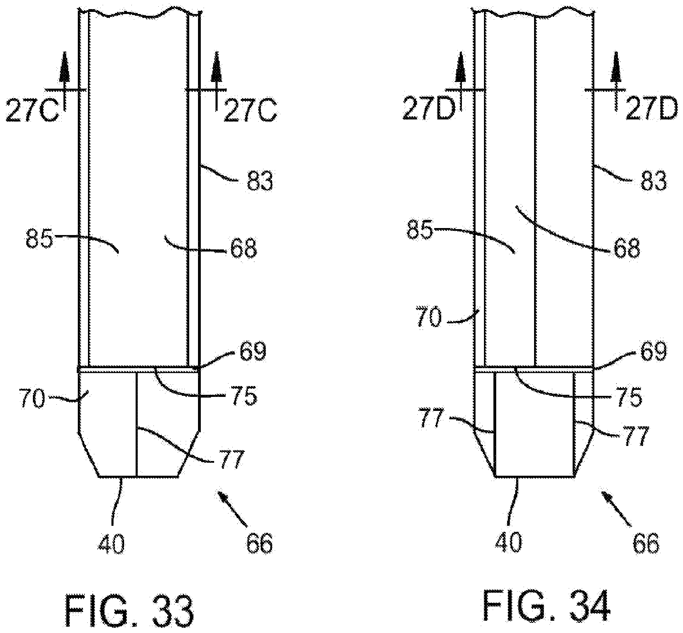

In some aspects, a sheath **66** can comprise a partial slit or score line along at least a portion of its length. For example, as shown in **FIG. 33****,** a sheath **66** can comprise an outer polymeric tubular layer **70** over an inner polymeric liner **68**. The inner polymeric layer can extend through a cut in the outer polymeric tubular layer **70** to form a folded region **85** on the outer surface of the outer polymeric tubular layer **70,** such as also shown in **FIG. 27C****.** The folded region **85** of the inner layer, in some aspects, terminates before the outer polymeric tubular layer **70** (i.e., the outer polymeric tubular layer **70** is longer than the inner layer). As shown in **FIG. 33**, in these aspects, the sheath **66** can comprise a partial slit or score line **77** that can extend from the termination (distal end) **75** of the folded region **85** to the distal end **40** of the sheath **66**. In some aspects, score line **77** can facilitate expansion of the sheath **66**.

Score line **77** can be substantially centrally located with respect to the folded region **85**. In alternative aspects, score line **77** can be positioned in other locations relative to the folded region **85**. Also, sheath **66** can comprise one or more score lines **77.** For example, as shown in **FIG. 34****,** one or more score lines **77** can be peripherally located with respect to the folded region **85**. The one or more score lines **77** can be positioned anywhere around the circumference of the outer polymeric tubular layer **70**. In aspects comprising a radiopaque marker **69** as seen in **FIG. 33**, a score line **77** can extend from, for example, the distal end of the radiopaque marker **69** substantially to the distal end **40** of the sheath **66**.

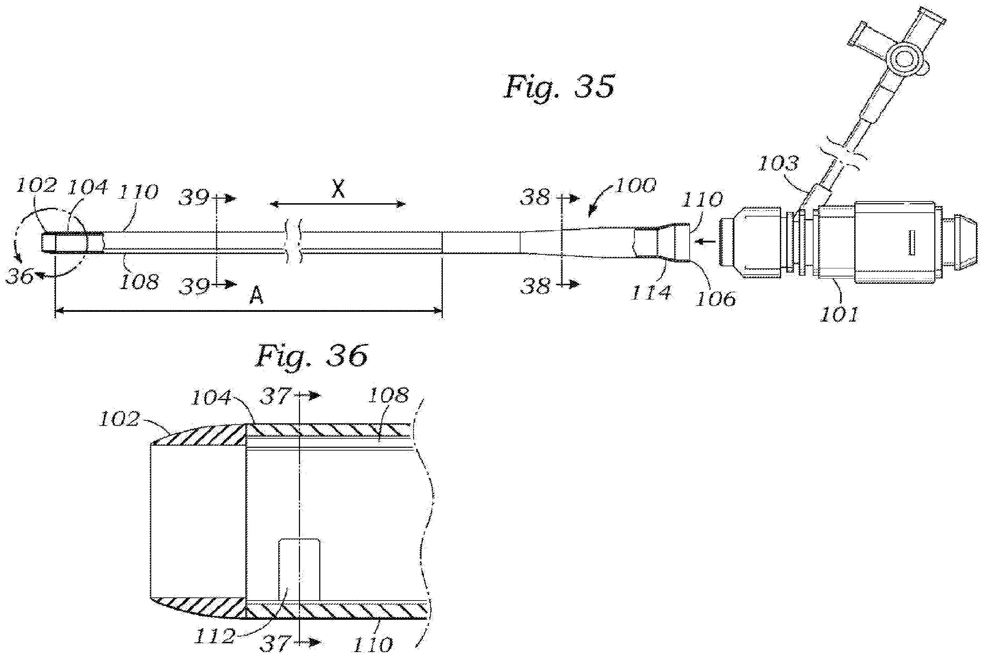

**FIGS. 35** and **36** illustrate an expandable sheath **100** according to the present disclosure, which can be used with a delivery apparatus for delivering a prosthetic device, such as a tissue heart valve into a patient. In general, the delivery apparatus can include a steerable guide catheter (also referred to as a flex catheter) a balloon catheter extending through the guide catheter, and a nose catheter extending through the balloon catheter (e.g., as depicted in **FIG. 1**). It is understood, however, the sheath **100** can refer to any type of the sheath as disclosed herein and that can be used together with the delivery apparatus. The specific configuration of the sheath **100** is not limited to one specific description and can include any configurations disclosed herein. The guide catheter, the balloon catheter, and the nose catheter can be adapted to slide longitudinally relative to each other to facilitate delivery and positioning of the valve at an implantation site in a patient's body. However, it should be noted that the sheath **100** can be used with any type of elongated delivery apparatus used for implanting balloon-expandable prosthetic valves, self-expanding prosthetic valves, and other prosthetic devices. Generally, sheath **100** can be inserted into a vessel (*e.g*., the femoral or iliac arteries) by passing through the skin of the patient, such that a soft tip portion **102** at the distal end **104** of the sheath **100** is inserted into the vessel. The sheath **100** can also include a proximal flared end portion **114** to facilitate mating with an introducer housing **101** and catheters mentioned above (*e.g.*, the proximal flared end portion **114** can provide a compression fit over the housing tip and/or the proximal flared end portion **114** can be secured to the housing **101** via a nut or other fastening device or by bonding the proximal end of the sheath to the housing). The introducer housing **101** can house one or more valves that form a seal around the outer surface of the delivery apparatus once inserted through the housing, as known in the art. The delivery apparatus can be inserted into and through the sheath **100,** allowing the prosthetic device to be advanced through the patient's vasculature and implanted within the patient.

In certain aspects, sheath **100** can include a plurality of layers. For example, sheath **100** can include an inner layer **108** and an outer layer **110** disposed around the inner layer **108**. The inner layer **108** can define a lumen through which a delivery apparatus can travel into a patient's vessel in order to deliver, remove, repair, and/or replace a prosthetic device, moving in a direction along the longitudinal axis X. As the prosthetic device passes through the sheath **100,** the sheath locally expands from a first, resting diameter to a second, expanded diameter to accommodate the prosthetic device. After the prosthetic device passes through a particular location of the sheath **100,** each successive expanded portion or segment of the sheath **100** at least partially returns to the smaller, resting diameter. In this manner, the sheath **100** can be considered self-expanding in that it does not require the use of a balloon, dilator, and/or obturator to expand.

The inner and outer layers **108, 110** can comprise any suitable materials. Suitable materials for the inner layer **108** include polytetrafluoroethylene (PTFE), ethylene tetrafluoroethylene (ETFE), nylon, polyethylene, polyether block amide (*e.g.*, PEBAX^{®}), and/or combinations thereof. In one specific aspect, the inner layer 108 can comprise a lubricious, low-friction, or hydrophilic material, such as PTFE. Such a low coefficient of friction materials can facilitate the passage of the prosthetic device through the lumen defined by the inner layer **108**. In some aspects, the inner layer **108** can have a coefficient of friction of less than about 0.1. Some aspects of a sheath **100** can include a lubricious liner on the inner surface of the inner layer **108**. Examples of suitable lubricious liners include materials that can further reduce the coefficient of friction of the inner layer **108,** such as PTFE, polyethylene, polyvinylidene fluoride, and combinations thereof. Suitable materials for a lubricious liner also include other materials desirably having a coefficient of friction of about 0.1 or less.

Suitable materials for the outer layer **110** include nylon, polyethylene, PEBAX^{®}, HDPE, polyurethanes (*e.g.*, Tecoflex^{™}), and other medical grade materials. In one aspect, the outer layer 110 can comprise high density polyethylene (HDPE) and Tecoflex^{™} (or other polyurethane material) extruded as a composite. In some aspects, the Tecoflex^{™} can act as an adhesive between the inner layer **108** and the outer layer 110 and may only be present along a portion of the inner surface of the outer layer **11**0. Other suitable materials for the inner and outer layers are also disclosed in U.S. Patent Application Publication No. 2010/0094392.

Additionally, some aspects of a sheath **100** can include an exterior hydrophilic coating on the outer surface of the outer layer **110**. Such a hydrophilic coating can facilitate insertion of the sheath **100** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (e.g., PTFE, polyethylene, polyvinylidene fluoride), are also suitable for use with the sheath **100**. The elongate tube serving as an outer jacket can then be disposed on the outer layer of the sheath.

Best seen in **FIG. 36**, the soft tip portion **102** can comprise, in some aspects, lowdensity polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100**, such as by thermally bonding the soft tip portion **102** to the inner and outer layers of the sheath **100**. Such a soft tip portion **102** can be provided with a lower hardness than the other portions of the sheath **100**. In some aspects, the soft tip **102** can have a Shore hardness from about 25 D to about 40 D. The tip portion **102** is configured to be radially expandable to allow a prosthetic device to pass through the distal opening of the sheath **100**. For example, the tip portion **102** can be formed with a weakened portion, such as an axially extending score line or perforated line that is configured to split and allow the tip portion to expand radially when the prosthetic device passes through the tip portion (such as shown in the aspects of **FIGS. 33** and **34**).

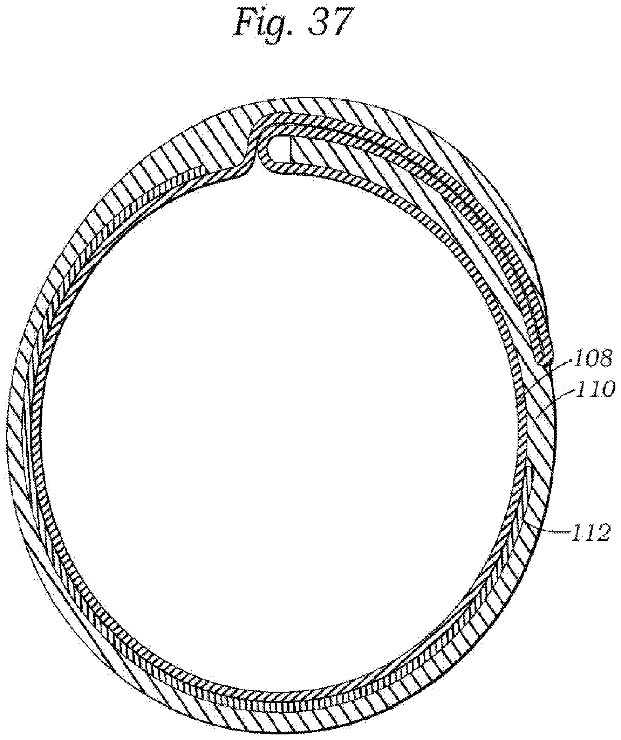

**FIG. 37** shows a cross-section view of the sheath **100** taken near the distal end **104** of the sheath **100.** As shown in **FIGS. 36** and **37****,** the sheath **100** can include at least one radiopaque filler or marker, such as a discontinuous or C-shaped band **112** positioned near the distal end **104** of the sheath **100**. The marker **112** can be associated with the inner and/or outer layers **108, 110** of the sheath **100**. For example, as shown in **FIG. 37**, the marker **112** can be positioned between the inner layer **108** and the outer layer **110**. In alternative aspects, the marker **112** can be associated with the outer surface of the outer layer **110**. In some aspects, the marker **112** can be embedded or blended within the inner or outer layers **108, 110.**

The C-shaped band **112** can serve as a radiopaque marker or filler to enable visibility of the sheath **100** under fluoroscopy during use within a patient. The C-shaped band **112** can comprise any suitable radiopaque material, such as barium sulfite, bismuth trioxide, titanium dioxide, bismuth subcarbonate, platinum, iridium, and combinations thereof. In one specific aspect, the C-shaped band can comprise 90% platinum and 10% iridium. In other aspects, the marker **112** needs not to be a C-shaped band. Other shapes, designs, and configurations are possible. For example, in some aspects, the marker **112** can extend around the entire circumference of the sheath **100.** In other aspects, the marker **112** can comprise a plurality of small markers spaced around the sheath **100**.

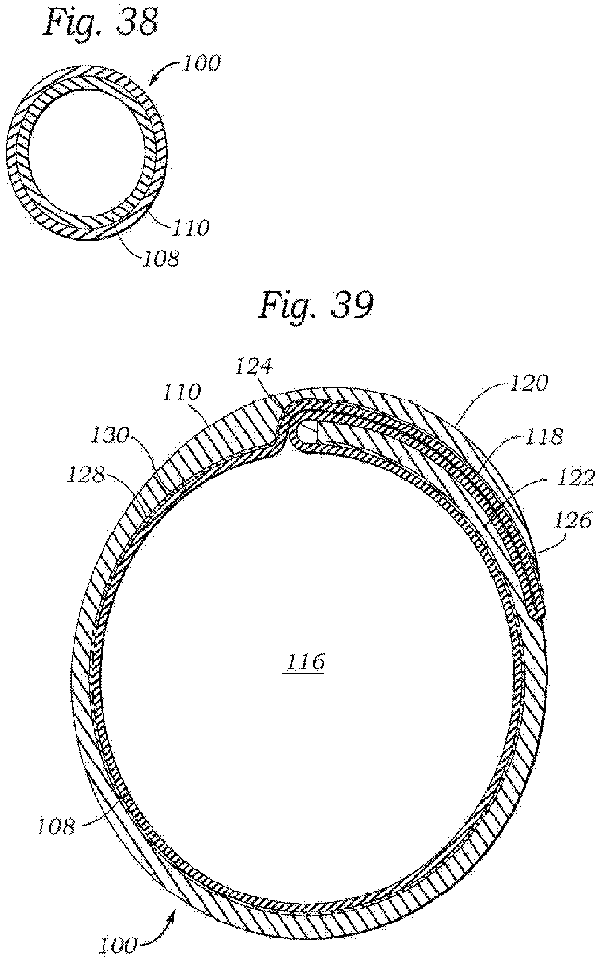

**FIGS. 38** and **39** show additional cross sections taken at different points along the sheath **100.** **FIG. 38** shows a cross-section of a segment of the sheath near the proximal end **106** of the sheath **100,** as indicated by line **38-38** in **FIG. 35****.** The sheath **100** at this location can include an inner layer (or liner) **108** and outer layer **110**. At this location, near the proximal end of the sheath, the layers **108, 110** can be substantially tubular, without any slits or folded portions in the layers. By contrast, the layers **108, 110** at different locations along the sheath **100** (*e.g.*, at the point indicated by line **39-39** in **FIG. 35**) can have a different configuration.

As shown in **FIG. 39**, the inner layer (liner) **108** can be arranged to form a substantially cylindrical lumen **116** therethrough. Inner liner **108** can include one or more folded portions **118.** In the aspect shown in **FIG. 39****,** inner liner **108** is arranged to have one folded portion **118** that can be positioned on either side of the inner layer (liner) **108**. The folded portion **118** includes a first fold (e.g., a longitudinally extending fold line) and a second fold and an overlapping portion extending circumferentially therebetween (when the sheath is in an unexpanded configuration). As illustrated in **FIG. 39****,** the folded portion **118** comprises an overlap in a radial direction of at least two thicknesses of the inner layer **108**. Inner liner **108** can be continuous in that there are no breaks, slits, or perforations in inner layer **108**. Outer layer **110** can be arranged in an overlapping fashion such that an overlapping portion of **120** overlaps at least a part of the folded portion **118** of the inner layer **108**. As shown in **FIG. 39**, the overlapping portion **120** also overlaps an underlaying portion **122** of the outer layer **110**. The underlaying portion **122** can be positioned to underlie both the overlapping portion **120** of the outer layer **110**, as well as the folded portion **118** of the inner layer **108**. Thus, the outer layer **110** can be discontinuous in that it includes a slit or a cut in order to form the overlapping and underlaying portions **120, 122.** In other words, a first edge **124** of the outer layer **110** is spaced apart from a second edge **126** of the outer layer **110** so as not to form a continuous layer.

As shown in **FIG. 39**, the sheath **100** can also include a thin layer of bonding or adhesive material **128,** also referred to as a tie layer, positioned between the inner and outer layers **108, 110.** In one aspect, the adhesive material **128** can comprise a polyurethane material such as Tecoflex^{™} or etched PTFE tubing. The adhesive material **128** can be positioned on an inner surface **130** of at least a portion of the outer layer **110** so as to provide adhesion between selected portions of the inner and outer layers **108, 110.** For example, the outer layer **110** may only include an adhesive layer **128** around the portion of the inner surface **130** that faces the lumen-forming portion of the inner layer **108**. In other words, the adhesive layer **128** can be positioned so that it does not contact the folded portion **118** of the inner layer **108** in some aspects. In other aspects, the adhesive layer **128** can be positioned in different configurations as desired for the particular application. For example, as shown in **FIG. 39****,** the adhesive layer **128** can be positioned along the entire inner surface **130** of the outer layer **110**. In an alternative aspect, the adhesive layer can be applied to the outer surface of the inner liner **108** instead of the inner surface of the outer layer. The adhesive layer **128** can be applied to all or selected portions on the inner layer **108**; for example, the adhesive layer **128** can be formed only on the portion of the inner layer that faces the lumen-forming portion of the outer layer and not on the folded portion. The configuration of **FIG. 39** allows for radial expansion of the sheath **100** as an outwardly directed radial force is applied from within (e.g., by passing a medical device such as a prosthetic heart valve through the lumen **116**). As radial force is applied, the folded portion **118** can at least partially separate, straighten, and/or unfold, and/or the overlapping portion **120** and the underlaying portion **122** of the outer layer **110** can slide circumferentially with respect to one another, thereby allowing the diameter of lumen **116** to enlarge.

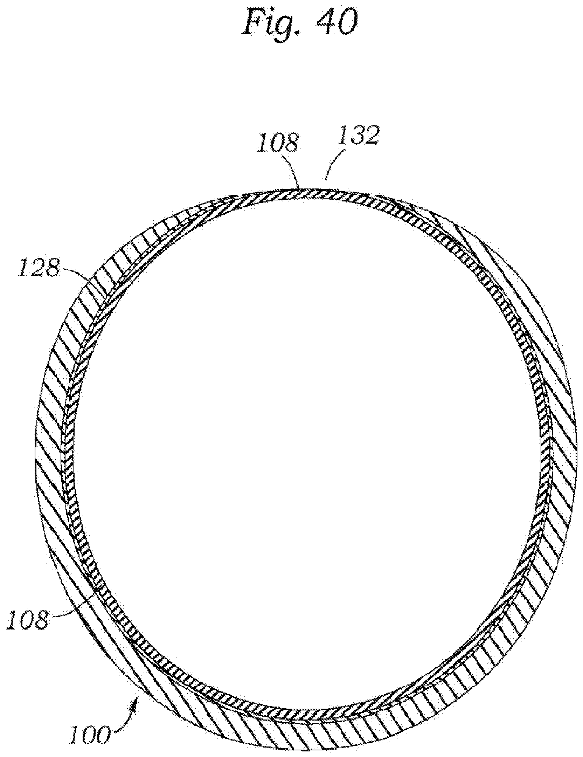

In this manner, the sheath **100** is configured to expand from a resting configuration (**FIG. 39**) to an expanded configuration shown in **FIG. 40**. In the expanded configuration, as shown in **FIG. 40****,** an annular gap **132** can form between the longitudinal edges of the overlapping portion **120** and the underlaying portion **122** of the outer layer **110**. As the sheath **100** expands at a particular location (i.e., locally expands at the location of the passing prosthetic device), the overlapping portion **120** of the outer layer **110** can move circumferentially with respect to the underlaying portion **122** as the folded portion **118** of the inner layer **108** unfolds. This movement can be facilitated by the use of a low-friction material for inner layer **108,** such as PTFE. Further, the folded portion **118** can at least partially separate and/or unfold to accommodate a medical device having a diameter larger than that of lumen **116** in the resting configuration. As shown in **FIG. 40****,** in some aspects, the folded portion of the inner layer **108** can completely unfold so that the inner layer **108** forms a cylindrical tube at the location of the expanded configuration.

The sheath **100** can be configured such that it locally expands at a particular location corresponding to the location of the medical device along the length of the lumen **116,** and then locally contracts once the medical device has passed that particular location. Thus, a bulge may be visible, traveling longitudinally along the length of the sheath as a medical device is introduced through the sheath, representing continuous local expansion and contraction as the device travels the length of the sheath **100.** In some aspects, each segment of the sheath **100** can locally contract after removal of any radial outward force such that it regains the original resting diameter of lumen **116.** In some aspects, each segment of the sheath **100** can locally contract after removal of any radial outward force such that it at least partially returns to the original resting diameter of lumen **116.**

The layers **108, 110** of sheath **100** can be configured, as shown in **FIG. 39** along at least a portion of the length of the sheath **100.** In some aspects, the layers **108, 110** can be configured as shown in **FIG. 39** along the length A **(****FIG. 35**) extending from a location adjacent the soft tip portion **102** to a location closer to the proximal end 106 of the sheath **100.** In this matter, the sheath is expandable and contractable only along a portion of the length of the sheath corresponding to length A (which typically corresponds to the section of the sheath inserted into the narrowest section of the patient's vasculature).

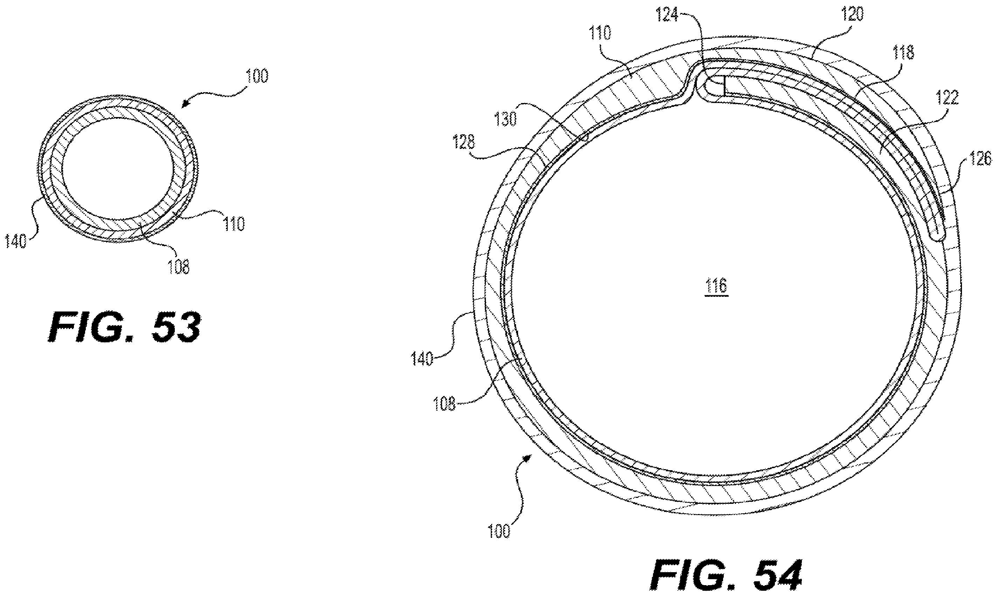

**FIGS. 53** and **54** show additional cross sections taken at different points along the sheath **100** that include the disclosed herein elongated tube **140** that behaves as an outer jacket of the sheath. Similar to **FIG. 38****,** **FIG. 53** shows a cross-section of a segment of the sheath near the proximal end **106** of the sheath **100,** as indicated by line **38-38 in** **FIG. 35****.** The sheath **100** at this location can comprise an inner layer (liner) **108,** outer layer **110,** adhesive layer **128,** and a second outer layer (the outer jacket) **140.** In this exemplary aspect, at this location, near the proximal end of the sheath, the layers **108, 110,** and **140** can be substantially tubular, without any slits or folded portions in the layers. By contrast, the layers **108, 110** at different locations along the sheath **100** (*e.g*., at the point indicated by line **39-39** in **FIG. 35**) can have a different configuration, while the second outer layer (the outer jacket) **140** maintains a substantially tubular shape, without slits or folds. It is understood that outer jacket **140,** as shown herein, can have any composition and characteristics of the elongated tube disclosed above.

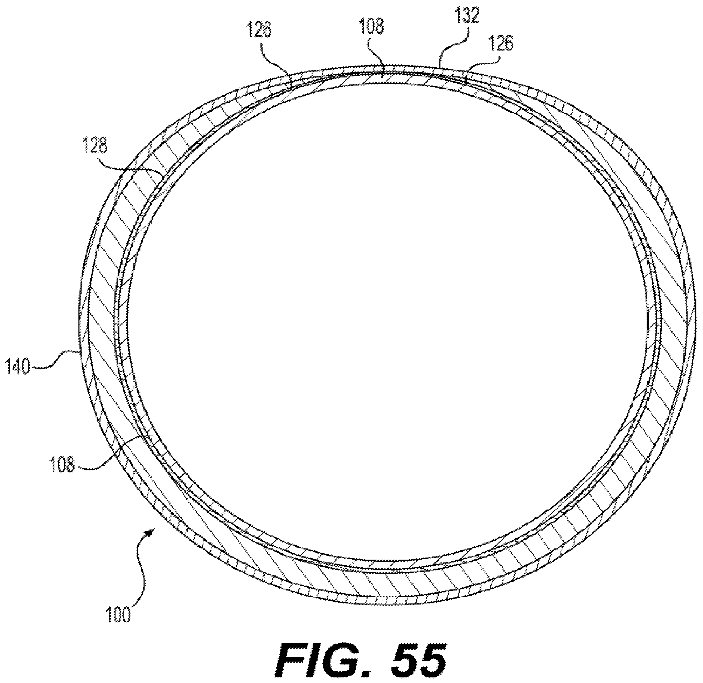

As shown in **FIG. 54****,** and described above with respect to **FIG. 39****,** the inner layer (liner) **108** can be arranged to form a substantially cylindrical lumen **116** extending therethrough. The inner layer **108** can include one or more folded portions **118.** The outer layer **110** can be arranged in an overlapping fashion such that an overlapping portion **120** overlaps at least a part of the folded portion **118** of the inner layer **108** as well as the underlaying portion **122** (positioned to underlie the folded portion **118** of the inner layer **108**) when the sheath is unexpanded. The sheath **100** is configured to locally expand from an unexpanded configuration in which the lumen **116** has a first diameter to an expanded configuration in which the lumen **116** has a second diameter larger than the first diameter. The sheath **100** expands in response to an outwardly directed radial force exerted by a medical device against the inner layer **108** as it passes through the lumen **116**. During expansion, the first fold/folded edge moves closer to the second fold/folded edge to shorten the folded portion **118**. As shown in **FIG. 55****,** in some aspects, the folded portion **118** of the inner layer **108** can completely unfold so that the inner layer (liner) **108** forms a cylindrical tube at the location of the expanded configuration. When the sheath is expanded, a portion of the inner layer **108** extends through the opening/gap provided in the outer layer **110,** where the opening is formed by the longitudinally extending edge of the overlapping portion **120** and a longitudinally extending edge of the underlaying portion **122**. As the prosthetic device passes, the sheath **100** then locally contracts at least partially back to the unexpanded configuration.

As described above, the sheath **100** includes an inner layer **108**. The inner layer **108** can be surface-treated, such as by plasma etching, chemical etching, or other suitable methods of surface treatment. In certain aspects, the inner liner **108** is selectively etched, forming various etched and nonetched portions, as disclosed above. By treating the surface of the inner layer **108,** the outer surface of the inner liner **108** can have areas with altered surface angles that can provide better adhesion between the inner layer **108** and the outer layer **110**. As described above, the inner liner **108** can comprise polytetrafluoroethylene (PTFE), polyimide, polyetheretherketone (PEEK), polyurethane, nylon, polyethylene, polyamide, or combinations thereof. In an example sheath **100,** the inner layer **108** is composed of an etched PTFE material. It is contemplated that the inner layer **108** can have a fully etched outer surface or a partially etched outer surface. When partially etched, the unetched portions of the outer surface of the inner liner **108** can extend longitudinally along a length of the inner layer **108** and/or circumferentially around the circumference of the inner layer **108**. For example, the desired unetched location on the inner layer **108** can be masked or otherwise covered during the etching process to prevent etching at that location. It is also contemplated that the entire outer surface of the inner liner **108** can be etched and the etching removed at the desired locations of the unetched surface.

In an example sheath **100,** unetched portions are provided along those surfaces of the inner layer **108** that come into contact with the outer surface of the outer layer **110**. That is, those portions of the inner layer **108,** excluding the tie layer **130,** would not include etching. For example, it is contemplated that etching is not included between the inner surface of the folded portion **118** of the inner layer **108** and the underlaying portion **122** of the outer layer **110.** By excluding etching on the portions where the inner layer **108** and the outer surface of the outer layer **110** are in direct contact helps to facilitate release of the inner surface of the folded portion **118** and the outer layer **110** during expansion of the sheath **100**.

The wall thickness of the inner liner can vary, but in some examples, the wall thickness of the inner layer **108** ranges between about 0.002 inches and about 0.006 inches (including about 0.002 inches, about 0.003 inches, about 0.004 inches, about 0.005 inches, about 0.006 inches). In other examples, the wall thickness of the inner layer ranges between about 0.003 includes and about 0.005 inches. In a further example, the wall thickness of the inner layer (liner) **108** ranges between about 0.0035 inches and about 0.0045 inches (including about 0.0035 inches, about 0.0040 inches, about 0.0045 inches).

As described above, the sheath **100** includes an outer layer **110** exerting a radially inward force on the inner layer **108.** In general, the outer layer **110** can comprise a polymeric material. As described above, the outer layer **110** can be comprised of PTFE, polyimide, PEEK, polyurethane, nylon, polyethylene, polypropylene, polyamide, polyether block amides, polyether block ester copolymer, thermoset silicone, latex, polyisoprene rubbers, high density polyethylene (HDPE), Tecoflex^{™}, or combinations thereof. In an exemplary aspect, the inner layer 108 can comprise PTFE, and the outer layer 110 can comprise a combination of HDPE and Tecoflex^{™}. The outer layer 110 can have a wall thickness ranging between about 0.007 inches and about 0.013 inches (including 0.007 inches, about 0.008 inches, about 0.009 inches, about 0.010 inches, about 0.011 inches, about 0.012 inches, about 0.013 inches). In another example, the outer layer 110 can have a wall thickness ranging between about 0.008 inches and about 0.012 inches. In another example, the outer layer 110 can have a wall thickness ranging between about 0.009 inches and about 0.011 inches.

As described above, the sheath **100** includes an outer jacket **140** that extends over and envelopes the outer layer **110.** While the outer layer **110** can be discontinuous, in that it includes a slit or a cut in order to form the overlapping and underlaying portions **120, 122** as described above, the outer jacket **140** comprises a continuous outer layer covering the inner and outer layers **108, 110.** It is understood that the outer jacket is formed by the disclosed above elongated tube having a disclosed composition and characteristics.

Generally, the outer jacket **140** has a relatively low tensile modules compared to the inner and outer layers **108, 110.** In certain aspects and as disclosed above, the outer jacket exhibits an elongation at break of ranging between about 40% and about 800% (including about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 800%).

The outer jacket **140** in this aspect can have a first polymer layer and a second polymer layer, similarly to the aspects disclosed above. For example, the first polymer layer can comprise a first compound composition comprising from greater than 0 wt% to less than 100 wt % of a polymer comprising a polyether block amide, a polyurethane, or a combination thereof; less than about 65 wt% of an inorganic filler based on a total weight of the first compound composition; and up to about 20 wt% of a solid lubricant filler based on a total weight of the first compound composition. In yet further aspects, the first polymer layer can also comprise at least one tackiness reducing compound in an amount from about 1 wt% to about 20 wt%. In still further aspects, the solid lubricant filler can comprise one or more of graphene, reduced graphene oxide, carbon black, boron nitride, silicones, talc, polytetrafluorethylene (PTFE), fluorinated ethylene propylene, and the like. In yet further aspects, the inorganic filler can comprise bismuth oxychloride, barium sulfate, bismuth subcarbonate, calcium carbonate, aluminum trihydrate, barite, kaolin clay, limestone, or any combination thereof. In still further aspects, and as disclosed above, the least one tackiness reducing compound comprises ProPell^{™},

In yet further aspects, the outer jacket **140** can comprise a second polymer layer. In such aspects, the second polymer layer can have any composition, as disclosed above. For example, and without limitation, the second polymer layer can comprise a second compound composition comprising from 0 wt% to 100 wt% of a second polymer comprising polyether block amide, a polyurethane, or a composition thereof. In still further aspects, the second compound composition is substantially free of inorganic filler. While in other aspects, the second compound composition is substantially free of the solid lubricant filler.

The outer jacket **140** can comprise the first and/or the second polymer layer with a wall thickness ranging between about 1 mil to about 5 mils, including exemplary values of about 1.5 mils, about 2 mils, about 2.5 mils, about 3 mils, about 3.5 mils, about 4 mils, about 4.5 mils, and about 4.9 mils. The wall thickness is measured radially between the inner surface of the outer jacket **140** and the outer surface of the outer jacket **140.**

In alternative aspects, the first (and/or second) polymer layer composition and/or wall thickness can change along the length of the outer jacket **140.** For example, the outer jacket (elongated tube) **140** can be provided with one or more segments, where the composition and/or thickness changes from segment to segment. In an example aspect, the Durometer rating of the composition changes along the length of the outer jacket **140** such that segments near the proximal end comprise a stiffer material or combination of materials, while segments near the distal end comprise a softer material or combination of materials. Similarly, the wall thickness of the outer jacket **140** the wall thickness of the outer jacket **140** in segments near the proximal end can be thicker/greater than the wall thickness of the outer jacket **140** near the distal end.

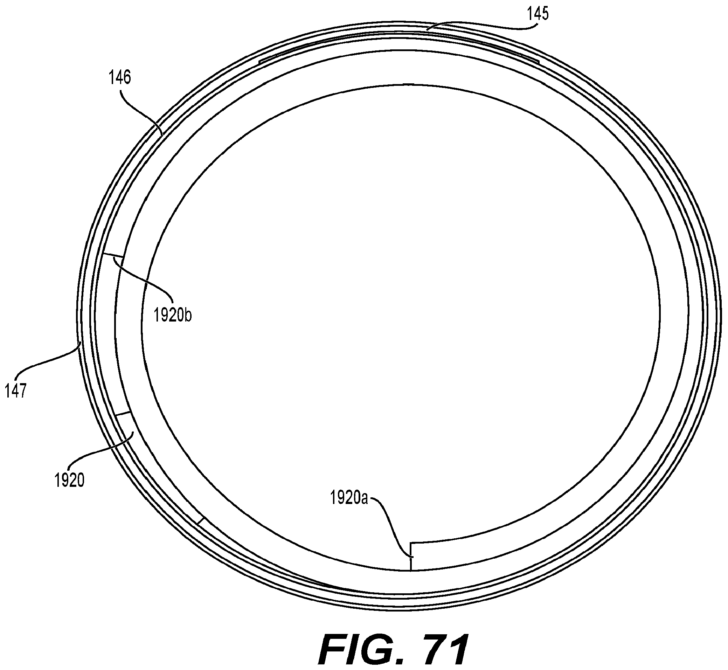

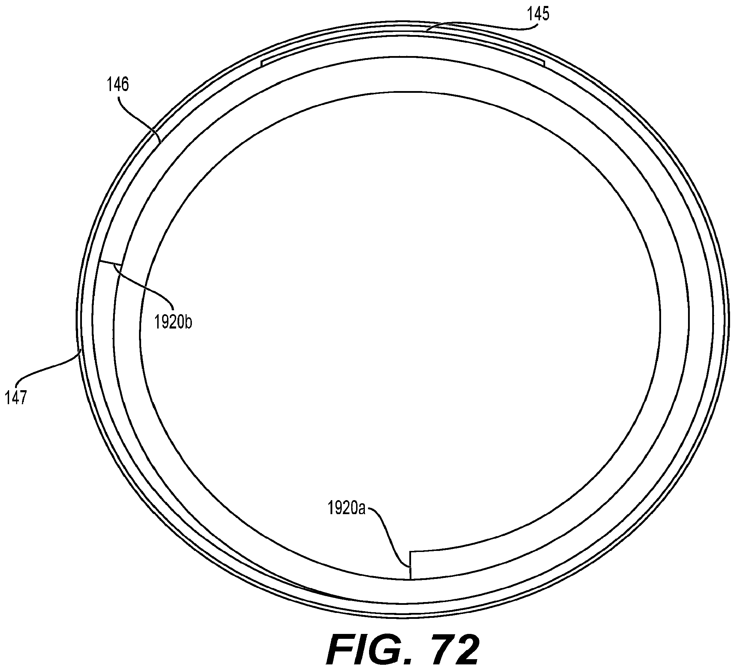

As illustrated in **FIG. 71-72****,** the outer jacket **140** includes one or more axial reinforcing members **145** that extend longitudinally along all or a portion of the outer jacket **145.** The reinforcing member **145** helps to prevent axial bunching of the outer jacket **140** during insertion into the patient's vasculature while not sacrificing the low radial expansion force of the outer jacket **140.**

As illustrated in **FIG. 35****,** the sheath **100** can include a tapered segment adjacent the flared end portion **114** at the proximal end of the sheath **100.** Referred to as a strain relief section, the tapered segment and the flared end portion **114** help ease the transition between the smaller diameter portion of the sheath **100** and the housing **101**. The thickness and/or composition of the outer jacket **140** can be adjusted to improve the performance of the strain relief section and to reduce the push force as disclosed above.

The outer jacket (or elongated tube) **140** can be bonded to the outer layer **110** to prevent the elongated tube **140** from sliding over the outer layer **110** and "bunching up" in response to the friction forces applied by the surrounding tissue during insertion of the sheath **100** into the patient's vasculature. For example, the outer jacket (the elongated tube) **140** can be bonded at the proximal end and/or distal end of the outer layer **110.** At the proximal and distal ends, the outer jacket **140** can be bonded to the outer layer **110** around the full circumference of the outer layer. At the distal end of the sheath **100,** the outer jacket (the elongated tube) **140** can alternatively be bonded to the inner layer **108**. For example, the outer jacket (the elongated tube) **140** can be bonded to the distal end surface of the inner layer **108**.

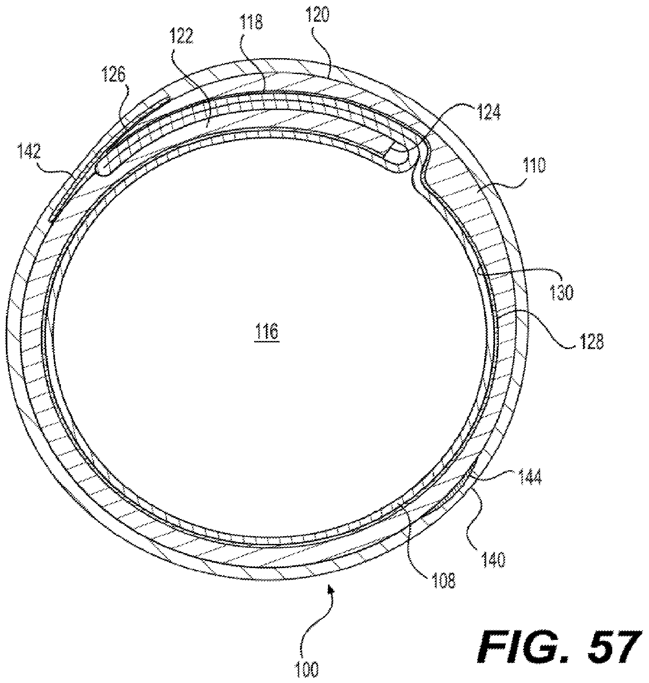

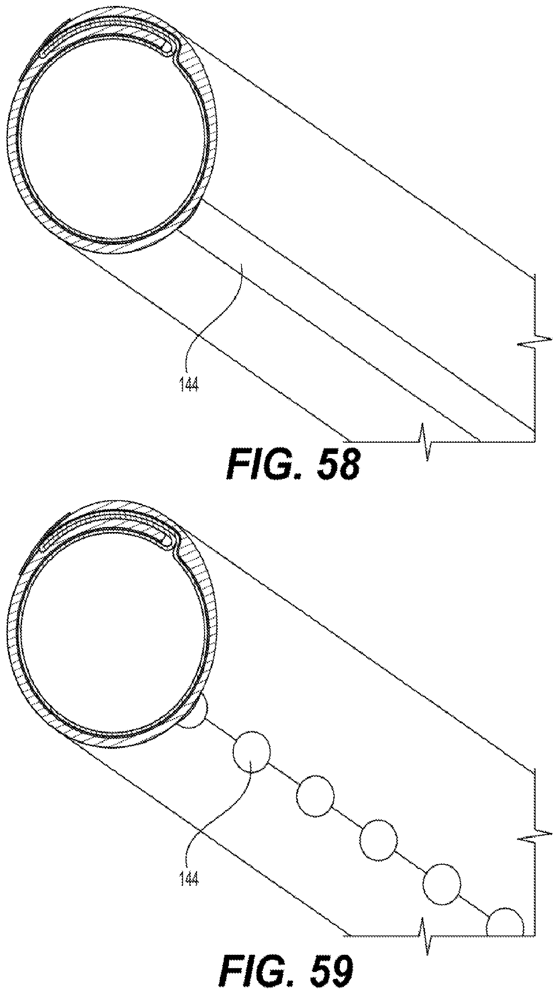

As illustrated in **FIG. 57****,** the outer jacket (elongated tube) **140** can be bonded **144** to the outer layer **110** at a circumferential location opposite the folded portion **118** of the inner layer **108**. As provided in **FIGS. 58-59****,** the bond **144** can be spot bonds or linear bond lines extending along all or a portion of the outer layer **110**. As provided in **FIG. 57****,** the bond **144** line/spot will also have a width, extending circumferentially around the outer layer **110**. For example, the bond line can cover about 5° to about 90° of the circumference of the outer layer 110 (including about 5°, about 10°, about 15°, about 20°, about 25°, about 30°, about 35°, about 40°, about 45°, about 50°, about 55°, about 60°, about 65°, about 70°, about 75°, about 80°, about 85°, about 90°).

The outer jacket (elongated tube) **140** can be bonded to the outer layer **110** and/or inner layer **108** using any mechanical and/or chemical (e.g., adhesive) fastener known in the art. In one example, sheath **100,** the outer jacket **140** and the outer layer **110** and/or inner layer **108** may have similar melting temperatures. Accordingly, an example bonding method includes a thermally bonded coupling between the outer jacket (elongated tube) **140,** the outer layer **110,** and/or inner layer **108**. For example, the bond between the outer jacket **140** and the outer layer **110** can be achieved by laser welding and/or a heat compression (e.g., using a heat compression jaw), allowing the location of the bond line to be closely controlled.

As shown in **FIG. 54**, and described above with respect to **FIG. 39****,** an adhesive layer **128** (e.g., a tie layer) is provided between the inner layer **108** and the outer layer 110 to at least partially adhere the inner layer **108** to the outer layer **110**. That is, the adhesive layer **128** is selectively provided/located between the inner layer **108** and the outer layer **110** to bond the inner and outer layers **108, 110** at the selected locations of the adhesive layer **128**.

As illustrated in **FIGS. 54** (and **FIG. 39**) the adhesive layer **128** is provided on the outer surface of the inner liner **108** and/or the inner surface **130** of the outer layer **110**. For example, the adhesive layer **128** can be provided partially or entirely around the outer surface of the inner liner **108**. Additionally, or alternatively, the adhesive layer **128** can be provided partially and/or entirely around the inner surface **130** of the outer layer **110.** As illustrated in **FIG. 54**, the adhesive layer **128** extends between the outer layer **110** and the overlapping folded portion **118** of the inner layer **108**. That is, the adhesive layer **128** extends between the outer surface of the folded portion **118** of the inner layer **108** and the corresponding inner surface of the overlapping portion **120** of the outer layer **110**. As illustrated in **FIG. 54**, the adhesive layer **128** does not extend between an inner surface of the overlapping folded portion **118** of the inner layer **108** and a corresponding surface of the underlaying portion **122** of the outer surface of the outer layer **110**. Excluding the adhesive layer **128** on the portion of the sheath between the inner surface of the folded portion **118** and the underlaying portion **122** facilitates expansion of the sheath and prevents undesirable bonding/sticking between the inner and outer layers **108, 110** at this location.

The adhesive material **128** can comprise a material having a Shore A hardness (durometer) less than about 90A. For example, the adhesive material **128** can comprise a thermoplastic polyurethane such as an aliphatic polyether-based thermoplastic polyurethane (TPU). An example of TPU includes Tecoflex^{™} 80A. The adhesive layer **128** can also be composed of an aromatic polyether or polyesters-based thermoplastic polyurethane such as, for example, Pellethane^{™} 80A. The adhesive layer can also be composed of a polyolefin or polyamide, including, for example, a polyolefin (PE, PP, or EVA) modified with maleic anhydride such as an Orevac^{™} resin.

The thickness (wall thickness) of the adhesive layer **128** can vary, but in some examples, the wall thickness of the adhesive layer ranges between about 0.002 inches and about 0.005 inches (including about 0.002 inches, about 0.003 inches, about 0.004 inches, about 0.005 inches). In other examples, the wall thickness of the adhesive layer **128** ranges between about 0.0025 and about 0.0040 (including about 0.0025 inches, about 0.0030 inches, about 0.0035 inches, about 0.0040 inches). In a further example, the wall thickness of the adhesive layer **128** ranges between about 0.0025 inches and about 0.0035 inches (including about 0.0025 inches, about 0.0030 inches, about 0.0035 inches).

In some examples, the sheath **100** can include a lubricant to reduce friction and facilitate expansion/contraction between the outer layer **110** and the outer jacket **140.** The lubricant **142** allows the outer layer **110** and inner layer **108** to unroll easily under the outer jacket (elongated tube) **140,** ensuring that the hemostasis and atraumatic benefits, achieved by the addition of an outer jacket **140,** do not compromise the push force performance of the sheath **100**. That is, the lubricant **142** reduced the push force necessary to move the prosthetic device through the central lumen **116** of the inner layer **108** during delivery of the prosthetic device and the corresponding local expansion of the sheath **100.**

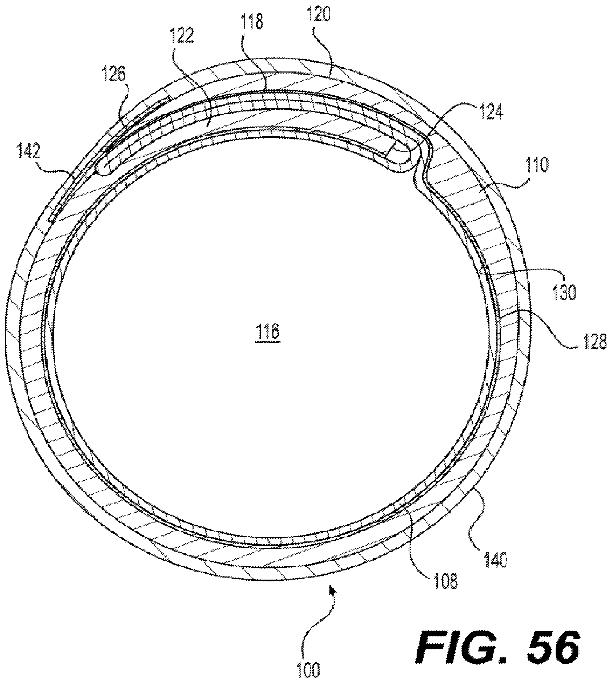

As illustrated in **FIG. 56****,** the lubricant **142** can be selectively applied along an outer surface of the outer layer **110** proximate the longitudinally extending edge **126** of the overlapping portion **120.** In some examples, a portion of the folded portion **118** of the inner layer **108** extends beyond the longitudinally extending edge **126** of the overlapping portion **120** and along an outer surface of the outer layer **110.** In this example, the lubricant **142** is also provided along the protruding portion, the folded portion **118** of the inner layer **108** extending along the outer surface of the outer layer **110** (beyond the edge **126**). In this location, the lubricant **142** also reduces friction between the outer jacket **140** and the inner layer **108** during expansion of the sheath **100.** As illustrated in **FIG. 56****,** the lubricant **142** extends around the circumference of the outer layer **110** beyond the protruding portion of the folded portion **118.**

The lubricant is applied as a band (or spot) that extends both circumferentially and longitudinally along the outer layer **110** (and the protruding portion of the inner layer 108). In an example sheath **100,** the lubricant **142** is applied as a band that extends both circumferentially around the outer layer **110** and longitudinally along a length of the outer layer **110.** To prevent migration, the lubricant **142** can be composed of a heat-curable material, e.g., a material curable at room temperature. As a result, the material can be applied to a desired location along the outer layer **110** and does not migrate during assembly and/or use of the sheath **100.** The lubricant **142** can be composed of a medical-grade lubricant, such as silicone. Example lubricants include medical-grade curable silicone lubricants, including a platinum catalyzed thermal curing silicone lubricant such as NuSil^{™} MED10-6670 (heat-curable), a PTFE lubricant such as Duraglide^{™} (curable at room temperature) and/or CHRISTO-LUBE^{™}.

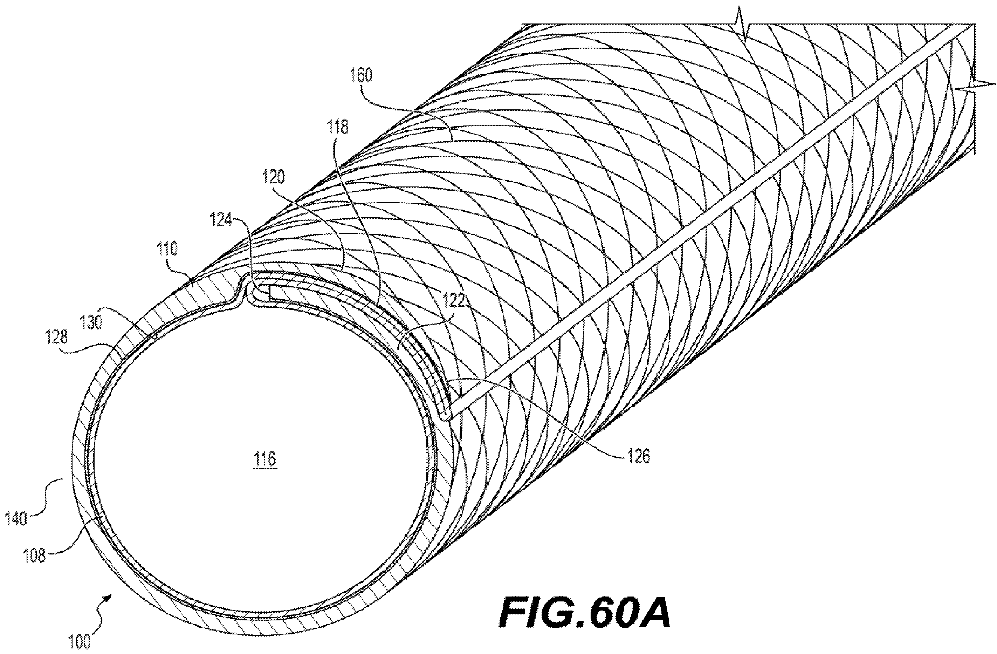

**FIG. 60A** illustrates an additional aspect of the sheath **100** of **FIG. 35****.** In this example, the sheath **100** can include a coiled wire **160,** or coiled wire mesh, along a length of the sheath **100.** The coiled wire **160** provides uniform bending of the sheath and prevents kinking. The coiled wire **160** can be embedded in the outer layer **110.** For example, the coiled wire **160** can be co-extruded with the outer layer 110. Alternatively, the coiled wire **160** can be provided between the outer layer and the adhesive layer **128.** In another example, the coiled wire **160** is embedded, at least partially, within both the outer layer **110** and the adhesive layer **128**. For example, the coiled wire **160** can be provided on an outer surface of the adhesive layer **128,** and the outer layer **110** is reflowed over.

As illustrated in **FIG. 60A****,** the coiled wire **160** defines a helical-shaped path around the longitudinal axis of the sheath **100**. The example of coiled wire **160** includes an overlapping helical-shaped path around the longitudinal axis of the sheath **100,** resulting in a continuous diamond-shaped pattern along the length of the sheath.

The coiled wire **160** can be comprised of a metal or a polymer wire. For example, the coiled wire **160** can be composed of PET, PEEK, stainless steel, and/or nitinol. The coiled wire **160** can be comprised of a flat wire, a round wire, or a combination thereof. The individual wires of the coiled wire **160** can have a diameter/thickness ranging between about 0.002 inches and about 0.008 inches (including about 0.002 inches, about 0.003 inches, about 0.004 inches, about 0.005 inches, about 0.006 inches, about 0.007 inches, about 0.008 inches). In another example, the individual wires of the coiled wire 160 can have a diameter/thickness ranging between about 0.004 inches and about 0.007 inches. In a further example, the individual wires of the coiled wire 160 can have a diameter/thickness of about 0.006 inches. The pitch/distance between adjacent coils of the coiled wire **160** can correspond to the diameter/thickness of the coiled wire. For example, where the diameter/thickness of a single coil of the coiled wire is about 0.006 inches, the spacing/pitch between the wire and the next adjacent coiled wire is about 0.006 inches.

Methods of using the sheath of **FIGS. 53-60A** include first inserting the expandable sheath **100** into the vasculature of a subject and advancing a prosthetic device through the inner lumen **116** of the inner layer **108**/sheath **100**. The prosthetic device applies an outwardly directed radial force on the inner layer **108** of the expandable sheath **100.** In some aspects, the outwardly directed radial force is transmitted through the inner layer **108,** the adhesive layer **128,** and the outer layer **110.** The lumen **116** of the sheath **100** expands at the axial location of the prosthetic device due to the outwardly directed radial force exerted by a prosthetic device against an inner surface of the lumen during advancement. During expansion of the lumen **116,** the first fold (folded edge) of the folded portion **118** is moved circumferentially closer to the second fold (folded edge), shortening the overlapping portion of the folded portion 118 that extends circumferentially between the first and second folds, thereby increasing the circumference of the lumen **116.**

As the sheath **100** expands at a particular location (i.e., locally expands at the location of the passing prosthetic device), the overlapping portion **120** of the outer layer **110** can move circumferentially with respect to the underlaying portion **122** as the folded portion **118** of the inner layer **108** least partially separate and/or unfold, causing the elongate gap(s) **132** provided in the outer layer **110** to widen/expand. The sheath thereby expands to accommodate a medical device having a diameter larger than that of lumen **116** in the resting (unexpanded) configuration. As shown in **FIG. 55****,** in some aspects, the folded portion of the inner layer **108** can completely unfold so that the inner layer **108** forms a cylindrical tube at the location of the expanded configuration. As illustrated in **FIGS. 54** and **55****,** the elongate gap **132** is generally aligned with the longitudinal axis of the lumen **116** such that during expansion, the unfolded portion of the inner layer **108** expands into the gap **132.**

In an unexpanded configuration, the sheath **100** can have an outer diameter less than about 0.30 inches (including less than about 0.29 inches, less than about 0.28 inches, less than about 0.27 inches, less than about 0.26 inches, less than about 0.25 inches, less than about 0.24 inches). Preferably, the unexpanded sheath has an outer diameter ranging between about 0.24 inches and about 0.26 inches. In a fully expanded configuration, the sheath **100** can have an inner diameter greater than 0.300 inches. Preferably, the expanded sheath **100** has an inner diameter ranging between 0.325 inches and 0.400 inches (including about 0.325 inches, about 0.327 inches, about 0.330 inches, about 0.333 inches, about 0.336 inches, about 0.340 inches, about 0.345 inches, about 0.350 inches, about 0.355 inches, about 0.360 inches, about 0.365 inches, about 0.370 inches, about 0.375 inches, about 0.380 inches, about 0.385 inches, about 0.390 inches, and about 0.395 inches).

As described above, the inner and outer layers **108, 110** can be bonded together using an adhesive layer **128**. The adhesive layer **128** prevents movement, both longitudinal and radial, between the inner and outer layers **108, 110**. As a result, expansion of the sheath 100 can be limited to only those regions excluding the adhesive layer **128**. For example, as illustrated in **FIG. 54**, because the adhesive layer **128** is not provided between the inner surface of the folded portion **118** and the underlaying portion **122** of the outer layer, expansion of the sheath results in the inner surface of the folded portion extending into the gap **132** created between the first and second edges **124, 126** of the expanded outer layer **110**.

Once the prosthetic device is passed through the lumen **116** (or a particular location along the lumen), the lumen **116** of the sheath can at least partially contracts back to an unexpanded configuration. The outer layer **110** can exert an inwardly directed radial force on the inner layer **108** urging it back to its original folded configuration. Similarly, if a coiled wire **160** was included, the coiled wire can exert an inwardly directed radial force on the outer layer **110** and the inner layer **108** urging them back towards an unexpanded configuration. In still further aspects, the outer jacket **140** can exert an inwardly directed radial force on the outer layer **110** and the inner layer **108** urging them back to an unexpanded configuration.

The prosthetic device can be delivered through the distal end of the sheath **100** to the delivery site within the patient. The prosthetic device can include a self-expanding heart valve or a stent-mounted heart valve. The heart valve can be extended through the distal end of the elongate lumen **116** at the delivery site. Once outside the lumen **116,** the heart valve can be expanded, and the sheath **100** removed from the treatment site.

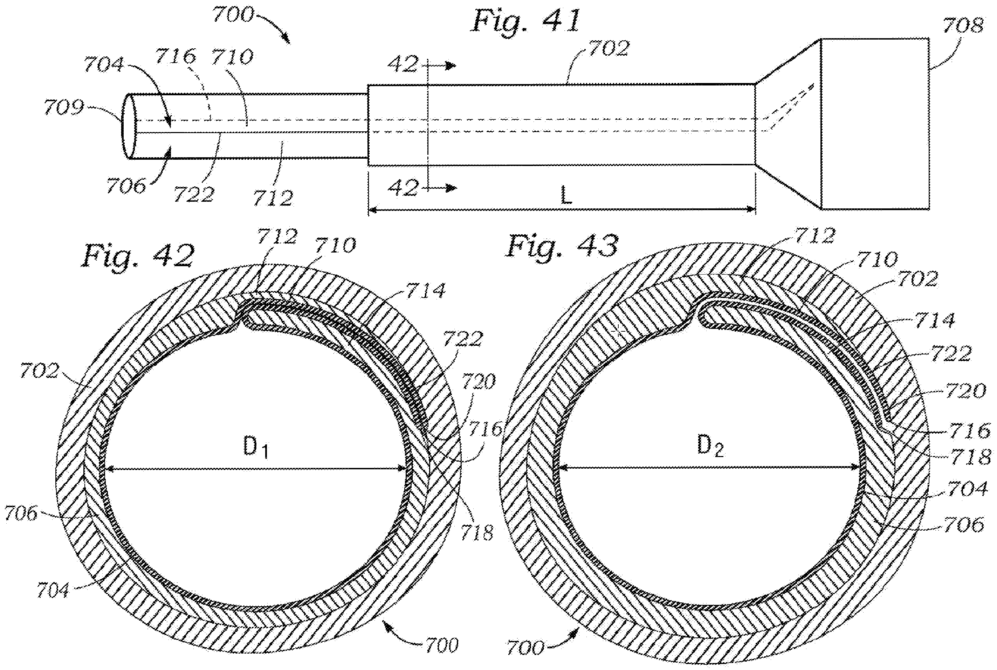

**FIGS. 41-49** illustrate additional aspects and variations on the general sheath **100** described above. It is to be understood that the variations (e.g., materials and alternate configurations) described above with reference to any previously disclosed figures and aspects can also apply to the aspects shown in **FIGS. 41-49** and vice versa.

**FIGS. 41-43** illustrate a sheath **700** that additionally includes a strain relief cover comprising disclosed herein elongated tube **702** positioned around at least a part of an inner layer **704** and outer layer **706.** As shown in **FIG. 41****,** the elongated tube (the outer jacket or a strain relief jacket) **702** can extend for a length L along at least a portion of the main body of the sheath **700.** In some aspects, the elongated tube **702** can extend from the proximal end **708** of the sheath **700** and towards the distal end **709** of the sheath. In some aspects, the elongated tube **702** extends only part way down the length of the sheath **700.** In alternate aspects, the elongated tube **702** can extend to a point adjacent the distal end **709** or can extend all the way to the distal end **709** of sheath **700.** Furthermore, the elongated tube **702** need not extend all the way to the proximal end **708** of the sheath **700.** In some aspects, the elongated tube **702** can extend only part way towards the proximal end **708.** In some aspects, the longitudinal length L of the elongated tube **702** can range from about 10cm to the entire length of the sheath **700.**

As shown in **FIGS. 42 and 43****,** the elongated tube **702** can be a continuous tubular layer without slits or other discontinuities. The elongated tube **702** can be positioned to surround the entire circumference of outer layer **706** and can extend longitudinally along any portion of the length of the sheath **700.** The elongated tube **702** can comprise any of the disclosed above compositions.

The elongated tube **702** can, in some aspects, provide hemostasis (e.g., prevent blood loss during implantation of the prosthetic device). For example, the elongated tube **702** can be sized or configured to form a seal with the patient's artery when inserted, such that blood is substantially prevented from flowing between the elongated tube **702** and the vessel wall. The elongated tube **702** can be inserted such that it passes the arteriotomy. For example, in aspects where the elongated tube **702** does not extend all the way to the distal end **709** of the sheath **700,** the elongated tube **702** can extend distally far enough such that when the sheath **700** is fully inserted into the patient, at least part of the elastic outer cover extends through the ateriotomoy site.

The elongated tube **702** can be configured to expand as the sheath expands, as shown in the expanded configuration in **FIG. 43**.

**FIG. 42** shows a cross-section of the sheath **700** in a resting configuration having an inner diameter D₁. **FIG. 43** shows a cross-section of the sheath **700** in an expanded configuration, having an inner diameter D₂, where D₂ is greater than D₁. Similar to the aspect of **FIGS. 35-40****,** the sheath **700** can include an inner layer **704** having a folded portion **710,** and an outer layer **706** having an overlapping portion **712** and an underlaying portion **714**. The overlapping portion **712** overlaps at least a portion of the folded portion **710** of the inner layer, and the underlaying portion **714** underlies at least a portion of the folded portion **710.** As shown in **FIGS. 42-43****,** in some aspects, the overlapping portion **712** does not overlap the entire folded portion **710** of the inner layer **704,** and thus a portion of the folded portion **710** can be directly adjacent to the elongated tube **702** in locations where the elongated tube **702** is present. In locations where the elongated tube **702** is not present, part of the folded portion **710** may be visible from the outside of the sheath **700,** as seen in **FIG. 41****.** In these aspects, the sheath **700** can include a longitudinal seam **722** where the overlapping portion **712** terminates at the folded portion **710.** In use, the sheath can be positioned such that the seam **722** is posterior to the point of the sheath that is 180 degrees from the seam **722** (*e.g.*, facing downward in the view of **FIG. 41**). The seam **722** can also be seen in **FIG. 41****,** which shows that the seam **722** need not extend the entire length of the sheath. In some aspects, the proximal end portion of the sheath includes two layers without a folded portion (*e.g.*, similar to **FIG. 38**), while the distal end portion of the sheath includes two layers with a folded portion (*e.g.*, similar to **FIG. 39**). In some aspects, the seam **722** can end at a transition point between portions of the sheath having a folded inner layer and portions of the sheath not having a folded inner layer.

In some aspects, the folded portion **710** can include a weakened portion, such as a longitudinal perforation, score line, and/or slit **716** along at least a portion of the length of the inner layer **704.** The slit **716** can allow for two adjacent ends **718, 720** of the folded portion **710,** to move relative to one another as the sheath **700** expands to the expanded configuration shown in **FIG. 43**. As a device having an outer diameter device larger than the initial resting inner diameter of the sheath **700** is inserted through the sheath **700,** the device can cause local expansion of the sheath **700** and cause the sheath **700** to expand at the partial score or split line location **716**. The weakened portion **716** can extend longitudinally along any portion of the expandable sheath **700.**

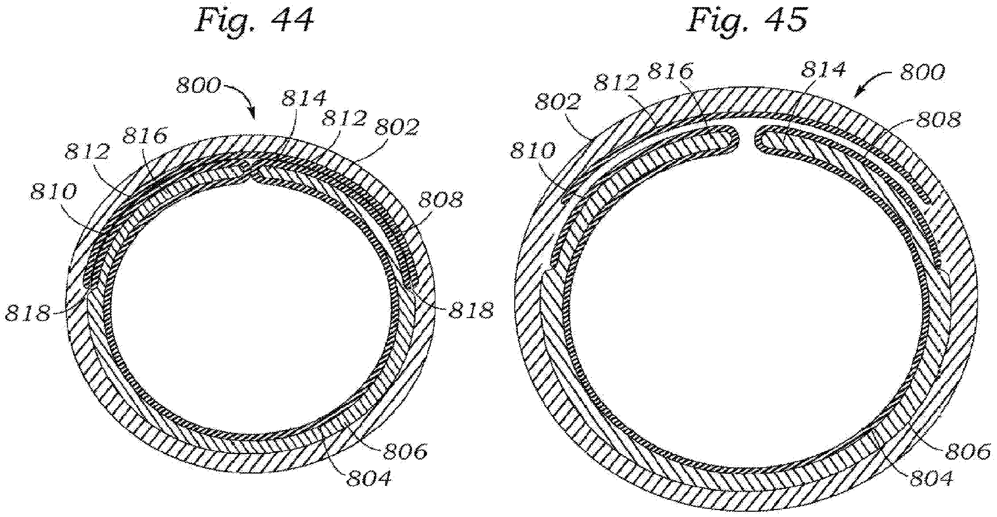

**FIGS. 44** and **45** show another aspect of an expandable sheath **800** having an initial diameter in a resting configuration (**FIG. 44**) and a larger expanded diameter in an expanded configuration (**FIG. 45**). The sheath **800** can include an elongated tube, as disclosed above, **802,** an inner layer **804,** and an outer layer **806**. Inner layer **804** can include first and second folded portions **808, 810.** The folded portions **808, 810** can be arranged such that they fold away from one another in opposite directions around the circumference of the sheath **800**. For example, folded portion **808** can be folded to the right in the view of **FIG. 44** and folded portion **810** can be folded to the left such that they do not overlap one another but share a common segment **812,** which is part of both folded portions **808, 810.** In contrast to previous aspects, the outer layer **806** does not include an overlapping portion in this aspect but rather has first and second underlaying portions **814, 816,** which underlie the first and second folded portions **808, 810,** respectively. The inner layer **804** can extend through a gap between the ends of the adjacent underlaying portions **814, 816** (*e.g.*, between a first end and a second end of discontinuous outer layer **806**).

Each folded portion **808, 810** can include a weakened portion **818,** such as a slit, score line, and/or perforation. Weakened portion **818** can allow the expandable sheath **800** to expand easily without a high radial force. As the sheath **800** expands, segment **812** along the top of the folded portions **808, 810** of inner layer **804** can be configured to split apart from the rest of the folded portions **808, 810** and the first and second underlaying portions **814, 816** can move away from one another so as to create an enlarged lumen within the inner layer **804**. Weakened portions **818** can allow for segment **812** to easily split apart from the inner layer **804** as the sheath **800** expands.

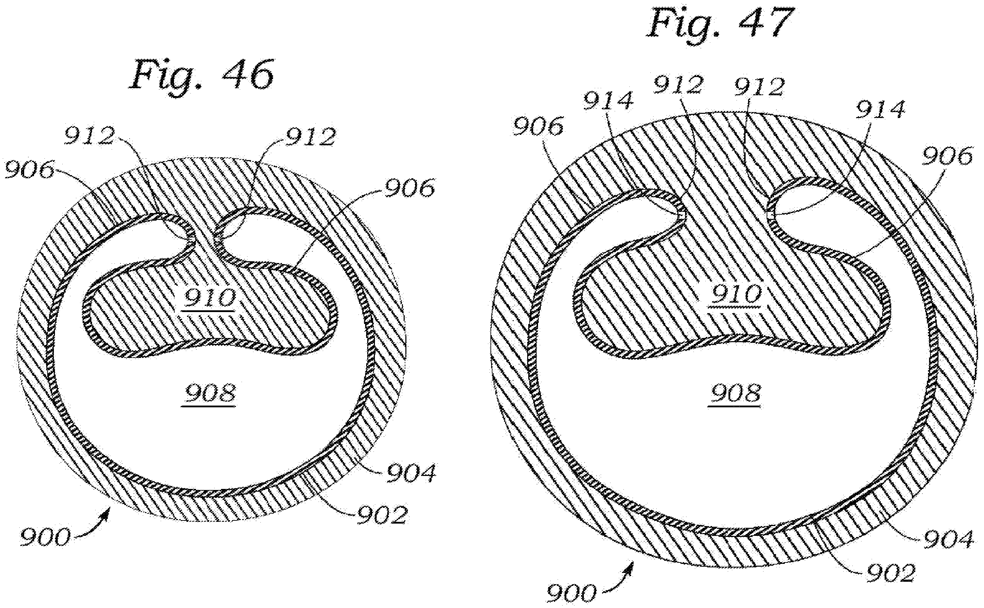

**FIGS. 46-47** show another aspect of an expandable sheath **900**. Sheath **900** can be provided with an inner layer **902** and an elongated tube **904** surrounding the inner layer **902**. While not shown, sheath **900** can additionally include an intermediate layer positioned between the inner layer **902** and the elongated tube **904**. If present, the intermediate layer can closely follow the contour of the inner layer **902**.

Inner layer **902** can be shaped to include one or more folded portions **906** arranged to form a generally horseshoe-shaped lumen **908** that extends longitudinally through sheath **900** along the inner surface of the inner layer **902**. The folded portions **906** can be arranged to form an area **910** positioned with the lumen **908** and radially inward from the elongated tube **904**. In some aspects, the area **910** can include one or more voids (*e.g.,* smaller lumens or openings extending through portion **910**). In some aspects, the area **910** can be filled with material (*e.g.,* HDPE) reflowed from an intermediate layer while the sheath is being made. In some aspects, area **910** can be filled with material reflowed from the elongated tube **904** during the sheath manufacturing process.

The inner layer **902** can include one or more weakened portions **912,** such as score lines, perforations, or slits. The weakened portions **912** can be configured to split apart, separate, or widen as the sheath expands from its initial resting configuration (**FIG. 46**) to an expanded configuration (**FIG. 47**) in the presence of a radial force. As the sheath **900** expands, material from the area **910** can cover any gaps **914** formed at the weakened portions **912,** thereby keeping the lumen **908** substantially sealed.

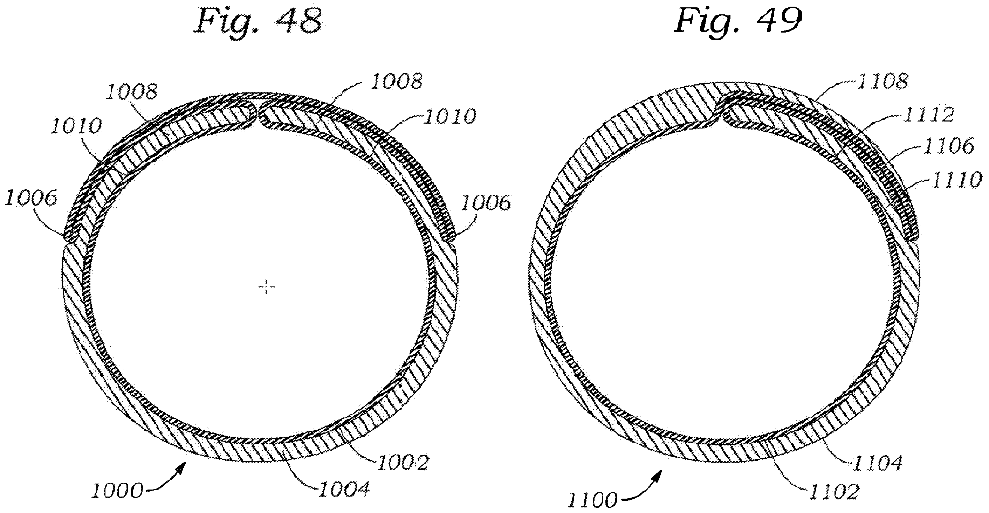

**FIG. 48** shows another aspect of an expandable sheath **1000** having an inner layer **1002** and a discontinuous outer layer **1004.** Sheath **1000** is similar to the sheath **800** of **FIG. 44****,** except that sheath **1000** is shown without the disclosed herein elongated tube behaving as an outer jacket and further, the inner layer **1002** is continuous, without weakened portions at the folds **1006.** As shown in **FIG. 48****,** the inner layer **1002** can be configured to have one or more folds **1006** (*e.g*., two folds positioned on the outer surface of the outer layer **1004**), with portions **1008** of the outer layer **1004** extending between the folds **1006** and the outer surface **1010** of the inner layer **1002** underlaying the folds **1006.**

**FIG. 49** shows yet another aspect of an expandable sheath **1100** having an inner layer **1102** and an outer layer **1104.** The sheath **1100** is similar to the sheath **100** shown in **FIG. 39** in that the inner layer **1102** can be continuous with a folded portion **1106,** and the outer layer **1104** can be discontinuous with an overlapping portion **1108** overlapping at least a part of the folded portion **1106** and an underlaying portion **1110** underlaying at least a part of the folded portion **1106.** The underlaying portion **1110** can thus be positioned between an outer surface **1112** of the lumen-forming portion of the inner layer **1102** and the folded portion **1106.**

The inner layers **1002, 1102** of the sheaths **1000, 1100,** respectively, of **FIGS. 48-49** can be optimized to perform slightly differently than the inner layers of sheaths described above. For example, different materials can be used for the inner liner to increase the durability and softness of the seam (although such materials can also be used with the other aspects of expandable sheaths described above). For example, materials such as woven fabrics or braid filaments can be used. Such fabrics, filaments, or yarns can comprise, for example, PTFE, PET, PEEK, and/or nylon yarns or filaments. These materials can advantageously provide a soft and flexible layer that can be easily formed into the desired shapes or folded portions. Additionally, such materials can withstand high temperatures, as well as can possess high tensile strength and tear resistance. Nonetheless, these materials can also be elastic, experience minimal kinking, and provide soft distal edges for less traumatic insertion into a patient's vessels.

It is further understood that the aspects disclosed herein that describe the presence of the outer layer in addition to the inner layer and the elongated tube (the outer jacket) are exemplary. Also described herein are aspects where this outer layer is substituted fully by an elongated tube comprising the disclosed herein composition. In such aspects, the elongated tube can have a structure and characteristics of the as disclosed herein outer layer. It is further understood that in such exemplary and unlimiting aspects, the additional elongated tube can be present and behave as disclosed herein outer jacket or a strain relief jacket.

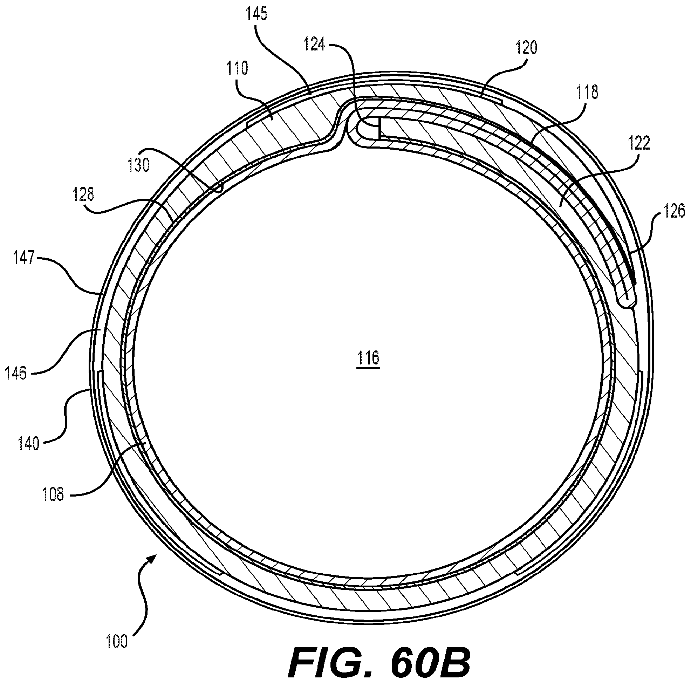

The elongated tube of this current disclosure can be used as an outer jacket **140** in various sheath configurations, as shown in **FIG. 60B** and **FIGs. 71****-77.** The sheath comprising the elongated tube (or as used interchangeably, "outer jacket") of the current disclosure can also comprise one or more axial reinforcing members **145** that extend longitudinally along all or a portion of the outer jacket **140.** The layer **145** can be disposed in the first polymer layer, in the second polymer layer, or between the first and second layers.

It is understood that the axial reinforcing members as used herein can also be used interchangeably with the term "at least one intermediate reinforcement layer." In certain aspects, the at least one intermediate reinforcement layer can be presented as a strip. While in yet other aspects, the at least one intermediate reinforcement layer can be a thermally bondable layer. Yet, in still further aspects, this thermally bondable layer can be presented as a strip.

The reinforcing member **145** provides stiffness and prevents axial bunching of the outer jacket **140** during insertion into the patient's vasculature while not sacrificing the low radial expansion force of the outer jacket **140.**

As illustrated in **FIG. 35****,** the sheath **100** can include a tapered segment adjacent the flared end portion **114** at the proximal end of the sheath **100.** Referred to as a strain relief section, the tapered segment and the flared end portion **114** help ease the transition between the smaller diameter portion of the sheath **100** and the housing **101.** The thickness and/or composition of the outer jacket **140** can be adjusted to increase the Durometer and/or stiffness along the strain relief section. Because this portion of the sheath **100** is usually outside of the patient's body during the procedure, providing the outer jacket **140** with an increased Durometer and/or stiffness along the strain relief section helps to withstand the blood pressure that would otherwise cause the outer jacket **140** to "balloon up" with body fluid/blood. As a result, it allows for a sheath **100** having a relatively stiff proximal end at the point of introducing a delivery apparatus while still having a relatively soft distal tip at the point of entry into the patient's vessel.

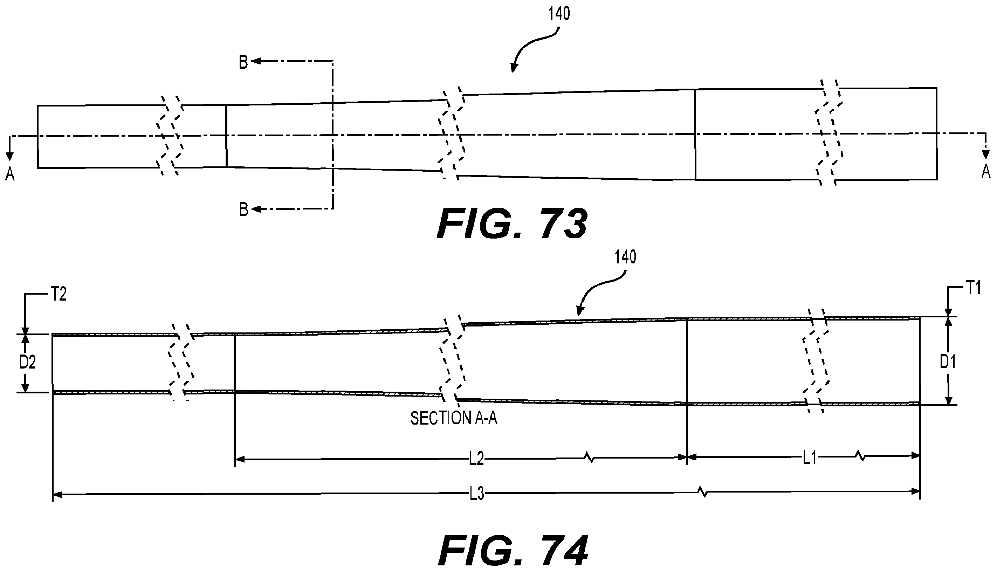

As disclosed herein, the elongated tube or an outer jacket can have the same diameter across the length of the sheath or can have varying diameters across the length of the sheath. **FIG. 73** is an elevation view of the outer jacket **140** showing a tapered segment adjacent the flared end portion at the proximal end of the sheath. **FIG. 74** is a cross section view of the outer jacket **140** taken along section A-A in **FIG. 73****.** As described above, the tapered portion is referred to as a strain relief section, and the tapered segment and the flared proximal end help ease the transition between the smaller diameter portion of the sheath **100** and the housing **101.** The length of the proximal end (L1) can range from 1.600 inches to 2.400 inches. In some aspects, the length of the proximal end is about 2.000 inches. The length of the tapered segment (L2) can range from 2.000 inches to 3.000 inches. In some aspects, the length of the tapered segment (L2) is about 2.500 inches. The overall length of the outer jacket **140**/sheath 100 (L3) can range from 17.600 inches to 26.400 inches. In some aspects, the overall length of the outer jacket **140**/sheath 100 (L3) is about 22.000 inches.

As provided in **FIG. 73****,** the diameter of the outer jacket **140** at the proximal end is greater than the diameter of the outer jacket **140** at the distal end. This allows the outer jacket **140** to be slid over the inner and outer layers **108, 110** without having to be expanded. For example, the diameter of the outer jacket **140** at the proximal end can range from 0.264 inches to 0.396 inches. In some examples, the diameter of the outer jacket 140 at the proximal end is about 0.330 inches. The diameter of the outer jacket 140 at the distal end can range from 0.176 inches to 0.264 inches. In some examples, the diameter of the outer jacket at the distal end is about 0.220 inches.

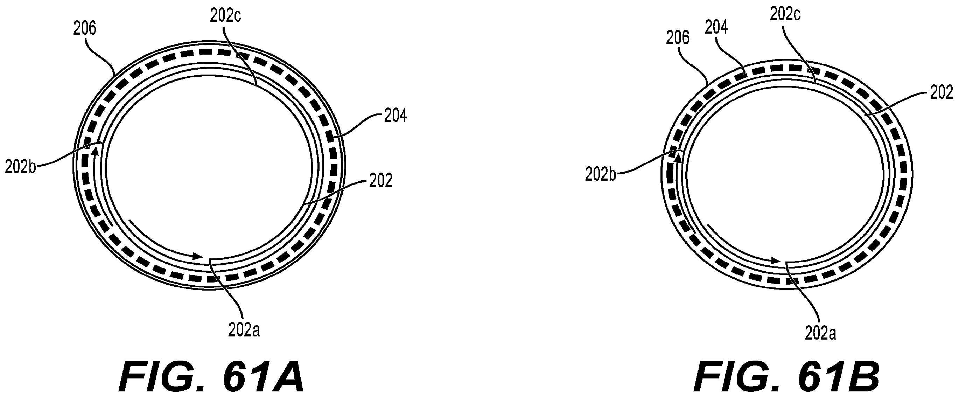

Additional aspects of the sheath comprising the disclosed herein elongated tube are shown in **FIGs. 61A-C.** In such aspects, the sheath further comprises a variable diameter inner liner comprising a sheet having a first edge and a second edge and is defined by an inner surface and an outer surface, wherein the sheet is wound in a spiral configuration such that at least a portion of the inner surface of the sheet overlays at least a portion of the outer surface of the sheet and wherein the first edge of the sheet is slidable along at least a portion the inner surface of the sheet and the second edge is slidable along at least a portion of the outer surface of the sheet, wherein the inner surface of the sheet defines a lumen of the cylinder having a longitudinal axis; wherein the variable diameter inner liner is configured to reversible expand from a predetermined rest diameter *dᵣ* to an expanded diameter *d₁* by sliding the first edge of the sheet along at least a portion of the inner surface and sliding the second edge of the sheet along the at least a portion of outer surface, during application of a radial outward force by passage of a medical device through the lumen of the inner liner; and wherein the elongated tube is positioned such that the inner surface of the elongated tube overlies at least a portion of the outer surface of the inner liner (layer).

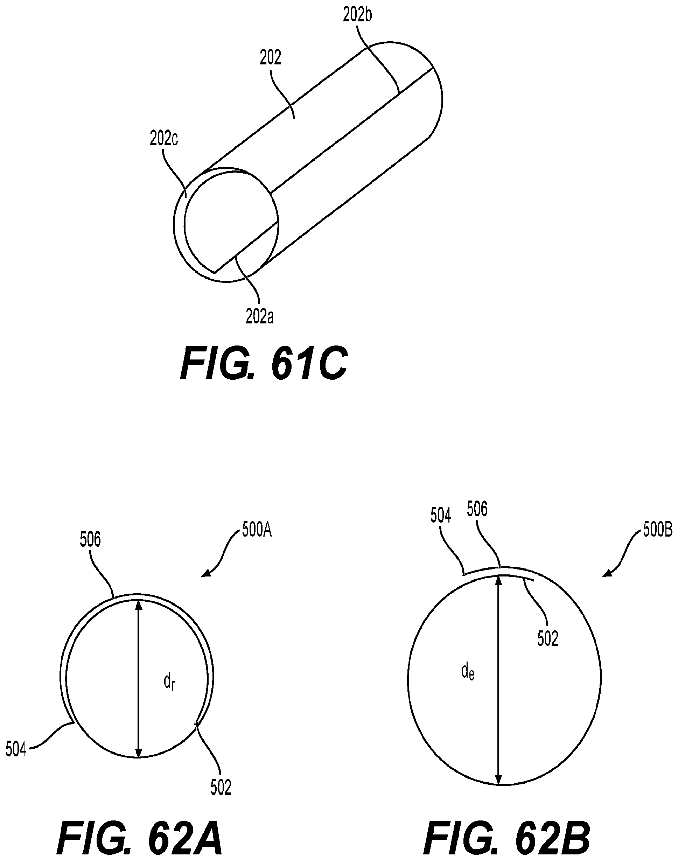

**FIGS. 61A** and **61B** show section views of aspects of two exemplary sheaths disclosed herein for use with a delivery apparatus such as that shown in **FIG. 1****.** **FIG. 61C** shows a perspective view of one aspect of an inner liner **202** for use with the disclosed sheath. As shown in **FIGS. 61A-C,** in some aspects, the disclosed sheath comprises an inner liner **202** wound in a spiral configuration such that at least a portion of the inner surface of the sheet overlays at least a portion of the outer surface of the sheet, forming an overlaying portion **202c,** and wherein the first edge **202a** of the sheet is slidable along at least a portion the inner surface of the sheet and the second edge **202b** is slidable along at least a portion of the outer surface of the sheet. Sheath, as shown in **FIG. 61A** and **FIG. 61B** can further include a braid **204** and an elongated tube, as disclosed herein, **206.** The braid **204** can be positioned between the inner liner and the elongated tube, for example, and as shown in **FIG. 61A****,** the braid **204** that is not embedded into the elongated tube **206.** While in the other aspect, and as shown in **FIG. 61B****,** the braid **204** can be at least partially embedded into the layer of the elongated tube **206.** In yet further aspects, the braid **204** and the elongated tube **206** form an outer layer of the sheath.

In still further aspects, the sheath, as disclosed herein, does not comprise the braid. In such exemplary and unlimiting aspects, the elongated tube **206** alone without the braid forms the outer layer of the sheath.

In still further exemplary and unlimiting aspects, an additional outer layer can be optionally positioned in between the inner liner and the elongated tube.

The inner liner **202** defines a lumen **201** through which a delivery apparatus can travel into a patient's vessel in order to deliver, remove, repair, and/or replace a prosthetic device. The disclosed sheath can also be useful for other types of minimally invasive surgery, such as any surgery requiring introduction of an apparatus into a subject's vessel. For example, the disclosed sheath also can be used to introduce other types of delivery apparatus for placing various types of intraluminal devices (e.g., stents, stented grafts, etc.) into many types of vascular and non-vascular body lumens (e.g., veins, arteries, esophagus, ducts of the biliary tree, intestine, urethra, fallopian tube, other endocrine or exocrine ducts, etc.). In still further exemplary aspects, the sheath can contract to the predetermined rest diameter *dᵣ* after passage of the medical device through the lumen.

In still further aspects, the sheet used to make the inner liner **202** can comprise a high-density polyethylene, polypropylene, polyamide, fluoropolymer, copolymers thereof, or blends thereof. In still further aspects, the sheet can comprise one or more layers. In some aspects, if one or more layers are present, each layer can comprise the same or different polymer. In still further aspects, the sheet can have a predetermined thickness, wherein the predetermined thickness can be defined by one of ordinary skill in the art depending on the specific application. In certain aspects, the predetermined thickness of the inner liner can be from about 0.002 inches to about 0.0025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner **202** can be varied depending on the desired amount of radial expansion, as well as the strength required.

In still further aspects, the internal surface of the sheet can be at least partially ribbed. In yet further aspects, the sheet can also be lubricious. In some exemplary aspects, the sheet that forms the inner liner can have a coefficient of friction less than about 0.5, less than about 0.4, less than about 0.3, less than about 0.2, less than about 0.1, or less than about 0.05, or even less than about 0.01. It is further understood that the sheet can have a coefficient of friction having any value between any two foregoing values. Such a liner can facilitate the passage of a delivery apparatus through the lumen **201** of the disclosed sheath. In some further exemplary aspects, materials that can be used to form suitable lubricious inner liners include materials that can reduce the coefficient of friction of the inner liner **202,** such as PTFE, polyethylene, polyvinylidene fluoride, and combinations thereof. Suitable materials for a lubricious liner also include other materials desirably having a coefficient of friction of about 0.1 or less, of about 0.09 or less, about 0.08 or less, about 0.07 or less, about 0.05 or less, about 0.04 or less, about 0.03 or less, about 0.02 or less, or about 0.01 or less.

In yet further aspects, the elongated tube can have any predetermined thickness. It is understood that the predetermined thickness of the elongated tube can be dependent on the specific application of the sheath. For example, and without limitation, the thicknesses of the inner liner **202,** the elongated tube **206,** and the braid **204** can also be varied depending on the particular application of the disclosed sheath. In some aspects, the thickness of the inner liner **202** ranges from about 0.0005 inches to about 0.010 inches, including exemplary values of about 0.0006, about 0.0007, about 0.0008, about 0.0009, about 0.001, about 0.002, about 0.003, about 0.004, about 0.005, about 0.006, about 0.007, about 0.008, about 0.009 inches, and in one particular aspect, the thickness can be about 0.002 inches. In yet other aspects, a total thickness of the elongated tube **206** and the braid **204** can have a thickness of from about 0.002 inches to about 0.015 inches, including exemplary values of about 0.003, about 0.004, about 0.005, about 0.006, about 0.007, about 0.008, about 0.009, and about 0.01 inches.

It is understood that the inner liner can have any shape or configuration depending on the desired application and the size of the delivery apparatus and prosthetic device. It is further understood that the inner liner is not limited to the specific shape or configuration. In certain aspects, the sheath disclosed herein is defined by the rest diameter *dᵣ,* and the outer diameter *dₒ.* As disclosed herein, the rest diameter *dᵣ* is defined by the inner liner, while the outer diameter can be defined by the inner liner and the outer layer, wherein the outer layer comprises the braid and the elongated tube.

The rest diameter *dᵣ* of the inner liner **202** can vary depending on the application and size of the delivery apparatus and prosthetic device. It is understood that in some aspects, the rest diameter *dᵣ* is substantially uniform along the longitudinal axis of the lumen without changing from the proximal end to the distal end. In yet other aspects, the rest diameter *dᵣ* can vary along the longitudinal axis of the lumen. In certain aspects, the rest diameter *dᵣ* at the proximal end is larger than the rest diameter *dᵣ* at the distal end. In yet further aspects, where the outer layer comprising the braid and the elongated tube conforms to the shape of the inner liner, the outer diameter *dₒ* (not shown) comprises the overall diameter of the inner liner and the outer layer. In such aspects, the outer diameter *dₒ* is defined by the specific application of the sheath.

Similar to the rest diameter *dᵣ,* the outer diameter *dₒ* of the unexpended sheath disclosed herein can be substantially uniform (constant) along the longitudinal axis of the lumen without changing from the proximal end to the distal end (not shown). In alternative aspects, the original unexpanded outer diameter *dₒ* of the disclosed sheath, similarly to the rest diameter *dᵣ,* can decrease from the proximal end to the distal end. In some aspects, and similarly to the rest diameter *dᵣ,* the original unexpanded outer diameter can decrease along a gradient, from the proximal end to the distal end; or it can incrementally step down along the length of the sheath having the largest original unexpanded outer diameter is near *dₒ* the proximal end, and the smallest original unexpanded outer diameter *dₒ* is near the distal end.

In some aspects, the rest diameter *dᵣ* can range from about 0.005 inches to about 0.400 inches, including exemplary values of about 0.01 about 0.02, about 0.03, about 0.04, about 0.05, about 0.06, about 0.07, about 0.08, about 0.09, about 0.1, about 0.2, and about 0.3 inches. As described above, in certain aspects, the sheath can comprise the inner liner having various *dᵣ.* In such aspects, the *dᵣ* can have any value between any two foregoing values and can depend on the specific application and the size and shape of the delivery apparatus and prosthetic device. Different sheaths can be provided with different expanded and unexpanded rest diameter *dᵣ* and outer diameter *dₒ*, depending on the size requirements of the delivery apparatus for various applications. Additionally, some aspects can provide more or less expansion depending on the particular design parameters, the materials, and/or configurations used.

As disclosed herein, the outer layer comprises a braid **204** and the elongated tube **206** having a predetermined thickness and having an inner surface and outer surface (as shown in **FIGs. 61A** and **61B**). In certain aspects, the braid can be an expandable braid. In yet further aspects, the braid can comprise at least one filament comprising stainless steel, nitinol, a polymer material, or a composite material. In certain unlimiting aspects, the braid comprises filaments comprising Nitinol and/or other shape memory alloys. In yet other unlimiting aspects, the braid can have filaments comprising polyester or nylon. In yet some other exemplary aspects, the braid can comprise filaments comprising spectra fiber, polyethylene fiber, aramid fiber, or combinations thereof. Again, as described above, the braid **204** is optional, and aspects without the braid are also disclosed.

It is understood that the braid can have any configurations known in the art. In certain aspects, the braid **204** is generally a thin, hollow, substantially cylindrical tube comprising an arrangement, pattern, structure, or configuration of filaments or struts, however other geometries can also be used. Suitable filaments can be round having a diameter less than about 0.015", less than about 0.01", less than about 0.008", less than about 0.005", less than about 0.002", less than about 0.001", less than about 0.0008", or less than about 0.0005". In yet other aspects, suitable filaments can be round and having a diameter ranging from about 0.0005" inches thick to about 0.015" thick, including exemplary values of about 0.0006", about 0.0007", about 0.0008", about 0.0009", about 0.001", about 0.002", about 0.003", about 0.004", about 0.005", about 0.006", about 0.007", about 0.008", about 0.009", about 0.01", about 0.012", about 0.013", and about 0.014". In yet other aspects, the suitable filaments can be flat filaments having a height of less than about 0.006", less than about 0.005", less than about 0.004", less than about 0.003", less than about 0.001", less than about 0.0009", less than about 0.0008", less than about 0.0007", less than about 0.0006", and about 0.0005". In yet other aspects, the flat filaments can have a width from greater than about 0.003" to about 0.015", including exemplary values of about 0.004", about 0.005", about 0.006", about 0.007", about 0.008", about 0.009", about 0.01", about 0.012", about 0.013", and about 0.014". However, other geometries and sizes are also suitable for certain aspects.

In yet further aspects, the braid can have a per-inch crosses (PIC) count of less than 50, less than 40, less than 30, less than 20, or less than 10. In yet other aspects, the braid can have the PIC count from 10 to 2, including exemplary values of 9, 8, 7, 6, 5, 4, and 3. In still further aspects, the PIC can vary along the longitudinal axis of the lumen. In yet other aspects, the braid pattern can vary along the longitudinal axis of the lumen. In the aspects where the braid comprises filament that is nitinol, the nitinol is heat-set at the expanded diameter *dₑ*. In yet further aspects, where the filament comprises stainless steel or nitinol, the filament is configured to be atraumatic, at least at the distal end of the sheath. **FIGS. 9-23** illustrate partial elevation views of various structures for the braid **28.** It is understood that the structure of the braid **28** can vary from section to section, changing along the length of the sheath. It is further understood that the structures shown in **FIGS. 9-23** are not necessarily drawn to scale and show just exemplary and unlimiting aspects. It is further understood that the braid is configured to provide the torquability of the sheath during the insertion of the prosthetic device. Again, it is understood that the presence of the braid in these aspects is optional, and aspect having a similar configuration without the presence of the braid is also disclosed.

It is understood that the elongated tube **206,** as shown in **FIGs. 61A** and **61B** can comprise any composition and exhibit any of the characteristics disclosed herein.

Alternative aspects of a sheath for introducing a prosthetic device are also described. For example, **FIGS. 62A-62B** illustrate a section view of the inner liners **500A** and **500B** of the disclosed sheath in unexpended and expended configurations **(****FIGS. 62A** and **62B****,** respectively). Upon introduction of the prosthetic device into the inner liner, the first edge **502** and the second edge **504** slid along and expand the inner liner from the rest diameter *dᵣ* to the expanded diameter *dₑ*, thereby shortening the overlaying portion **506** of the inner liner. It is understood that the expanded diameter *dₑ* is configured to accommodate the medical device passing through the lumen. In yet further aspects, the sheath contracts to the predetermined rest diameter *dᵣ* after passage of the medical device through the lumen.

In certain aspects, an amount of a first lubricant is disposed between at least a portion of the inner liner and at least a portion of the outer layer that comprises the braid and the disclosed elongated tube. In yet other aspects, an amount of a second lubricant is disposed between at least a portion of the overlying portion of the sheet and at least a portion of the sliding portions of the sheet. It is understood that the first lubricant and the second lubricant can be the same or different. In certain and unlimiting aspects, the first and/or second lubricants can comprise Christo Lube supplied by ECL or MED10/6670 supplied by Nusil. In still further aspects, it is understood that the amount of the first and/or second lubricant can be easily determined by one of ordinary skill in the art.

In still further aspects, the outer surface of the elongated tube defines at least a portion of the outer surface of the outer layer. In yet other aspects, at least a portion of the inner surface of the elongated tube is at least partially bonded to at least a portion of the outer surface of the sheet of the inner liner. It is understood that the outer layer of the disclosed sheath is configured to provide hemostasis and prevent bleeding of the patient during the procedure.

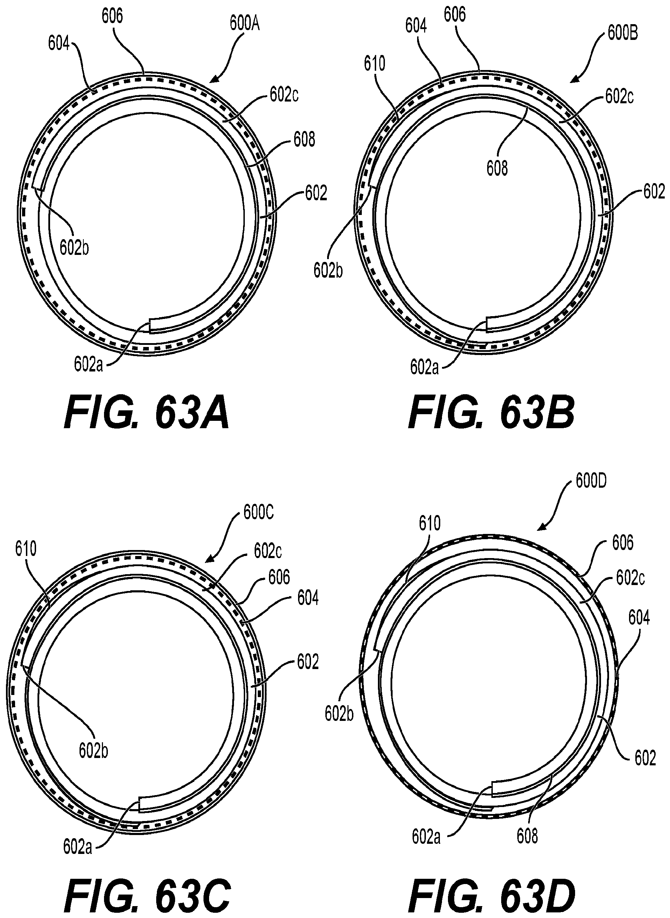

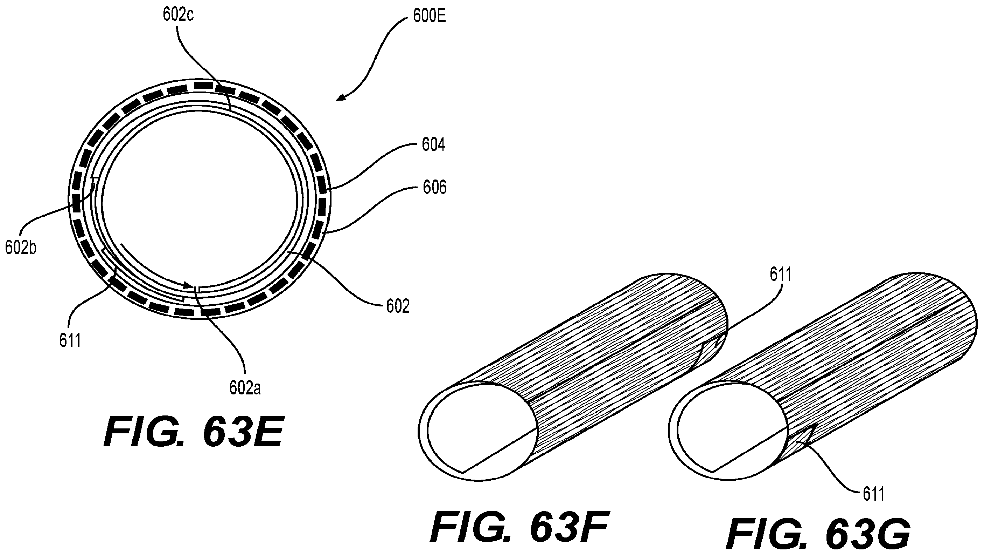

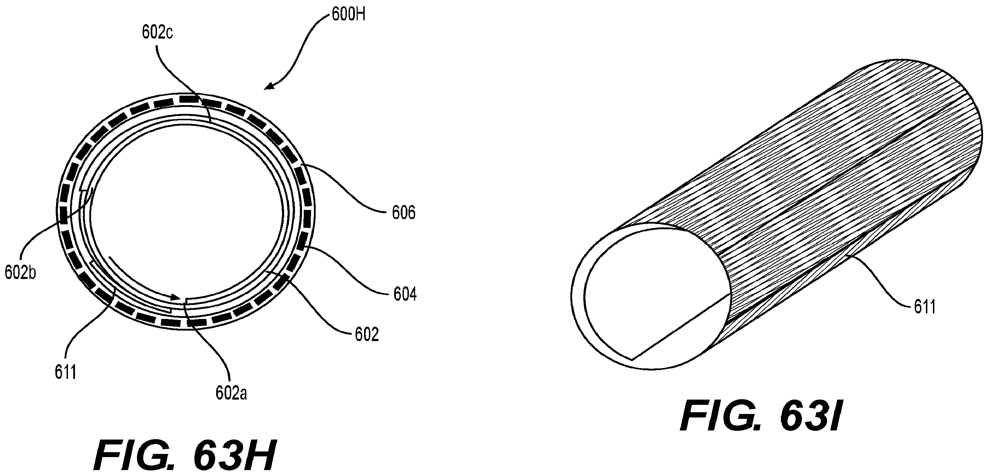

**FIGS. 63A-63I** show other alternative aspects of a sheath for introducing a prosthetic device. **FIG. 63A** shows the sheath **600A** comprising the inner liner **602** having the first edge **602a** and the second edge **602b,** and the overlaying portion **602c,** where the inner and outer surfaces of the inner liner overlay each other. The sheath **600A** further comprises an amount of the second lubricant **608** as disclosed herein that is disposed between the sliding and overlaying portions of the inner sheath. The sheath further comprises the braid **604** and the elongated tube **606.** In this exemplary aspect, the braid **604** is not embedded into the elongated tube **606.** **FIG. 63B** depicts an alternative aspect of the sheath **600B** where an amount of the first lubricant **610** is applied between the inner liner and the outer layer comprising the braid **604** and the elongated tube **606.** An additional aspect of the sheath **600C** is shown in **FIG. 63C****.** In this aspect, the sheath **600C** comprises the inner liner **602,** having the first edge **602a** and the second edge **602b,** and the overlaying portion **602c,** where the inner and outer surfaces of the inner liner overlay each other. The sheath further comprises the braid **604** and the elongated tube **606** that together can form the outer layer of the sheath. The sheath **600C** further comprises an amount of the first lubricant **610,** as disclosed herein, that is disposed between the outer layer and the inner liner of the inner sheath. In this exemplary aspect, the braid **604** is not embedded into the elongated tube **606.** In the exemplary aspect shown in **FIG. 63D****,** the exemplary sheath **600D,** comprises the braid **604** embedded within the elongated tube **606.**

In still further aspects, the sheath of the instant disclosure can comprise a hemostasis valve inside the lumen of the sheath, at or near the proximal end of the sheath (not shown). Additionally, the exemplary sheaths disclosed herein can comprise a soft tip at the distal end of the sheath (not shown). Such a soft tip can be provided with a lower hardness than the other portions of the sheath. In some aspects, the soft tip can have a Shore hardness from about 25 D to about 40 D, including exemplary values of about 26 D, about 27 D, about 28 D, about 29 D, about 30 D, about 31 D, about 32 D, about 33 D, about 34 D, about 35 D, about 36 D, about 37 D, about 38 D, and about 39 D. In yet other aspects, the soft tip can have a Shore hardness from about 25 A to about 40 A, including exemplary values of about 26 A, about 27 A, about 28 A, about 29 A, about 30 A, about 31 A, about 32 A, about 33 A, about 34 A, about 35 A, about 36 A, about 37 A, about 38 A, and about 39 A.

In certain aspects, the elongated tube and the inner liner can be bonded together or otherwise physically associated with one another. It is understood that the amount of adhesion between the inner liner **602** and the outer polymer layer that comprises braid **604** and the elongated tube **606** can be variable over the surfaces of the layers. The bonding between the layers can be created by, for example, thermal bonding. In certain aspects, the bonding can be facilitated by the presence of an additional portion of the elastomeric polymer. For example, in certain aspects, the sheath, as described herein and as shown in **FIGS. 63H-I** can further comprise a first strip **611** of the elastomeric polymer disposed along at least a portion of the longitudinal axis of the lumen between at least a portion of the outer surface of the sheet that does not comprise the overlaying portion **602c** of the sheet and the inner surface of the elongated tube. In such aspects, the bonding between the elongated tube and the inner liner can be facilitated by the first strip of the elastomeric polymer. In yet other aspects, the sheath can optionally, if desired, further comprise a second strip **611** (**FIGS. 63E-F**) of the elastomeric polymer disposed between at least a portion of the outer surface of the sheet at the proximal end of the sheath and the inner surface of the elongated tube. In still further aspects, the sheath can further comprise a third strip **611** of the elastomeric polymer disposed between at least a portion of the outer surface of the sheet at the distal end of the sheath and the inner surface of the elongated tube (**FIGS. 63E** and **63G**). Again, in such aspects, the bonding between the elongated tube and the inner liner can be facilitated by the second and/or third strips of the elastomeric polymer.

Applications can utilize a sheath of the present disclosure with the rest diameter *dᵣ* of the lumen formed by the inner liner **602** that is expandable to an expanded diameter *dₑ* of from about 3 Fr to about 26 Fr, including exemplary values of about 5 Fr, about 8 Fr, about 10 Fr, about 12 Fr, about 15 Fr, about 18 Fr, about 20 Fr, about 22 Fr, about 25 Fr. The expanded diameter can vary slightly along the length of the disclosed sheath. For example, the expanded outer diameter at the proximal end of the sheath can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, about 25 Fr, while the expanded outer diameter at the distal end of the sheath can range from about 3 Fr to about 25 Fr, including exemplary values of about 8 Fr, about 10 Fr, about 12 Fr, about 15 Fr, about 18 Fr, about 20 Fr, and about 22 Fr. Aspects of the disclosed sheath can expand to an expanded outer diameter that is from about 10% greater than the original unexpanded outer diameter to about 100% greater than the original unexpanded outer diameter, including exemplary values of about 15 % greater, about 20 % greater, about 25 % greater, about 30 % greater, about 35 % greater, about 40 % greater, about 45 % greater, about 50 % greater, about 55 % greater, about 60 % greater, about 65 % greater, about 70 % greater, about 75 % greater, about 80 % greater, about 85 % greater, about 90 % greater, and about 95 % greater than the original unexpanded outer diameter.

It is understood, and as described above, the disclosed sheath can expand from its rest position. The expansion of the disclosed sheath can result in an expansion of the rest diameter *dᵣ* of from about 10% or less to about 430% or more. In certain aspects, expansion of the sheath can result in expansion of the rest diameter *dᵣ* to about 10 % or less, to about 9 % or less, to about 8 % or less, to about 7 % or less, to about 6 % or less, to about 5 % or less, to about 4 % or less, to about 3 % or less, to about 2 % or less, to about 1 % or less. In yet other aspects, expansion of the disclosed sheath can result in expansion of the rest diameter *dᵣ* to about 10 % or more, about 20 % or more, about 30 % or more, about 40 % or more, about 50 % or more, about 60 % or more, about 70 % or more, about 80 % or more, about 90 % or more, about 100 % or more, about 125 % or more, about 150 % or more, about 175 % or more, about 200 % or more, about 225 % or more, or about 250 % or more.

As with previously disclosed aspects, the aspects illustrated in **FIGS. 63A-63D** can be applied to sheaths having a wide variety of rest diameters *dᵣ* and outer diameter *dₒ.* In some aspects, the outer diameter *dₒ* of the sheath gradually decreases from the proximal end of the sheath to the distal end of the sheath. For example, in one aspect, the outer diameter *dₒ* can gradually decrease from about 26 Fr at the proximal end to about 18 Fr at the distal end. The diameter *dₒ* of the sheath can transition gradually across substantially the entire length of the sheath. In other aspects, the transition or reduction of the diameter of the sheath can occur only along a portion of the length of the sheath. For example, the transition can occur along a length from the proximal end to the distal end, where the length can range from about 0.5 inches to about the entire length of the sheath, including any values between any two foregoing values. In yet further aspects, the *dₒ* is minimal and constant along the section of the sheath that passes through the vasculature. In such aspects, the tapered section is about 4" or less at the proximal side of the sheath.

In some aspects, the braid and/or the elongated tube can comprise the same material or combination of materials along the entire length. In alternative aspects, the material composition of each layer can change along the length of the sheath. For example, the outer layer comprising both the braid and the elongated tube can be provided with one or more segments, where the composition changes from segment to segment. For example, in one segment, the braid can comprise nitinol having a different PIC count than another segment. In yet another exemplary aspect, the elongated tube in one segment can be different from the layer of the elastomeric material in another segment. In still further exemplary aspects, one segment of the sheath can comprise the braid embedded within the elongated tube, while another segment can comprise the braid that is not embedded within the elongated tube. It is understood that the exemplary sheath disclosed herein is not limiting. In certain exemplary aspects, the sheath can comprise an *n* number of segments, wherein each segment can be the same or different. In still further exemplary aspects, the Durometer rating of the composition of the outer layer can also change along the length of the sheath such that segments near the proximal end comprise a stiffer material or combination of materials, while segments near the distal end comprise a softer material or combination of materials. This can allow for a sheath having a relatively stiff proximal end at the point of introducing a delivery apparatus while still having a relatively soft distal tip at the point of entry into the patient's vessel.

Additionally, some aspects of the sheath, as disclosed herein, can include an exterior hydrophilic coating on the outer surface of the elongated tube. Such a hydrophilic coating can facilitate insertion of the sheath into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g.*, PTFE, polyethylene, polyvinylidene fluoride), are also suitable for use with the sheath. This exemplary sheath, similarly to other sheaths disclosed herein in some aspects, can comprise a soft tip portion comprising, in some aspects, low-density polyethylene (LDPE) and can be configured to minimize trauma or damage to the patient's vessels as the sheath is navigated through the vasculature. Any materials described herein for a soft tip can be used to form the soft tip of this exemplary sheath.

In certain aspects, the exemplary sheath, as described herein, can also be generally represented, as shown in **FIG. 35**. In such exemplary aspects, the described herein sheath can have additional components, as shown in **FIGs. 35** and **36****.** It is, however, understood that the sheath disclosed herein does not have to comprise the components shown in FIGs. 35 and 36 and can be adapted to any other applications. In such exemplary aspects, it can be adapted to passing any medical devices that require an introducer sheath.

Some exemplary aspects of these additional sheaths are also shown in **FIGS. 64A-64B** and **65A-65B.** **FIGS. 64A-64B****,** for example, show a cross-section view of the exemplary sheath taken near the distal end. **FIG. 64A** shows the sheath **1200A** comprising the inner liner **1202** having the first edge **1202a** and the second edge **1202b,** and the overlaying portion **1202c,** where the inner and outer surfaces of the inner liner overlay each other. The sheath **1200A** further comprises an amount of the second lubricant **1208** as disclosed herein that is disposed between the sliding and overlaying portions of the inner sheath. The sheath further comprises the braid **1204** and the elongated tube **1206.** In this exemplary aspect, the braid **1204** is not embedded into the layer of the elongated tube **1206.** **FIG. 64B** depicts an alternative aspect of the sheath **1200B** where an amount of the first lubricant **1210** is applied between the inner liner and the outer layer comprising the braid **1204** and the elongated tube **1206.** An additional aspect of the sheath **1200C** is shown in **FIG. 64C****.** In this aspect, the sheath **1200C** comprises the inner liner **1202,** having the first edge **1202a** and the second edge **1202b,** and the overlaying portion **1202c,** where the inner and outer surfaces of the inner liner overlay each other. The sheath further comprises the braid **1204** and the elongated tube **1206** that together form the outer layer of the sheath. The sheath **1200C** further comprises an amount of the first lubricant **1210,** as disclosed herein, that is disposed between the outer layer and the inner liner of the inner sheath. In this exemplary aspect, the braid **1204** is not embedded into the elongated tube **1206.** In the exemplary aspect shown in **FIG. 64D****,** the exemplary sheath **1200D,** comprises the braid **1204** embedded within the elongated tube **1206.**

**FIGs. 65A-D** show a section view of a proximal section of the sheath. **FIG. 65A** shows the sheath **1300A** comprising the inner liner **1302** having the first edge **1302a** and the second edge **1302b,** and the overlaying portion **1302c,** where the inner and outer surfaces of the inner liner overlay each other. The sheath **1300A** further comprises an amount of the second lubricant **1308** as disclosed herein that is disposed between the sliding and overlaying portions of the inner sheath. The sheath further comprises the braid **1304** and the elongated tube **1306.** In this exemplary aspect, the braid **1304** is not embedded into elongated tube **1306.** **FIG. 65B** depicts an alternative aspect of the sheath **1300B,** where an amount of the first lubricant **1310** is applied between the inner liner and the outer layer comprising the braid **1304** and the elongated tube **1306.** An additional aspect of the sheath **1300C** is shown in **FIG. 65C****.** In this aspect, the sheath **1300C** comprises the inner liner **1302,** having the first edge **1302a** and the second edge **1302b,** and the overlaying portion **1302c,** where the inner and outer surfaces of the inner liner overlay each other. The sheath further comprises the braid **1304** and the elongated tube **1306** that together form the outer layer of the sheath. The sheath **1300C** further comprises an amount of the first lubricant **1310,** as disclosed herein, that is disposed between the outer layer and the inner liner of the inner sheath. In this exemplary aspect, the braid **1304** is not embedded into the elongated tube **1306.** In the exemplary aspect shown in **FIG. 65D****,** the exemplary sheath **1300D,** comprises the braid **1304** embedded within elongated tube **1306.**

In yet further aspects, as shown in **FIG. 66****,** the sheath **1400,** whether with the braid embedded within the elongated tube (as shown in **FIG. 66**) or with the braid that is not embedded within the layer of the elastomeric polymer (not shown), is configured to expand from a resting configuration to an expanded configuration shown in **FIG. 67****.** In such aspects, the first and the second edges (**1502a** and **1502b**) of the inner liner slide such that a length of the overlaying portion shortens. In some exemplary aspects, this movement can be facilitated by the presence of the first and/or second lubricant, as disclosed above.

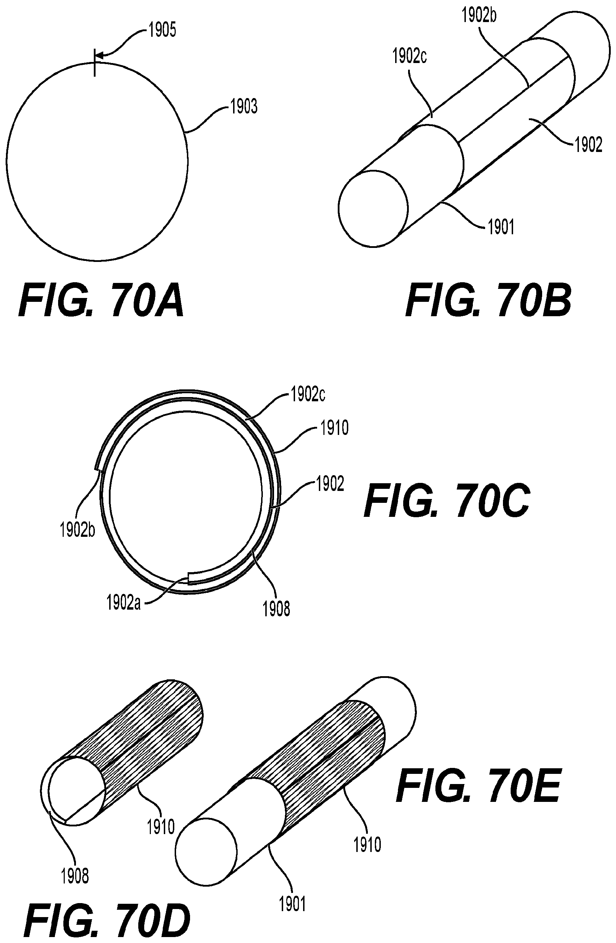

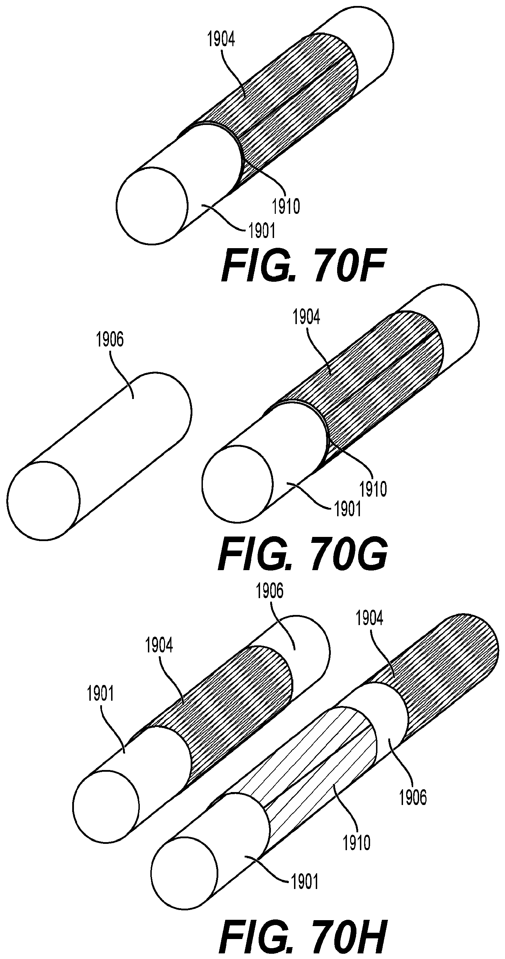

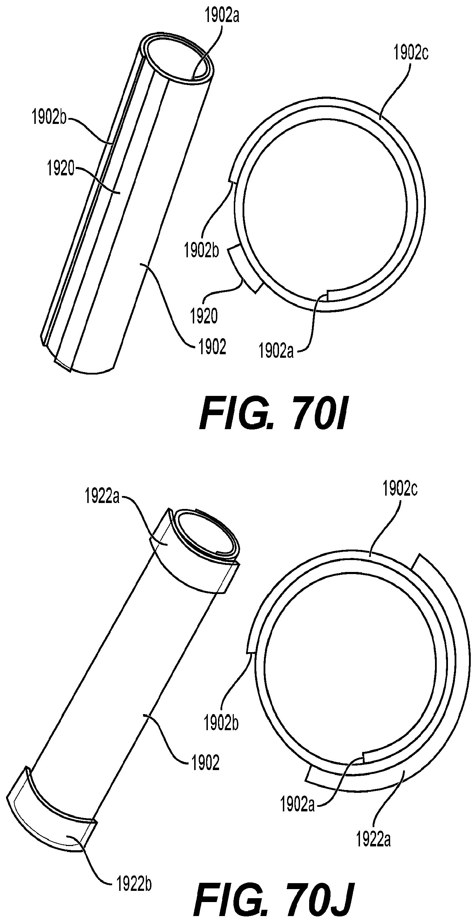

Now referring to **FIGS. 71-72****.** As disclosed herein, the elongated tube can be used as an outer jacket. The outer jacket **140** disclosed herein can comprise at least two polymer layers **146** and **147.** In still further aspects, the outer jacket disclosed herein can comprise at least one intermediate reinforcement layer/member **145** is disposed in the first polymer layer, in the second polymer layer, or between the first and second layers. Such an outer jacket can be disposed on an additional optional polymer strip **1920** as shown and further described as referenced to **FIG.70****.**

**FIGS. 73-77** illustrate an expandable outer jacket including longitudinally extending reinforcing members **145.** The outer jacket **140** can be used with any of the expandable sheaths described herein. The reinforcing members **145** prevent axial bunching of the outer jacket **140** during insertion into the patient's vasculature while not sacrificing the low radial expansion force of the outer jacket **140.** The reinforcing members **145** are typically constructed from a stiffer material (e.g., Pebax, polyurethane, nylon, flat wire) than the main body portion of the outer jacket **140.** The resistance of the reinforcing members **145** to elongation and/or compression prevents bunching/crumpling of the outer jacket **140** during insertion while still allowing the outer jacket **140** to radially expand.

The wall thickness of the outer jacket can range from 0.0040 inches to 0.0066 inches. In some examples, the thickness of the outer jacket is about 0.0055 inches. The wall thickness of the outer jacket **140** can remain constant along the entire length of the outer jacket **140.** However, in some examples, the thickness of the outer jacket **140** at the proximal end (T1) is greater than the thickness of the outer jacket **140** at the distal end (T2).

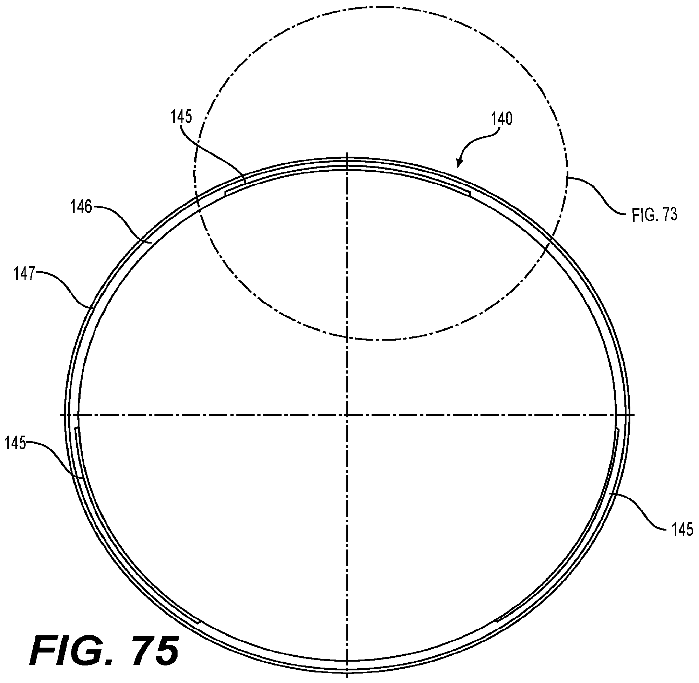

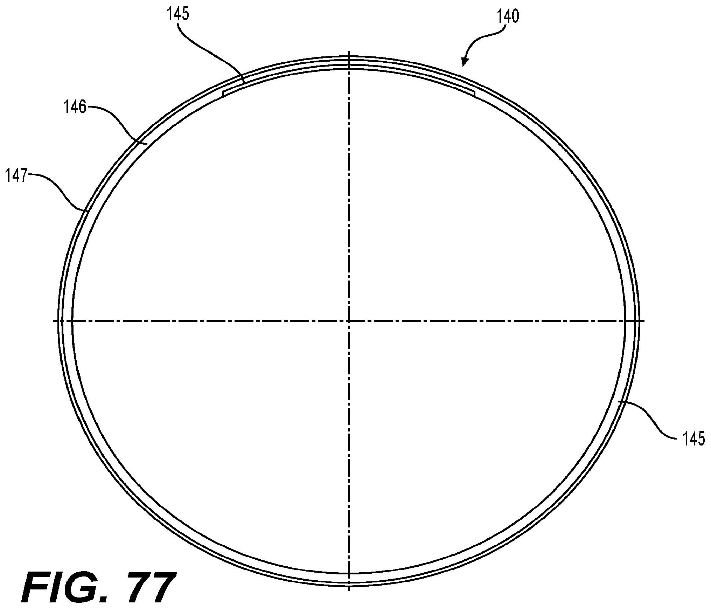

In still further aspects, the outer jacket **140** can comprise two or more reinforcing members **145.** In such aspects, the two or more reinforcing members **145** can be disposed, as individual strips, disposed circumferentially in the first polymer layer, in the second polymer layer, or between the first and second layers at a predetermined distance from each other. **FIG. 75** is a cross section view of the outer jacket **140** taken along section lines B-B in **FIG. 73****.** As provided in **FIG. 75****,** the outer jacket **140** includes three reinforcing members **145.** In some examples, the outer jacket **140** includes only one reinforcing member **145** (**FIG. 77**). In other examples, the outer jacket includes up to eight reinforcing members **145.** When more than one reinforcing member **145** is used, the reinforcing members are spaced evenly around the circumference of the outer jacket **140.** As further illustrated in **FIG. 75****,** the reinforcing member **145** can have a rectilinear shape (e.g., rectangular) in the cross section. However, any other regular or irregular shape is contemplated.

In further aspects, the reinforcing member **145** has a finite width that is smaller than the circumference of the outer jacket **140.** The total combined width (w) of the reinforcing members **145** can range from 5% to 50% of the circumference of the outer jacket **140.** In still further aspects, the total combined width of the strips is about 5%, about 15%, about 20%, about 25%, about 30%, about 35%, about 40%, about 45%, or about 50% of the circumference of the elongated tube.

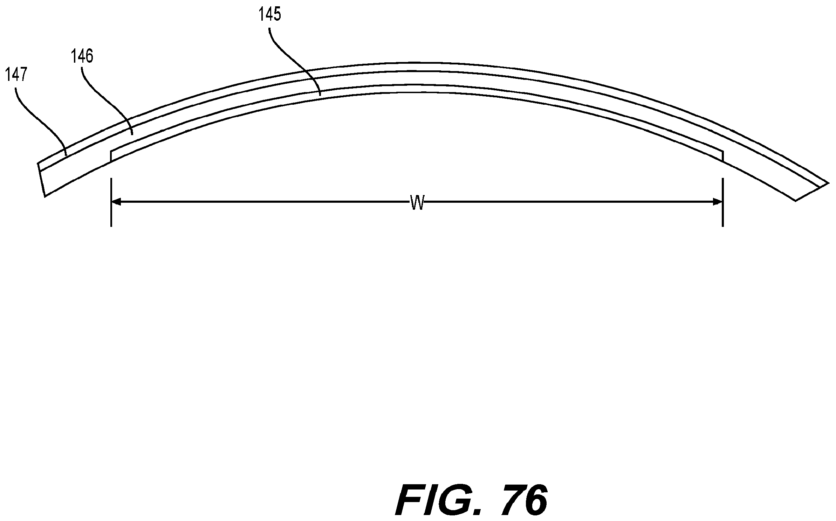

**FIG. 76** includes a partial view of the outer jacket **140** of **FIG. 75****.** As provided in **FIG. 76****,** the circumferential width of the reinforcing members **145** can range from 0.010 inches to 0.150 inches. In some examples, the distal end of the outer jacket **140** has a diameter of 0.200 inches, and the circumferential width of the reinforcing members **145** can range from 0.010 inches to 0.150 inches. In some exemplary and unlimiting aspects, the diameter of the outer jacket at the distal end is about 0.200", the reinforcing member can have a width between about 0.010" to about 0.150", including exemplary values of about 0.03", about 0.035", about 0.04", about 0.045", about 0.05", about 0.055", about 0.06", about 0.065", about 0.07", about 0.075", about 0.08", about 0.085", about 0.09", about 0.095", about 0.10", about 0.105", about 0.110", about 0.115", about 0.120", about 0.125", about 0.130", about 0.135", about 0.140", and about 0.145". It is understood that the widths shown above are exemplary, and if the distal outer diameter of the elongated sheath has a size different from 0.200", the strip width can be adjusted in the same or a different ratio. In yet still, further aspects, as described above, the width of the reinforcing member can be measured as a percentage of the elongated tubing circumference.

It is further understood that in the aspects where the reinforcing member **145** is present as one or more strips disposed circumferentially along the length of the outer jacket **140,** the width of the reinforcing member **145** can be the same along the length, or it can vary along the length. In aspects where the reinforcing member **145** width varies along the length of the outer jacket **140,** such a reinforcing member **145** can have any of the disclosed above width values.

In still further aspects, at least one reinforcing member **145** is configured to provide an axial reinforcement to the outer jacket **140** and, as a result, to the sheath where the outer jacket **140** can be used. In such exemplary aspects, the at least one reinforcing member **145** can be disposed along the length of the outer jacket **140** or along a portion of the length of the outer jacket **140.** In some aspects, the portion of the length of the outer jacket **140** where the at least reinforcing member **145** is disposed at the distal end and/or proximal end of the outer jacket **140.** In yet other aspects, the reinforcing member **145** can also be positioned anywhere along the length of the outer jacket **140.**

As described above, and as illustrated in **FIG. 75****,** the outer jacket **140** includes a two-layer construction, inner layer (first polymer layer) **146** and outer layer (second polymer layer) **147,** where the outer layer provides abrasion resistance (for example, between the sheath and a calcific lesion) and better resistance to the hydrophilic coating process, and the inner layer is a more lubricious material (for example, to prevent sticking of the outer jacket against the outer layer of the sheath during expansion) the and provides higher pressure resistance or ballooning resistance and hemostasis. In some aspects, the inner layer **146** (first polymer layer) forms the inner surface of the outer jacket **140** and the outer layer **147** (second polymer layer) forms the outer surface of the outer jacket, the reinforcing members **145** are disposed between the outer surface of the inner layer (liner) **146** and the inner surface of the outer layer **147.**

In some examples, the inner layer **146** can be composed of Pebax or polyurethane, having Shore 25D to 35D. In some examples, the inner layer **146** includes a PTFE powder, an optional inorganic filler, and an optional tackiness reducing additive to lower friction when outer layer **108** of the sheath expanding by sliding against the outer jacket **140.** In some examples, the outer layer **147** of the outer jacket **140** is composed of polyurethane or polyurethane/Styrene Block Copolymer (SBC) having Shore A durometer lower than about 60, e.g., Neusoft 597-50A having Shore A hardness of about 55A. In certain examples, the inner layer 146 is constructed from Polyether Block Amide, such as Pebax having Shore D durometer less than about 35.

As provided in **FIG. 75** and **76****,** the reinforcing members **145** are at least partially embedded in the inner layer 1**46**. In some examples, the thickness of the reinforcing member **145** is less than the thickness of the inner layer **146.** For example, as illustrated in **FIG. 76****,** the reinforcing members **145** have a thickness ranging from 0.0005 inches to 0.0015 inches. In some examples, the reinforcing members **145** have a thickness of about 0.001 inches. In an example configuration, the reinforcing members **145** have a thickness of 0.001 inches, and the inner layer has a thickness of 0.00154 inches. In another example, not shown, the reinforcing member **145** has a thickness corresponding to the thickness of the inner layer **146.** In a further example, the reinforcing member **145** has a thickness greater than the thickness of the inner layer **146.** In some examples, the inner layer **146** and the reinforcing member **145** are coextruded. Similarly, the inner layer **146,** reinforcing member **145** and the outer layer **147** are co-extruded with the reinforcing member **145** positioned between the inner and outer layers **146, 147.** In other examples, the inner layer 146 is provided over the reinforcing member **145,** and the two components are bonded or fused together by at least one of heat or compression.

As described above, the reinforcing members **145** are constructed from a stiffer material than the main body portion of the outer jacket **140** (inner layer **146,** outer layer **147)** and also a material having a low coefficient of friction (e.g., high density polyethylene). In some examples, the reinforcing members **145** are constructed from a polymer compatible with the inner layer and outer layer, including, for example, high durometer Pebax or polyurethane. The reinforcing member **145** can also be constructed from a material having a Shore D durometer ranging from 45D to 76D.

Additional examples of the sheath that can be used with the disclosed herein elongated tube can be found in US application No. 63/021,945.

The disclosed herein sheath can be configured such that it locally expands at a particular location corresponding to the location of the medical device along the length of the lumen and then locally contracts once the medical device has passed that particular location. Thus, a bulge may be visible, traveling longitudinally along the length of the sheath as a medical device is introduced through the sheath, representing continuous local expansion and contraction as the device travels the length of the sheath. In some aspects, each segment of the sheath can locally contract after removal of any radial outward (insertion) force such that it regains the original resting diameter of lumen *dᵣ.*

In some aspects, each segment of the sheath can locally contract after removal of any radial outward force such that it at least partially returns to the original resting diameter of lumen *dᵣ.*

### METHODS

An example method of making the sheath is as follows. These steps are not meant to be limiting. The steps given can be reordered as needed. Other steps can be added, or in other examples, some steps may not be necessary.

Disclosed herein are the methods of making a sheath having a proximal end and a distal end and comprising: a) extruding a tubular body to form an elongated tube comprising a first polymer layer, wherein the first polymer layer comprises a first compound composition comprising from greater than 0 wt% to less than 100 wt% of a first polymer comprising a polyether block amide, a polyurethane, or a combination thereof; less than about 65 wt% of an inorganic filler based on a total weight of the first compound composition; and up to about 20 wt% of a solid lubricant filler based on a total weight of the first compound composition; b) disposing the elongated tube on the sheath such that the elongated tube forms an outer layer of the sheath, and wherein the elongated tube is positioned at at least the proximal end of the sheath and extending along at least a portion of a length of the sheath, wherein the elongated tube is configured to reversibly expand from an initial diameter d₀ in an unexpended position to an expanded diameter dₑ in an expanded position upon passage of a medical device; and wherein the formed sheath exhibits at least a 10% reduction in an insertion force when compared with a substantially identical reference sheath that does not comprise the first polymer layer.

It is understood that any known in the art methods can be utilized to form the composition of the elongated tube. In certain aspects, the components that are present in the elongated tube are provided to form a compound. The compound is then mixed to form a substantially homogeneous mixture. Yet, in other aspects, the mixture is homogeneous. In still further aspects, the mixture is extruded to form an elongated tube having a first polymer layer. The formed first polymer layer can comprise any (and in any combination) of the compositions and characteristics disclosed above.

In yet further aspects, the methods also comprise steps of forming the elongated tube comprising two or more layers, as disclosed above. In such aspects, for example, when the elongated tube comprises any of the disclosed above first polymer and the second polymer layers, such layers can be coextruded to form the elongated tube as disclosed. Any of the known in the art extrusion devices can be used to obtain the desired elongated tube.

The methods disclosed herein produce any of the disclosed above elongated tubes.

In still further aspects, also disclosed are the methods of forming additional parts of the sheath. In certain aspects, the elongated tube can behave as an outer jacket of the sheath. While yet in other aspects, the elongated tube can form an outer layer of the sheath without an additional outer jacket being present.

For example, and without limitation, the inner layer **108,** as shown in one of the exemplary sheaths, can be formed to include a first fold and a second fold and an overlapping folded portion **118** extending circumferentially between the first and second folds. The overlapping folded portion **118** can be formed to include overlap in a radial direction of at least two thicknesses of the inner layer **108.** The inner layer **108** can be extruded, including the folded portion **118.** Alternatively, the folded portion **118** can be formed after the inner layer **108** is extruded (e.g., formed on a cylindrical tubular structure).

A discontinuous outer layer **110** can be further provided (at least partially) around the inner layer **108.** The outer layer **110** is formed to include an overlapping portion **120** and an underlaying portion **122** such that at least a portion of the folded portion **118** of the inner layer **108** is positioned between the overlapping portion **122** and the underlaying portion **120.** In some aspects, the inner layer **108** and the outer layer **110** are coextruded. In alternative aspects, the inner layer **108** and the outer layer **110** are separately formed and joined together.

An adhesive layer **128** can be provided between the inner layer **108** and the outer layer **110** for bonding (at least partially) the inner layer **108** to the outer layer **110.** The adhesive layer **128** can be applied to and bond the inner and outer layers **108, 110** axially along a length of the sheath, e.g., along a portion of the entire length of the sheath or along the entire length of the sheath. In an example, the adhesive layer **128** is coextruded with the outer layer **110.** In a further example, the adhesive layer **130** is coextruded with the inner layer **108.** In an alternate example method, the adhesive layer **142** can be applied to the outer surface of the inner liner **108** and/or an inner surface of the outer layer **110.**

After the adhesive layer **128** is applied to the desired locations along the inner and/or outer layer **110,** the outer layer **110** is applied over the inner layer **108.** The outer layer **110** can then be bonded to the inner layer **108** by heat curing the adhesive layer **128.** The adhesive layer **128** can comprise a material curable at a temperature above room temperature, in which case a heat treatment may be applied to the assembled inner layer **108**/outer layer **110.** The adhesive layer **128** may also be composed of a material that cures at room temperature. Accordingly, after application of the adhesive layer **128** and assembly of the outer layer **110** over the inner layer **108** (at a temperature below room temperature), the temperature of the combined layers may be increased to room temperature.

As illustrated in **FIG. 56****,** the lubricant **142** can be selectively applied to the desired location along a length of outer layer **110** and/or outer jacket (the elongated tube, as described) **140.** It is understood that the elongated tube **140** can be formed by any of the methods disclosed above.

As described above, the lubricant **142** can be provided on an outer surface of the outer layer **110** proximate a longitudinally extending edge **126** of the overlapping portion **120,** on any portion of the folded portion **118** extending/protruding beyond edge **126,** and/or on any portion of the outer surface of the outer layer **110** adjacent the protruding portion of the folded portion **118.** The lubricant **142** can be applied as a band extending around a portion of the circumference of the outer layer, the band of lubricant **142** also extending longitudinally along a length of the outer layer **110.** After the lubricant **142** is selectively applied to the outer surface of the outer layer **110,** the outer jacket **140** can be applied over the outer layer **110.** As outlined above, the lubricant **142** can comprise a heat-curable material, in which case a heat treatment can be applied to the outer layer **110**/elongated tube **140.** The lubricant **142** may also be composed of a material that cures at room temperature. Accordingly, after application of the lubricant **142** (at a temperature below room temperature), the temperature of the outer layer **110** (separately or in combination with the outer jacket (elongated tube) **140**) can be increased to room temperature.

The outer jacket **140,** formed by any of the methods disclosed above, can then be applied over/around the outer layer **110** and bonded to the outer layer **110** at at least one of the proximal and distal ends of the outer layer **110.** The outer jacket **140** can also be bonded to the outer layer **110** along a length of the outer layer **110.** The outer jacket **140** can be bonded to the outer layer **110** via a heat treatment process, e.g., a reflow process where the outer layer **110** and the outer jacket are headed to a temperature high enough such that the outer layer **110** and the outer jacket **140** are at least partially melted and are then fused together are the heat is removed, and the assembly cools. The entire sheath **100** assembly may be reflowed to reduce the overall outer diameter and regain/ensure a circular shape in cross-section.

As described above, select portions of the outer surface of the inner liner **108** can include a surface treatment such as surface etching. In an example method, surface treatment of the inner layer **108** would occur before application of the outer layer **110.** It is contemplated that it may be desirable to exclude etching from those surfaces of the inner layer **108** that come into contact with the outer surface of the outer layer **110.** For example, etching may not be included between the inner surface of the folded portion **118** of the inner layer **108** and the underlaying portion **122** of the outer layer **110.** By excluding etching on the portions where the inner layer **108** and the outer surface of the outer layer **110** are in direct contact helps to facilitate release of the inner surface of the folded portion **118** and the outer layer **110** during expansion of the sheath **100.**

In some instances, it may be necessary to release ("break") any undesirable bonding that occurs between the outer layer **110** and the inner layer **108.** This bonding can occur due to the etching on the outer surface of the inner liner **108** that allows it to stick directly to the outer layer **110** (with/without the adhesive layer **128**). Undesirable bonding can also occur if the outer layer **110** can flows on top of the folded inner layer **108** and "grabs" it during the reflow process.

However, when the inner layer **108** is unetched along those locations excluding the adhesive layer **128,** e.g., those portions adjacent the underlaying portion of the outer layer **110,** undesirable bonding between the outer layer **110** and the inner layer **108** at this location is limited. Therefore, it may not be necessary to precondition the sheath to release undesirable bonding between the inner layer **108** and the outer layer **110** because the bonding has not occurred (or is less likely to occur).

Nonetheless, undesirable bonding between the inner layer **108** and the underlaying portion **122** of the outer layer **110** can be released while maintaining desirable bonding at the proximal and distal ends of the sheath **100.** For example, a mandrel can be passed at least partially through the lumen **116** of the inner layer **108,** expanding the inner layer **108** and the outer layer **110,** and breaking/releasing any undesirable bonding between the inner layer **108** and the underlaying portion **122** of the outer layer **110.**

Various methods can be used to produce the sheaths discussed above and below throughout the present disclosure. For example, a method of making the sheath shown in **FIGS. 2A-2D** can comprise providing a mandrel and applying an inner layer on the mandrel, such as by spray coating or dip coating the mandrel. An intermediate layer, such as a mesh structure, can then be mounted on the inner layer. An outer layer can be applied over the intermediate layer, such as by a second spray coating or dip coating step. Methods can comprise etching or surface treating at least a portion of the inner layer. Also, methods can comprise providing one or more notches and/or cuts in the inner layer and/or the outer layer. Cuts and/or notches can be provided by, for example, laser cutting or etching one or more layers.

In some aspects of methods of making a sheath, such as the sheaths illustrated in **FIGS. 2A-2D****,** layers can be pre-formed and mounted on a mandrel and then fused or thermally bonded together. For example, in one method, an inner layer is applied to a mandrel. An intermediate layer can be applied to the outer surface of the inner liner. An outer layer can be applied to the outer surface of the intermediate layer. Heat shrink tubing can be applied and the assembly heated, such that the inner layer, the intermediate layer, and/or the outer layer are thermally bonded and compressed together under the heat shrink tubing.

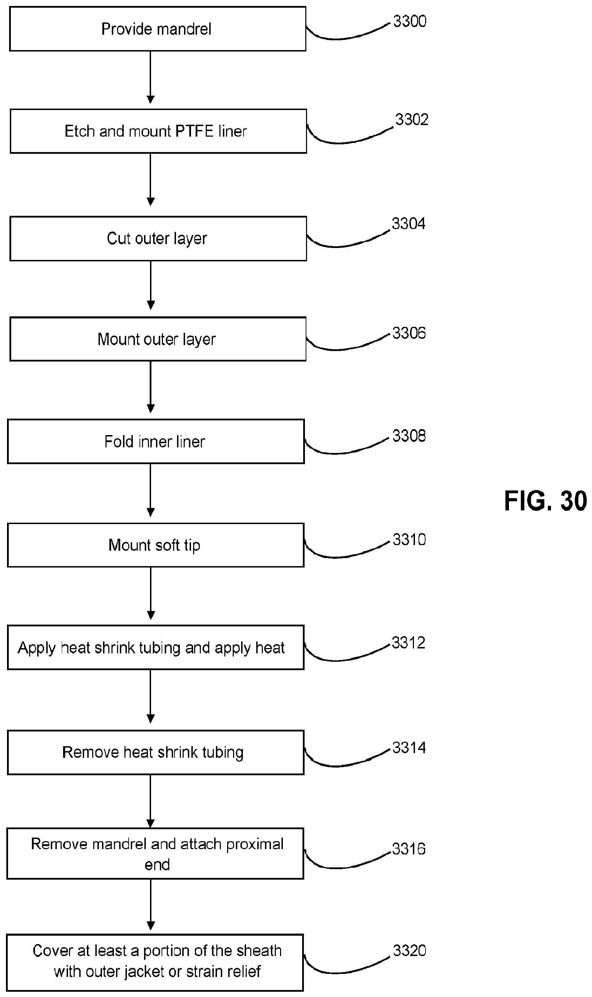

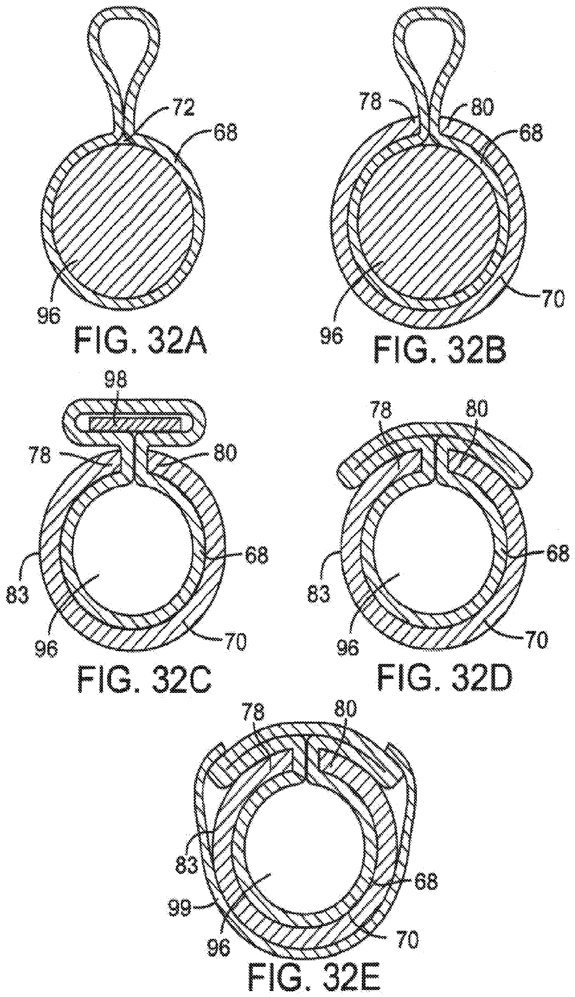

**FIG. 30** illustrates a block diagram of one method of producing a sheath for use with a delivery apparatus in minimally invasive surgery. One or more mandrels can be provided (step **3300**). The mandrel can be provided with an exterior coating, such as a Teflon^{®} coating, and the mandrel's diameter can be predetermined based on the desired size of the resulting sheath. A liner that will become the inner polymeric layer of the sheath, such as a PTFE or high-density polyethylene liner, can be mounted on the mandrel (step **3302**). The liner can be etched and/or surface treated prior to being mounted on the mandrel, according to conventional etching and surface treatment methods. **FIG. 32A** illustrates a section view of a sheath at steps **3300** and **3302** of **FIG. 30****.** A coated mandrel **96** is inserted within the lumen **72** of the inner polymeric liner **68.** The circumference of the inner polymeric liner **68** is larger than the circumference of the mandrel **96,** such that an excess portion of the inner polymeric liner **68** can be gathered above the mandrel **96.**

A layer of material that will become the outer polymeric tubular layer, such as a layer comprising polyurethane or polyolefin, can be cut or notched through all, substantially all, or a part of the thickness of the layer (step **3304**). Such a cut or notch can extend longitudinally along the length of the layer and can extend along substantially the entire length of the outer polymeric tubular layer. In alternative aspects, the cut or notch can be provided along only a portion of the outer polymeric tubular layer. For example, the outer polymeric tubular layer can be cut starting at the distal end of the outer polymeric tubular layer, with the cut ending before the proximal end of the outer polymeric tubular layer. In one aspect, the cut can end at a transition, where the outer diameter of the outer polymeric tubular layer increases or decreases. In one specific aspect, the cut or notch can extend longitudinally along about 75% of the length of the sheath.

The cut or notched outer polymeric tubular layer can be applied, positioned, adhered, mounted, thermally fused or bonded, dip coated, and/or otherwise coupled to the etched inner liner (step **3306**). **FIG. 32B** shows a section view of the sheath at step **3306** of **FIG. 30****,** with outer polymeric tubular layer **70** applied to the inner polymeric liner **68** such that a portion of the inner polymeric liner **68** extends between the cut formed between first and second portions **78, 80** of the outer polymeric tubular layer **70.**

In alternative aspects, the outer polymeric tubular layer can be notched or cut after being mounted on the inner liner/mandrel assembly. The outer polymeric tubular layer can optionally be provided with a hydrophilic coating and/or provided with additional layers, such as being dip coated with polyurethane. Some portion of the inner liner can protrude through the cut in the outer polymeric tubular layer after such an outer polymeric tubular layer is mounted onto the inner liner/mandrel arrangement. Using, for example, a split tool, the protruding portion of the inner liner can be folded down onto the outer surface of the outer polymeric tubular layer (step **3308**)**.** In some aspects, the protruding portion of the inner liner is folded down along the entire length of the resulting sheath, while in other aspects, the protruding portion of the inner liner is only present along a portion of the length of the sheath or is only folded down along a portion of the length of the resulting sheath. **FIG. 32C** shows a section view of the sheath at step **3308** of **FIG. 30****.** A split tool **98** is used to fold the excess portion of inner polymeric liner **68** over a portion of the outer surface **83** of the outer polymeric tubular layer **70.** **FIG. 32D** shows a section view of the sheath after completion of step **3308** of **FIG. 30****.** Split tool **98** has been removed, and folding of the excess portion of the inner polymeric liner **68** has been completed. **FIG. 32E** shows a section view of an outer covering, such as outer polymeric covering **99,** that can be applied such that it overlaps a portion of the folded portion of inner polymeric liner **68.** The outer polymeric covering **99** contacts at least a portion of the outer surface **83** of the outer polymeric tubular layer **70.**

A soft, atraumatic tip can be provided at the distal end of the resulting sheath **(step 3310).** Additional outer layers can also be applied if desired. Then, a layer of heat shrink tubing, such as fluorinated ethylene propylene (FEP) heat shrink tubing, can be positioned over the entire assembly (step **3312**). An appropriate amount of heat is applied, thus shrinking the heat shrink tubing and compressing the layers of the sheath together, such that components of the sheath can be thermally bonded or fused together where desired. Once the components of the sheath have been bonded together, the heat shrink tubing can be removed (step **3314**). The proximal end of the sheath can be adhered or otherwise attached to a housing of a catheter assembly, and the sheath can be removed from the mandrel (step **3316**). The disclosed herein elongated tube is then positioned (step **3320**) on the sheath to form an outer jacket or strain relief jacket. It is understood that the disclosed tube can be positioned at at least a portion of the sheath, for example, and without limitation, the proximal end of the sheath, or it can be positioned along the entire length of the sheath.

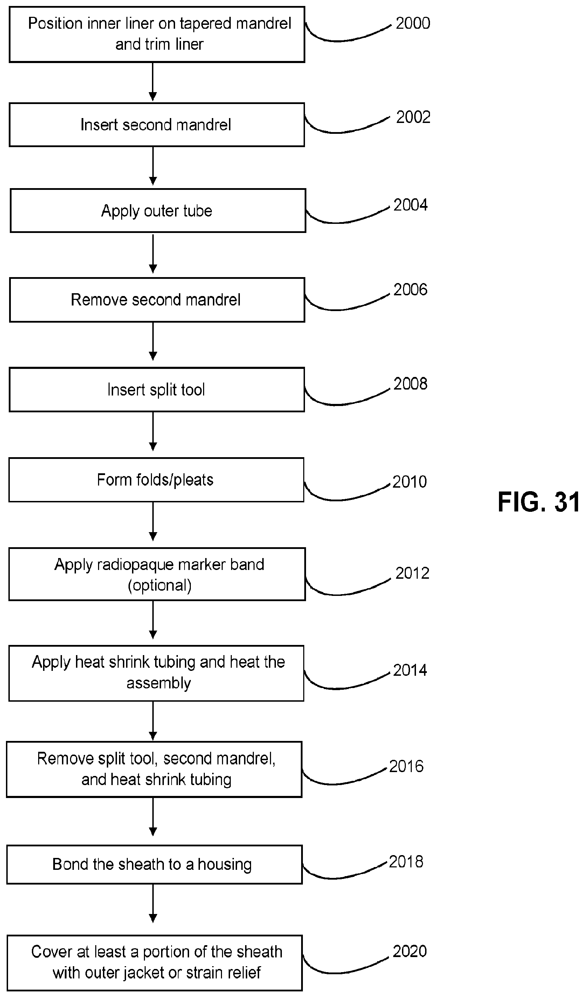

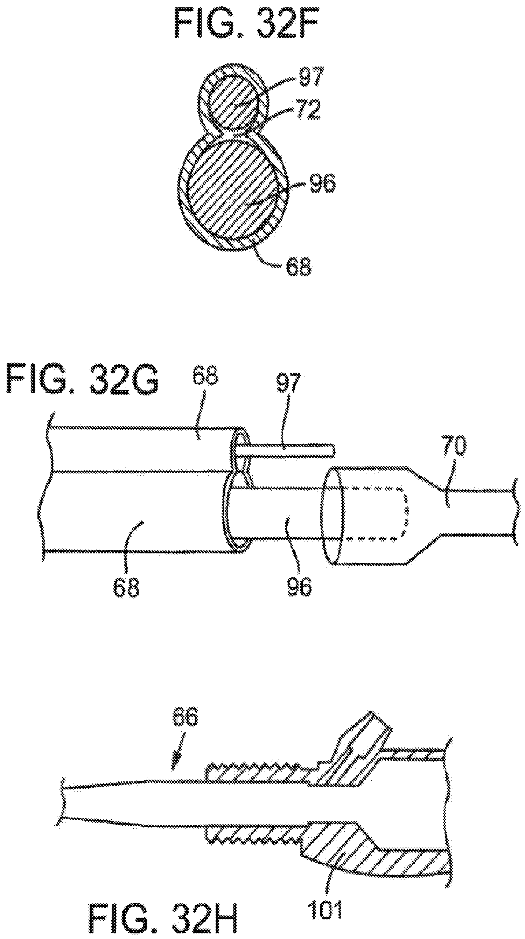

**FIG. 31** illustrates a block diagram of an alternative aspect of a method of making a sheath. An inner liner, such as an etched PTFE tubing, can be applied to a tapered mandrel, such as a 16 Fr tapered mandrel, and trimmed to an appropriate length (step **2000).** A second mandrel, such as a 0.070 inches diameter mandrel, can be inserted in the lumen of the inner liner such that the mandrels are arranged side by side in the inner liner (step **2002**). **FIG. 32F** shows a section view of a sheath at steps **2000** and **2002** of **FIG. 31****.** An inner liner or inner polymeric liner **68** is applied on a first, tapered mandrel **96.** A second mandrel **97** is inserted into the lumen **72** of the inner polymeric liner **68** created by the excess portion of the inner polymeric liner **68,** as described.

A notched or cut outer polymeric tubular layer, such as high-density polyethylene tubing that has been notched or cut longitudinally, can be slid onto the tapered mandrel and a portion of the inner liner, starting at the distal end of the tapered mandrel (step **2004).** The second mandrel can then be removed (step **2006**). **FIG. 32G** illustrates a perspective view of the sheath at steps **2004** and **2006** of **FIG. 31****.** An outer polymeric tubular layer **70** having a longitudinal cut is applied over the tapered mandrel **96** and inner polymeric liner **68.** The outer tubular layer conforms to the portion of the inner polymeric layer around the tapered mandrel **96,** and the portion of the inner polymeric liner **68** around the second mandrel **97** extends through the longitudinal cut in the outer polymeric tubular layer **70.**

A split tool can be inserted into the portion of the lumen of the inner liner that was previously occupied by the second mandrel (step **2008**). The split tool can then be used to form folds and/or pleats in the excess portion of the inner liner, which now extends through the longitudinal cut in the outer polymeric tubular layer (step **2010**). A radiopaque marker band can optionally be applied at the distal end of the sheath (step **2012).** Heat shrink tubing, such as FEP heat shrink tubing, can be applied over the entire sheath, and heat can be applied to compress the components of the sheath and bond or fuse them together (step b). The split tool, heat shrink tubing, and second mandrel can then be removed (step **2016**). The sheath can then be utilized with a delivery apparatus, such as by bonding the proximal end of the sheath to a polycarbonate housing of a delivery apparatus or catheter assembly (step **2018**).

**FIG. 32H** illustrates an elevation view of the sheath at step **2018** of **FIG. 31****.** The sheath **66,** made according to described methods and processes, can be attached or bonded to a housing **101,** such as by bonding the proximal end of the sheath **66** to the polycarbonate housing.

In another example, disclosed expandable sheaths can be made using a reflowed mandrel process. A mandrel can be provided, with the size of the mandrel defining the inner diameter of the sheath lumen in its resting configuration. A tube of material, such as a PTFE tube that will become the sheath's inner liner, can be provided with an inner diameter greater than that of the mandrel (e.g., a 9 mm PTFE tube can be mounted on a 6 mm mandrel). The PTFE tube can be mounted on the mandrel and prepared into the final folded configuration by folding the excess material of the PTFE tube over to one or both sides. An HDPE tube that will serve as the outer layer can then be placed over the PTFE liner. The two-layer assembly can then be thermally fused together. For example, a reflow process can be performed where the assembly is heated to a temperature high enough such that the inner and/or outer layers are at least partially melted and are then fused together as the heat is removed and the assembly cools.

An elongated tube, as described herein, is then placed over at least part of the fused layers (*e.g*., over a proximal section of the sheath) and held in place using a thermal process (step **2020**) to form an outer jacket or a strain relief jacket. In some aspects, the same thermal process can bond the layers of the sheath and the elongated tube. In other aspects, a first thermal process can be used to fuse the layers of the sheath, and a second thermal process can be used to secure the elongated tube to other layers of the sheath. In still further aspects, the elongated tube can cover the whole length of the sheath or at least a portion of the sheath. In some aspects, a distal soft tip can then be attached to the shaft of the expandable sheath.

In some aspects, the outer layer can be co-extruded with an adhesive layer, such as a layer formed from Tecoflex^{™}, such that the Tecoflex^{™} is positioned on an inner surface of the outer layer - in this manner, the Tecoflex^{™} will be positioned between the inner and outer layers in the completed sheath. In these aspects, an HDPE tube can be provided with a coating of Tecoflex^{™} on the inner surface. The HDPE tube can be slit along the length of the tube to open and flatten it and then cut using a template in some aspects. For example, for specific applications, portions of the outer layer can be cut and removed using a template. The cut HDPE can then be placed on the inner layer on the mandrel. In some aspects, only a portion of the outer layer will have the adhesive Tecoflex^{™}. In these aspects, the sections without Tecoflex^{™} will only be partially fused to the inner layer. In some aspects, the entire inner surface of the outer layer will have the Tecoflex^{™}, and the inner surface of the outer layer can be positioned so that it contacts the inner layer on the mandrel. To position the inner and outer layers, as shown in the sheath of **FIG. 39****,** the folded portion of the inner layer can be lifted up, and an edge of the outer layer can be tucked beneath the fold.

Some additional sheaths, as disclosed herein, are also can be prepared according to the methods disclosed below. For example, also disclosed are aspects where the sheath is formed by forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the cylinder having a longitudinal axis. The elongated tube formed as disclosed above can be used to form an outer layer of the sheath. In such aspects, the outer layer will comprise and the disclosed herein elongated tube and a braid.

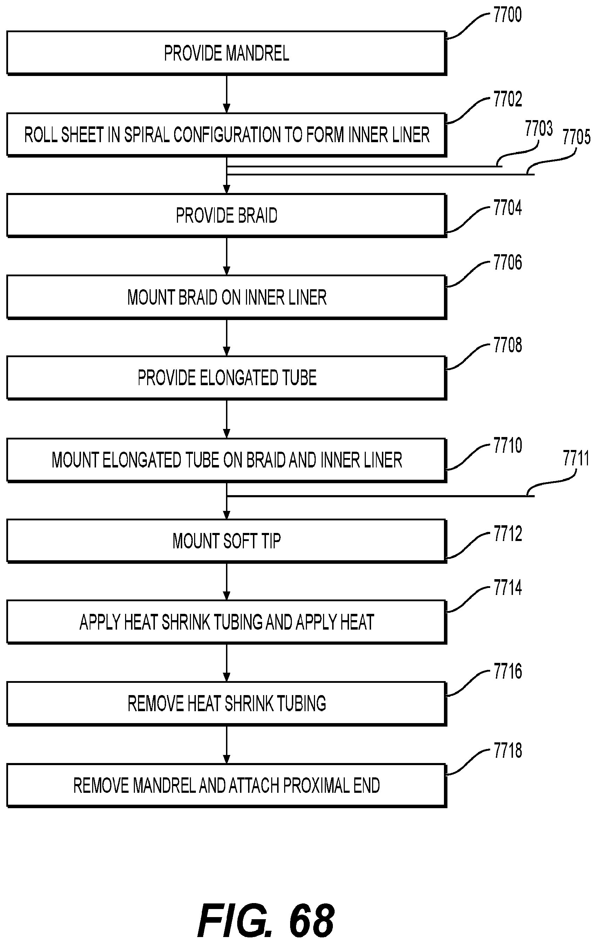

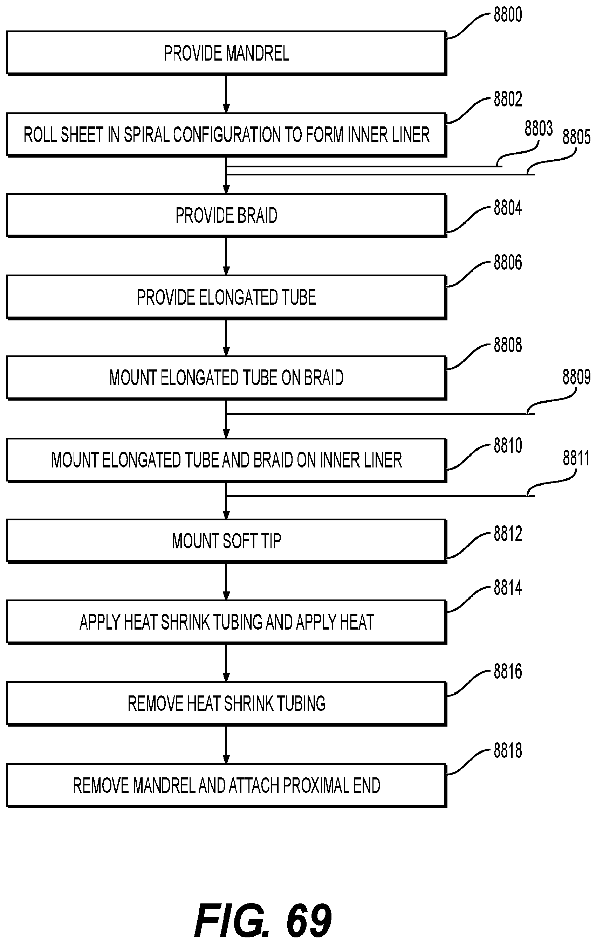

**FIGs. 68** and **69** exemplify block diagrams of exemplary methods of producing the sheath in various aspects. The various methods steps are also depicted in **FIGs. 70A-70J****.** In certain aspects, and as shown in **FIG. 70A****,** the inner liner can be formed from an extruded tube **1903,** having an inner surface and outer surface and having any thickness that is described above. This extruded tube can be cut **1905** along the length to form a sheet. In certain aspects, the inner surface and/or outer surface of the tube can be surface-treated, such as, for example, by plasma etching, chemical etching, or other suitable methods of surface treatment. In some exemplary aspects, where the outer surface of the inner liner is treated, the treatment can provide for better bonding with the outer layer when formed. In yet other aspects, the inner surface of the inner liner can be ribbed. In such exemplary aspects, the ribbed surface facilitates a reduction of contact points with the prosthetic device and can reduce friction. In still further aspects, the initial extruded tube **1903** can be produced by co-extrusion with multiple layers of the same or different polymers as described herein. It is understood that one of ordinary skill in the art can choose the composition of the inner liner depending on the desired application. In certain aspects, the decision to use a specific material for the inner liner can be dependent on the desired stiffness, wall-thickness, and lubricious optimization.

In still further aspects, one or more mandrels can be provided (step **7700** or **8800** in **FIG. 68** and **69****,** respectively). The mandrel can be provided with an exterior coating, such as a Teflon^{®} coating, and the mandrel's diameter can be predetermined based on the desired rest diameter *dᵣ* of the resulting sheath. As shown in **FIG. 70B****,** the sheet formed by cutting **1905** the extruded tube **1903** can be rolled in a spiral configuration (steps **7702** and **8802** in **FIGS. 68** and **69** respectively) around the mandrel **1901** to form the inner liner **1902a** such that at least a portion of the inner surface of the sheet overlays at least a portion of the outer surface of the sheet thereby forming an overlaying portion **1902c** and wherein the first edge (not shown) of the sheet is slidable along at least a portion the inner surface of the sheet and the second edge **1902b** is slidable along at least a portion of the outer surface of the sheet.

In still further exemplary aspects, in steps **7705** and **8805** (**FIGS. 68** and **69** respectively), an amount of a first lubricant **1910** (**FIGS. 70C-70E**) can be optionally applied on the outer surface of the inner liner. The presence of this lubricant material can reduce the friction between the inner liner and the outer layer of the final sheath. In yet other aspects, in steps **7703** and **8803,** an amount of a second lubricant **1908** can be applied between the overlaying and sliding portions of the inner liner to further improve slidability and decrease friction. (**FIG. 70D** depicts the inner liner with the two optional lubricants present with the mandrel hidden from the view). In still further aspects, it is understood that the inner liner formed with the use of mandrel can have any rest diameter, as described above. In certain aspects, the rest diameter is substantially uniform along the longitudinal axis of the lumen. While in the other aspects, the rest diameter *dᵣ* varies along the longitudinal axis of the lumen and wherein the rest diameter *dᵣ* at the proximal end that is larger than the rest diameter *dᵣ* at the distal end.

In still further aspects, the methods can further comprise a step of providing a braid (steps **7704** and **8804**). It is understood that any of the described above braids can be used in this step. In still further aspects, and as shown in step **7706** of **FIG. 68****,** the braid is mounted on the inner liner. In some exemplary aspects, and as shown in **FIG. 70F** the braid **1904** can be mounted on the first lubricant **1910** that can be present on the outer surface of the inner liner. It is understood that in some aspects, the second lubricant can be present only at a portion of the outer surface of the inner liner. In yet other aspects, the disclosed sheath can have segments where the first lubricant is present, and the braid is mounted over it, while it can have other segments where the second lubricant is not present, and the braid is mounted directly on the outer surface of the inner liner. It is understood that the location of these specific segments can be determined by one of ordinary skill in the art depending on the desired application. It is understood that the mounting of the braid can be done by any known in the art methods. In some unlimiting aspects, the braid can be provided as a cylindrical tube, and it can be slid on top of the inner liner or the first lubricant if it is present.

In yet further aspects and as shown in step **7708,** the method can further comprise a step of providing the disclosed herein elongated tube. In certain and unlimiting aspects, the elongated tube is extruded from the disclosed herein compositions and is provided as a cylindrical tube **1906** (**FIG. 70G**). In a still further aspect, the disclosed elongated tube can be mounted on the inner liner and the braid (step **7710**). **FIG. 70G****,** for example, depicts an aspect where the disclosed herein elongated tube **1906** is used to slide on the inner liner having a first lubricant **1910** overlaying the inner liner's outer surface and the braid **1904.**

In yet further aspects, the disclosed method can comprise a step of embedding (step **7711,** **FIG. 68**) the braid into the elongated tube. It is understood that the sheath can comprise various segments. In some aspects, some of the segments can comprise the braid embedded within the elongated tube, while in other segments, the braid and the elongated tube are separate. It is further understood that in some aspects, the sheath can have a braid embedded within the elongated tube over the whole length of the sheath, while in other aspects, the braid is not embedded within the elongated tube over the whole length of the sheath. It is further understood that any methods known in the art can be used to embed the braid within the elongated tube. In some aspects, application of the heat can be utilized. In certain aspects, the use of heat shrink tubing can be utilized to embed the braid within the disclosed herein elongated tube. It is understood that after the step of embedment is complete, the heat shrink tubing is removed.

In still further aspects, a soft, atraumatic tip can be provided at the distal end of the resulting sheath (step **7712**). In yet further aspects, the outer layer comprising the braid and the layer of the elastomeric polymer is at least partially bonded to the inner liner. It is understood that this bonding can also be achieved by any known in the art methods. In certain aspects, and as shown in step **7714,** a heat shrink is applied to the portion that is being bound and heated to form a bonding between the inner liner and the outer layer. In yet other aspects, the bonding between the inner liner and the outer layer can be achieved by placing the assembly in an oven or otherwise heating it. In still further aspects, the bonding is performed by heating at a temperature from about 350 °F to about 550 °F for a time period effective to form a bond between at least a portion of the outer layer and at least a portion of the inner liner. In yet further aspects, the heating can be done at a temperature of about 375 °F, about 400 °F, about 425 °F, about 450 °F, about 475 °F, about 500 °F, or about 525 °F. In yet other aspects, the time period effective to form a bond can comprise from about 1 second to about 60 seconds, including exemplary values of about 5 seconds, about 10 seconds, about 15 seconds, about 20 seconds, about 25 seconds, about 30 seconds, about 35 seconds, about 40 seconds, about 45 seconds, about 50 seconds, and about 55 seconds. However, it is further understood that this time period is not limiting, and it can have any value needed to provide for an effective bond, for example, it can have any value from about 1 second to about 5 hours. It is further understood that if the heat shrink tubing is used to obtain the desired bonding, the heat shrink tubing is removed (step **7716,** **FIG. 68**).

In still further aspects and as shown in **FIG. 70I****,** the bonding step can also comprise applying a first strip of a polymer **1920** along at least a portion of the longitudinal axis of the lumen to at least a portion of the outer surface of the sheet that does not comprise the overlaying portion **1902c** prior to or during the step of bonding the at least a portion of the inner surface of the elongated tube to at least a portion of the outer surface of the sheet of the inner liner. It is understood that in some aspects, the first strip of the polymer can be made of the same material as the elongated tube itself. While in other aspects, the first strip of the polymer can be made of any material that allows an efficient bonding between the inner liner and the elongated tube. It is understood that in some exemplary aspects and as shown in the **FIG. 70I****,** this first strip can be applied prior to mounting the braid. In still further aspects, the location where the first strip is applied does not comprise the first lubricant. However, it is understood that in such aspects, the first lubricant can be present in other locations.

In still further exemplary aspects, the methods can comprise applying a second strip **1922a** of a polymer to at least a portion of the outer surface of the sheet at the proximal end of the sheath prior to or during the step of bonding the at least a portion of the inner surface of the disclosed herein elongated tube to at least a portion of the outer surface of the sheet of the inner liner. It is understood that in some aspects, the second strip of the polymer can be made of the same material as the elongated tube itself. While in other aspects, the second strip of the polymer can be made of any material that allows an efficient bonding between the inner liner and the elongated tube. It is further understood that the first and the second strips can be made from the same or different polymers.

It is understood that in some exemplary aspects and as shown in the **FIG. 70J****,** this second strip can be applied prior to mounting the braid. In still further aspects, the location where the second strip is applied does not comprise the first lubricant. However, it is understood that in such aspects, the first lubricant can be present in other locations. In yet other aspects, the method can comprise a third strip of polymer **1922b** that can be applied to at least a portion of the outer surface of the sheet at the distal end of the sheath prior to or during the step of bonding the at least a portion of the inner surface of the disclosed herein elongated tube to at least a portion of the outer surface of the sheet of the inner liner. It is understood that in some aspects, the third strip of the polymer can be made of the same material as the elongated tube itself. While in other aspects, the third strip of the polymer can be made of any material that allows an efficient bonding between the inner liner and the elongated tube. It is further understood that the first, the second, and/or third strips can be made from the same or different polymers.

It is understood that in some exemplary aspects and as shown in the **FIG. 70J****,** this third strip can be applied prior to the mounting the braid. In still further aspects, the location where the third strip is applied does not comprise the first lubricant. However, it is understood that in such aspects, the first lubricant can be present in other locations. In still further aspects, both the second and third strips are present. While in other aspects, only one of the second or the third strips is present. It is further understood that the first, second, and third strips can be made from the same or different polymers.

Some alternative aspects are shown in **FIG. 69** and **FIG. 70H****.** In such alternative aspects, the outer layer is pre-formed by combining the elongated tube and braid together and then mounted on the inner liner positioned on the mandrel. In such aspects, the disclosed elongated tube is first mounted on the braid (step **8808**) prior to the mounting it on the inner liner. In yet other aspects, the method can also comprise a step of partially embedding the braid within the elongated tube before mounting both of them on the inner liner (step **8809**). However, the step of partially embedding the braid with the elongated tube can be done after the braid and the elongated tube are mounted on the inner liner (step **8811**). Steps **8812-8818** can be performed analogously to the steps **7712-7718.**

In still further aspects, and as shown in **FIG. 70I****,** the bonding step can also comprise a first strip **1920** being applied along at least a portion of the longitudinal axis of the lumen to at least a portion of the outer surface of the sheet that does not comprise the overlaying portion **1902c** prior to or during the step of bonding the at least a portion of the inner surface of the elongated tube to at least a portion of the outer surface of the sheet of the inner liner. It is understood that in some exemplary aspects and as shown in the **FIG. 70I****,** this first strip can be applied prior to the mounting pre-formed outer layer comprising the braid and the elongated tube. In still further aspects, the location where the first strip is applied does not comprise the first lubricant. However, it is understood that in such aspects, the first lubricant can be present in other locations.

It is also understood that these alternative aspects can also include a step where a second strip **1922a** can be applied to at least a portion of the outer surface of the sheet at the proximal end of the sheath prior to or during the step of bonding the at least a portion of the inner surface of the elongated tube to at least a portion of the outer surface of the sheet of the inner liner. It is understood that in some exemplary aspects and as shown in the **FIG. 70J****,** this second strip can be applied prior to the mounting pre-formed outer layer comprising the braid and the elongated tube. In still further aspects, the location where the second strip is applied does not comprise the first lubricant. However, it is understood that in such aspects, the first lubricant can be present in other locations. In yet other aspects, these methods can also comprise a third strip **1922b** that can be applied to at least a portion of the outer surface of the sheet at the distal end of the sheath prior to or during the step of bonding the at least a portion of the inner surface of the disclosed elongated tube to at least a portion of the outer surface of the sheet of the inner liner. It is understood that in some exemplary aspects and as shown in the **FIG. 70J****,** this third strip can be applied prior to the mounting pre-formed outer layer comprising the braid and the elongated tube. In still further aspects, the location where the third strip is applied does not comprise the first lubricant. However, it is understood that in such aspects, the first lubricant can be present in other locations. In still further aspects, both the second and the third elastomeric polymers are present.

Again, it is understood that the method that does not include the application of the braid is also disclosed. In such methods, the elongated braid as disclosed herein forms the outer layer of the sheath, and the braid is not present.

Also disclosed herein are aspects where the disclosed herein methods can comprise a step of disposing a hydrophilic coating layer on the outer surface of the elongated tube of any of the exemplary sheaths. Any disclosed herein hydrophilic coating can be used.

Any of the disclosed herein sheaths can also be attached to the housing **101,** as shown in **FIG. 32H** or a soft tip **102** as, for example, shown in **FIG.36****.**

Sheaths of the present disclosure are suitable for introducing a prosthetic device into a patient's vasculature. One such procedure comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some procedures, the expandable sheath can be sutured to the patient's skin at the insertion site so that once the sheath is inserted the proper distance within the patient's vasculature, it does not move once the implantable device starts to travel through the sheath.

Disclosed aspects of an expandable sheath can be used with other delivery and minimally invasive surgical components, such as an introducer and loader. In one aspect, the expandable sheath can be flushed to purge any air within the sheath, using, for example, flush port **103** (**FIG. 35**). An introducer can be inserted into the expandable sheath, and the introducer/sheath combination can be fully inserted into vasculature over a guiding device, such as a 0.35" guidewire. Preferably, the seam formed by the intersection of the folded portion of the inner layer and the overlapping portion of the outer layer can be positioned such it is oriented downward (posterior). Once the sheath and introducer are fully inserted into a patient's vasculature, in some aspects, the expandable sheath can be sutured in place at the insertion site. In this manner, the expandable sheath can be substantially prevented from moving once positioned within the patient.

The introducer can then be removed, and a medical device, such as a transcatheter heart valve, can be inserted into the sheath, in some instances, using a loader. Such procedures can additionally comprise placing the tissue heart valve in a crimped state on the distal end portion of an elongated delivery apparatus and inserting the elongated delivery device with the crimped valve into and through the expandable sheath. Next, the delivery apparatus can be advanced through the patient's vasculature to the treatment site, where the valve can be implanted.

Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (e.g., the expanded configuration) just in the area where the medical device is currently located. Once the medical device passes a particular location of the sheath, the sheath can at least partially contract to the smaller diameter of its original configuration. The expandable sheath can thus be expanded without the use of inflatable balloons or other dilators. Once the medical device is implanted, the sheath and any sutures holding in place can be removed. In some aspects, it is preferable to remove the sheath without rotating it.

## Claims

1. A sheath (22; 66) for delivering a medical device, wherein the sheath (22; 66) has a proximal and a distal end and comprises
an elongated tube forming an outer layer (26) of the sheath (22; 66) that is positioned at at least the proximal end of the sheath (22; 66) and extending along at least a portion of a length of the sheath (22; 66), having an inner surface (32) and an outer surface (34), and wherein the elongated tube comprises a first polymer layer, wherein the first polymer layer comprises a first compound composition comprising
from greater than 0 wt% to less than 100 wt% of a first polymer comprising a polyether block amide, a polyurethane, or a combination thereof based on a total weight of the first compound composition;
from greater than 0% to less than about 65% of an inorganic filler based on a total weight of the first compound composition; and
from greater than 0% up to about 20% of a solid lubricant filler based on a total weight of the first compound composition;
wherein the elongated tube is configured to reversibly expand from an initial diameter d₀ in an unexpended position to an expanded diameter dₑ in an expanded position upon passage of a medical device; and
wherein the sheath (22; 66) exhibits at least a 10% reduction in an insertion force when compared with a substantially identical reference sheath that does not comprise the first polymer layer; and wherein the elongated tube is substantially kink resistant.

2. The sheath (22; 66) of claim 1, wherein a durometer of the first polymer at a proximal end of the elongated tube is different from a durometer of the first polymer at a distal end of the elongated tube and has a Shore D from about 20D to about 35D.

3. The sheath (22; 66) of any one of claims 1 and 2, wherein the first polymer comprises polyether block amide elastomer or polyurethane.

4. The sheath (22; 66) of any one of claims 1 to 3, wherein the inorganic filler comprises bismuth oxychloride, barium sulfate, bismuth subcarbonate, calcium carbonate, aluminum trihydrate, barite, kaolin clay, limestone, or any combination thereof and is present in an amount of at least about 10 %, and preferably less than about 50 %, based on a total weight of the first compound composition.

5. The sheath (22; 66) of any one of claims 1 to 4, wherein the solid lubricant comprises a PTFE filler.

6. The sheath (22; 66) of any one of claims 1 to 5, wherein the first compound composition further comprises at least one tackiness reducing compound present in an amount from about 1 % to about 20 % based on a total weight of the first compound composition.

7. The sheath (22; 66) of any one of claims 1 to 6, wherein the elongated tube comprises two or more polymer layers (24, 26, 28; 68, 70; 81).

8. The sheath (22; 66) of claim 7, wherein the elongated tube comprises at least a second polymer layer (26; 70) comprising a second compound composition comprising from greater than 0 wt% to 100 wt% of a second polymer comprising polyether block amide, a polyurethane, or a composition thereof; and wherein the second polymer has a Shore A Durometer from about 20A to about 65A.

9. The sheath (22; 66) of claim 8, wherein the second compound composition further comprises up to 20 % of tackiness reducing additive based on a total weight of the second compound composition.

10. The sheath (22; 66) of any one of claims 1 to 9, wherein the elongated tube exhibits a friction force of less than about 10 N in the dry state against a substrate surface comprising one or more of polytetrafluoroethylene or high-density polyethylene.

11. The sheath (22; 66) of any one of claims 1 to 10, wherein the elongated tube exhibits a friction force of less than about 7 N in the dry state against a substrate surface comprising one or more of polytetrafluoroethylene or high-density polyethylene.

12. The sheath (22; 66) of any one of claims 1 to 11, wherein the elongated tube exhibits a hoop direction force at 10 mm extension of less than about 8 N.

13. The sheath (22; 66) of any one of claims 1 to 12, wherein the elongated tube exhibits an elongation at break of ranging between about 600% and about 800%.

14. The sheath (66) of any one of claims 1 to 13, further comprising
an expandable tubular inner liner (68) extending along the length of the sheath (66) and comprising at least one folded portion, wherein the expandable inner liner (68) has an inner surface, and an outer surface, wherein the inner surface of the expandable inner liner (68) defines a lumen and forms an inner surface of the at least one folded portion, and wherein the outer surface extends circumferentially to form an outer surface of the at least one folded portion; and
a first outer tubular layer extending at least partially along the length of the sheath (66) and having an inner surface and an outer surface, wherein the inner surface of the first outer tubular layer further extends at least partially around the outer surface of the inner liner (68) such that at least a portion of the inner surface of the first outer tubular layer is positioned adjacent to the outer surface of the at least one folded portion of the inner liner (68);
wherein the elongated tube is positioned such that at least a portion of the inner surface of the elongated tube overlies at least a portion of the outer surface of the first outer tubular layer; and wherein the sheath (66) exhibits an insertion force of less than about 55 N when a medical device is pushed through the sheath.

15. A method of making a sheath (22; 66) according to any of claims 1 to 14, comprising:
a) extruding a tubular body to form an elongated tube comprising a first polymer layer (24; 68), wherein the first polymer layer (24; 68) comprises a first compound composition comprising
from greater than 0 % to less than 100 % of a polymer comprising a polyether block amide, a polyurethane, or a combination thereof based on a total weight of the first compound composition;
from greater than 0 % to less than about 65% of an inorganic filler based on a total weight of the first compound composition; and
from greater than 0 % up to about 20 % of a solid lubricant filler based on a total weight of the first compound composition;
b) disposing the elongated tube on the sheath (22; 66) such that the elongated tube forms an outer layer (70) of the sheath (22; 66), and wherein the elongated tube is positioned at at least the proximal end of the sheath (22; 66) and extending along at least a portion of a length of the sheath (22; 66), wherein the elongated tube is configured to reversibly expand from an initial diameter d₀ in an unexpended position to an expanded diameter dₑ in an expanded position upon passage of a medical device; and
wherein the formed sheath (22; 66) exhibits at least a 10% reduction in an insertion force when compared with a substantially identical reference sheath that does not comprise the first polymer layer (24; 68).

## Patentansprüche

1. Hülle (22; 66) zum Zuführen einer medizinischen Vorrichtung, wobei die Hülle (22; 66) ein proximales und ein distales Ende aufweist und umfasst:
eine längliche Röhre, die eine äußere Schicht (26) der Hülle (22; 66) bildet, die an wenigstens dem proximalen Ende der Hülle (22; 66) positioniert ist und sich entlang wenigstens eines Abschnitts einer Länge der Hülle (22; 66) erstreckt, mit einer Innenfläche (32) und einer Außenfläche (34), und wobei die längliche Röhre eine erste Polymerschicht umfasst, wobei die erste Polymerschicht eine erste Verbundzusammensetzung umfasst, umfassend
von größer als 0 Gew.-% bis weniger als 100 Gew.-% eines ersten Polymers, umfassend ein Polyether-Blockamid, ein Polyurethan oder eine Kombination davon, bezogen auf ein Gesamtgewicht der ersten Verbundzusammensetzung;
von größer als 0 % bis weniger als etwa 65 % eines anorganischen Füllstoffs, bezogen auf ein Gesamtgewicht der ersten Verbundzusammensetzung; und
von größer als 0 % bis zu etwa 20 % eines festen Schmiermittel-Füllstoffs, bezogen auf ein Gesamtgewicht der ersten Verbundzusammensetzung;
wobei die längliche Röhre dazu konfiguriert ist, beim Durchtritt einer medizinischen Vorrichtung reversibel von einem anfänglichen Durchmesser d₀ in einer nicht-expandierten Stellung zu einem expandierten Durchmesser dₑ in einer expandierten Stellung zu expandieren; und
wobei die Hülle (22; 66) im Vergleich zu einer im Wesentlichen identischen Referenzhülle, die die erste Polymerschicht nicht umfasst, eine Reduzierung einer Einführkraft um wenigstens 10 % zeigt; und wobei die längliche Röhre im Wesentlichen knickresistent ist.

2. Hülle (22; 66) nach Anspruch 1, wobei sich ein Härtegrad (Shore-Härte) des ersten Polymers an einem proximalen Ende der länglichen Röhre von einem Härtegrad (Shore-Härte) des ersten Polymers an einem distalen Ende der länglichen Röhre unterscheidet und einen Shore D von etwa 20D bis etwa 35D aufweist.

3. Hülle (22; 66) nach einem der Ansprüche 1 und 2, wobei das erste Polymer ein Polyether-Blockamid-Elastomer oder Polyurethan umfasst.

4. Hülle (22; 66) nach einem der Ansprüche 1 bis 3, wobei der anorganische Füllstoff Bismutoxychlorid, Bariumsulfat, Bismutsubcarbonat, Calciumcarbonat, Aluminiumtrihydrat, Baryt, Kaolinton, Kalkstein oder eine beliebige Kombination davon umfasst und in einer Menge von wenigstens etwa 10 %, und vorzugsweise weniger als etwa 50 % vorhanden ist, bezogen auf ein Gesamtgewicht der ersten Verbundzusammensetzung.

5. Hülle (22; 66) nach einem der Ansprüche 1 bis 4, wobei das feste Schmiermittel einen PTFE-Füllstoff umfasst.

6. Hülle (22; 66) nach einem der Ansprüche 1 bis 5, wobei die erste Verbundzusammensetzung ferner wenigstens eine klebrigkeitsreduzierende Verbindung umfasst, die in einer Menge von etwa 1 % bis etwa 20 % vorhanden ist, bezogen auf ein Gesamtgewicht der ersten Verbundzusammensetzung.

7. Hülle (22; 66) nach einem der Ansprüche 1 bis 6, wobei die längliche Röhre zwei oder mehr Polymerschichten (24, 26, 28; 68, 70; 81) umfasst.

8. Hülle (22; 66) nach Anspruch 7, wobei die längliche Röhre wenigstens eine zweite Polymerschicht (26; 70) umfasst, die eine zweite Verbundzusammensetzung umfasst, umfassend von größer als 0 Gew.-% bis 100 Gew.-% eines zweiten Polymers, umfassend Polyether-Blockamid, ein Polyurethan oder eine Zusammensetzung davon; und wobei das zweite Polymer einen Härtegrad Shore A von etwa 20A bis etwa 65A aufweist.

9. Hülle (22; 66) nach Anspruch 8, wobei die zweite Verbundzusammensetzung ferner bis zu 20 % eines klebrigkeitsreduzierenden Additivs umfasst, bezogen auf ein Gesamtgewicht der zweiten Verbundzusammensetzung.

10. Hülle (22; 66) nach einem der Ansprüche 1 bis 9, wobei die längliche Röhre im trockenen Zustand eine Reibungskraft von weniger als etwa 10 N gegen eine Substratoberfläche, umfassend eines oder mehrere von Polytetrafluorethylen oder Polyethylen hoher Dichte, zeigt.

11. Hülle (22; 66) nach einem der Ansprüche 1 bis 10, wobei die längliche Röhre im trockenen Zustand eine Reibungskraft von weniger als etwa 7 N gegen eine Substratoberfläche, umfassend eines oder mehrere von Polytetrafluorethylen oder Polyethylen hoher Dichte, zeigt.

12. Hülle (22; 66) nach einem der Ansprüche 1 bis 11, wobei die längliche Röhre bei einer Ausdehnung von 10 mm eine Kraft in Umfangsrichtung von weniger als etwa 8 N zeigt.

13. Hülle (22; 66) nach einem der Ansprüche 1 bis 12, wobei die längliche Röhre eine Bruchdehnung im Bereich zwischen etwa 600 % und etwa 800 % zeigt.

14. Hülle (66) nach einem der Ansprüche 1 bis 13, ferner umfassend
eine expandierbare rohrförmige Innenauskleidung (68), die sich entlang der Länge der Hülle (66) erstreckt und wenigstens einen gefalteten Abschnitt umfasst, wobei die expandierbare Innenauskleidung (68) eine Innenfläche und eine Außenfläche aufweist, wobei die Innenfläche der expandierbaren Innenauskleidung (68) ein Lumen definiert und eine Innenfläche des wenigstens einen gefalteten Abschnitts bildet, und wobei sich die Außenfläche in Umfangsrichtung erstreckt, um eine Außenfläche des wenigstens einen gefalteten Abschnitts zu bilden; und
eine erste äußere rohrförmige Schicht, die sich wenigstens teilweise entlang der Länge der Hülle (66) erstreckt und eine Innenfläche und eine Außenfläche aufweist, wobei sich die Innenfläche der ersten äußeren rohrförmigen Schicht ferner wenigstens teilweise um die Außenfläche der Innenauskleidung (68) erstreckt, derart, dass wenigstens ein Abschnitt der Innenfläche der ersten äußeren rohrförmigen Schicht benachbart zu der Außenfläche des wenigstens einen gefalteten Abschnitts der Innenauskleidung (68) positioniert ist;
wobei die längliche Röhre derart positioniert ist, dass wenigstens ein Abschnitt der Innenfläche der länglichen Röhre wenigstens einen Abschnitt der Außenfläche der ersten äußeren rohrförmigen Schicht überlagert; und wobei die Hülle (66) eine Einführkraft von weniger als etwa 55 N zeigt, wenn eine medizinische Vorrichtung durch die Hülle geschoben wird.

15. Verfahren zur Herstellung einer Hülle (22; 66) nach einem der Ansprüche 1 bis 14, umfassend:
a) Extrudieren eines rohrförmigen Körpers, um eine längliche Röhre zu bilden, die eine erste Polymerschicht (24; 68) umfasst, wobei die erste Polymerschicht (24; 68) eine erste Verbundzusammensetzung umfasst, umfassend
von größer als 0 % bis weniger als 100 % eines Polymers, umfassend ein Polyether-Blockamid, ein Polyurethan oder eine Kombination davon, bezogen auf ein Gesamtgewicht der ersten Verbundzusammensetzung;
von größer als 0 % bis weniger als etwa 65 % eines anorganischen Füllstoffs, bezogen auf ein Gesamtgewicht der ersten Verbundzusammensetzung; und
von größer als 0 % bis zu etwa 20 % eines festen Schmiermittel-Füllstoffs, bezogen auf ein Gesamtgewicht der ersten Verbundzusammensetzung;
b) Anordnen der länglichen Röhre auf der Hülle (22; 66), derart, dass die längliche Röhre eine äußere Schicht (70) der Hülle (22; 66) bildet, und wobei die längliche Röhre an wenigstens dem proximalen Ende der Hülle (22; 66) positioniert ist und sich entlang wenigstens eines Abschnitts einer Länge der Hülle (22; 66) erstreckt, wobei die längliche Röhre dazu konfiguriert ist, beim Durchtritt einer medizinischen Vorrichtung reversibel von einem anfänglichen Durchmesser d₀ in einer nicht-expandierten Stellung zu einem expandierten Durchmesser dₑ in einer expandierten Stellung zu expandieren; und
wobei die gebildete Hülle (22; 66) im Vergleich zu einer im Wesentlichen identischen Referenzhülle, die die erste Polymerschicht (24; 68) nicht umfasst, eine Reduzierung einer Einführkraft um wenigstens 10 % zeigt.

## Revendications

1. Gaine (22 ; 66) pour la pose d'un dispositif médical, la gaine (22 ; 66) présentant une extrémité proximale et une extrémité distale et comprenant
un tube allongé formant une couche externe (26) de la gaine (22 ; 66) qui est positionnée au niveau d'au moins l'extrémité proximale de la gaine (22 ; 66) et s'étendant le long d'au moins une partie d'une longueur de la gaine (22 ; 66), présentant une surface interne (32) et une surface externe (34) et le tube allongé comprenant une première couche de polymère, la première couche de polymère comprenant une première composition combinée comprenant
plus de 0 % en poids à moins de 100 % en poids d'un premier polymère comprenant un polyéther-bloc-amide, un polyuréthane ou une combinaison correspondante sur la base d'un poids total de la première composition combinée ;
plus de 0 % à moins d'environ 65 % d'une charge inorganique sur la base d'un poids total de la première composition combinée ; et
plus de 0 % jusqu'à environ 20 % d'une charge lubrifiante solide sur la base d'un poids total de la première composition combinée ;
le tube allongé étant conçu pour s'étendre de manière réversible à partir d'un diamètre initial d₀ dans une position non étendue à un diamètre étendu dₑ dans une position étendue lors du passage d'un dispositif médical ; et
la gaine (22 ; 66) présentant au moins une réduction de 10 % d'une force d'insertion par rapport à une gaine de référence sensiblement identique qui ne comprend pas la première couche de polymère ; et le tube allongé étant sensiblement résistant au pliage.

2. Gaine (22 ; 66) selon la revendication 1, un duromètre du premier polymère au niveau d'une extrémité proximale du tube allongé étant différent d'un duromètre du premier polymère au niveau d'une extrémité distale du tube allongé et présentant un duromètre Shore D d'environ 20D à environ 35D.

3. Gaine (22 ; 66) selon l'une quelconque des revendications 1 et 2, le premier polymère comprenant un élastomère de polyéther-bloc-amide ou du polyuréthane.

4. Gaine (22 ; 66) selon l'une quelconque des revendications 1 à 3, la charge inorganique comprenant de l'oxychlorure de bismuth, du sulfate de baryum, du souscarbonate de bismuth, du carbonate de calcium, de l'aluminium trihydraté, de la baryte, de l'argile de kaolin, du calcaire ou toute combinaison correspondante et étant présente en une quantité d'au moins environ 10 % et de préférence inférieure à environ 50 %, sur la base d'un poids total de la première composition combinée.

5. Gaine (22 ; 66) selon l'une quelconque des revendications 1 à 4, le lubrifiant solide comprenant une charge de PTFE.

6. Gaine (22 ; 66) selon l'une quelconque des revendications 1 à 5, la première composition combinée comprenant en outre au moins un composé réducteur de pégosité présent en une quantité d'environ 1 % à environ 20 % sur la base d'un poids total de la première composition combinée.

7. Gaine (22 ; 66) selon l'une quelconque des revendications 1 à 6, le tube allongé comprenant deux couches de polymère (24, 26, 28 ; 68, 70 ; 81) ou plus.

8. Gaine (22 ; 66) selon la revendication 7, le tube allongé comprenant au moins une seconde couche de polymère (26 ; 70) comprenant une seconde composition combinée comprenant plus de 0 % en poids à 100 % en poids d'un second polymère comprenant un polyéther-bloc-amide, un polyuréthane ou une composition correspondante ; et le second polymère présentant un duromètre Shore A d'environ 20A à environ 65A.

9. Gaine (22 ; 66) selon la revendication 8, la seconde composition combinée comprenant en outre jusqu'à 20 % d'additif réducteur de pégosité sur la base d'un poids total de la seconde composition combinée.

10. Gaine (22 ; 66) selon l'une quelconque des revendications 1 à 9, le tube allongé présentant une force de frottement inférieure à environ 10 N à l'état sec contre une surface de substrat comprenant une ou plusieurs substances parmi le polytétrafluoroéthylène ou le polyéthylène haute densité.

11. Gaine (22 ; 66) selon l'une quelconque des revendications 1 à 10, le tube allongé présentant une force de frottement inférieure à environ 7 N à l'état sec contre une surface de substrat comprenant une ou plusieurs substances parmi le polytétrafluoroéthylène ou le polyéthylène haute densité.

12. Gaine (22 ; 66) selon l'une quelconque des revendications 1 à 11, le tube allongé présentant une force de direction circonférentielle à une extension de 10 mm inférieure à environ 8 N.

13. Gaine (22 ; 66) selon l'une quelconque des revendications 1 à 12, le tube allongé présentant un allongement à la rupture situé dans la plage entre environ 600 % et environ 800 %.

14. Gaine (66) selon l'une quelconque des revendications 1 à 13, comprenant en outre
un revêtement interne tubulaire extensible (68) s'étendant le long de la longueur de la gaine (66) et comprenant au moins une partie pliée, le revêtement interne extensible (68) présentant une surface interne et une surface externe, la surface interne du revêtement interne extensible (68) définissant une lumière et formant une surface interne de ladite au moins une partie pliée et la surface externe s'étendant de manière circonférentielle afin de former une surface externe de ladite au moins une partie pliée ; et
une première couche tubulaire externe s'étendant au moins partiellement le long de la longueur de la gaine (66) et présentant une surface interne et une surface externe, la surface interne de la première couche tubulaire externe s'étendant en outre au moins partiellement autour de la surface externe du revêtement interne (68) de telle sorte qu'au moins une partie de la surface interne de la première couche tubulaire externe est positionnée de manière adjacente à la surface externe de ladite au moins une partie pliée du revêtement interne (68) ;
le tube allongé étant positionné de telle sorte qu'au moins une partie de la surface interne du tube allongé recouvre au moins une partie de la surface externe de la première couche tubulaire externe ; et la gaine (66) présentant une force d'insertion inférieure à environ 55 N lorsqu'un dispositif médical est poussé à travers la gaine.

15. Procédé de fabrication d'une gaine (22 ; 66) selon l'une quelconque des revendications 1 à 14, comprenant :
a) l'extrusion d'un corps tubulaire afin de former un tube allongé comprenant une première couche de polymère (24 ; 68), la première couche de polymère (24 ; 68) comprenant une première composition combinée comprenant
plus de 0 % à moins de 100 % d'un polymère comprenant un polyéther-bloc-amide, un polyuréthane ou une combinaison correspondante sur la base d'un poids total de la première composition combinée ;
plus de 0 % à moins d'environ 65 % d'une charge inorganique sur la base d'un poids total de la première composition combinée ; et
plus de 0 % jusqu'à environ 20 % d'une charge lubrifiante solide sur la base d'un poids total de la première composition combinée ;
b) la disposition du tube allongé sur la gaine (22 ; 66) de telle sorte que le tube allongé forme une couche externe (70) de la gaine (22 ; 66) et le tube allongé étant positionné au niveau d'au moins l'extrémité proximale de la gaine (22 ; 66) et s'étendant le long d'au moins une partie d'une longueur de la gaine (22 ; 66), le tube allongé étant conçu pour s'étendre de manière réversible à partir d'un diamètre initial d₀ dans une position non étendue à un diamètre étendu dₑ dans une position étendue lors du passage d'un dispositif médical ; et
la gaine (22 ; 66) formée présentant au moins une réduction de 10 % d'une force d'insertion par rapport à une gaine de référence sensiblement identique qui ne comprend pas la première couche de polymère (24 ; 68).
